# EUROPÄISCHE PATENTSCHRIFT

(11) **EP 0 918 775 B1**
(45) Veröffentlichungstag und Bekanntmachung des Hinweises auf die Patenterteilung: **09.11.2005**
(21) Anmeldenummer: 97918958.6
(22) Anmeldetag: 05.08.1997
(51) Int. Cl.: C07D 491/10, C07D 491/20, C07D 493/10, C07D 319/06, C07D 319/08, A01N 43/90

(54) **PHENYLSUBSTITUIERTE CYCLISCHE KETOENOLE**
PHENYL-SUBSTITUTED CYCLIC KETOENOL
CETO-ENOLS CYCLIQUES SUBSTITUES AU NIVEAU DU GROUPE PHENYLE

(30) Priorität: 09.08.1996 DE 19632126
(43) Veröffentlichungstag der Anmeldung: 02.06.1999
(73) Patentinhaber: Bayer CropScience AG, 40789 Monheim (DE)
(72) Erfinder: FISCHER, Reiner, D-40789 Monheim (DE); SCHNEIDER, Udo, D-51373 Leverkusen (DE); DAHMEN, Peter, D-41470 Neuss (DE); DOLLINGER, Markus, D-51381 Leverkusen (DE); ERDELEN, Christoph, D-42799 Leichlingen (DE); WACHENDORFF-NEUMANN, Ulrike, D-56566 Neuwied (DE)
(86) Internationale Anmeldenummer: PCT/EP1997/004246
(87) Internationale Veröffentlichungsnummer: WO 1998/006721

(56) Entgegenhaltungen:
- EP-A- 0 613 884
- EP-A- 0 704 448
- WO-A-94/29268
- WO-A-95/26954
- DE-A- 4 326 909
- DE-A- 4 415 334
- DE-A- 4 431 225
- DE-A- 4 431 730
- DE-A- 19 520 936
- U. SCHMIDT ET AL.: SYNTHESIS, August 1993, Seiten 815-8, XP002050243 in der Anmeldung erwähnt
- M. SENKUS: J. AM. CHEM. SOC., Bd. 63, 1941, Seiten 2635-6, XP002050244 in der Anmeldung erwähnt
- CHEMICAL ABSTRACTS, vol. 95, no. 1, 6.Juli 1981 Columbus, Ohio, US; abstract no. 7174e, Seite 679; XP002050245

## Beschreibung

Die Erfindung betrifft neue phenylsubstituierte cyclische Ketoenole, mehrere Verfahren zu ihrer Herstellung und ihre Verwendung als Schädlingsbekämpfungsmittel und Herbizide.

Bekannt sind 1H-Arylpyrrolidin-dion-Derivate (EP-456 063, EP-521 334, EP-596 298, EP-613 884, EP-613 885, WO 94/01 997, WO 95/01358, WO 95/26954, WO 96/00382, WO 95/20572, EP 668 267) mit herbizider, akarizider und insektizider Wirkung.

Die Wirksamkeit und Wirkungsbreite dieser Verbindungen ist jedoch, insbesondere bei niedrigen Aufwandmengen und Konzentrationen, nicht immer voll zufriedenstellend. Weiterhin ist die Pflanzenverträglichkeit dieser Verbindungen nicht immer ausreichend.

Es wurden nun neue Verbindungen der Formel (I) 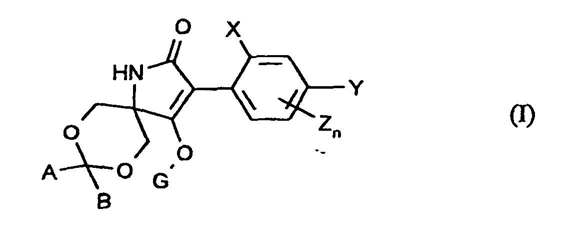 gefunden,
in welcher
- X: steht bevorzugt für Halogen, C₁-C₆-Alkyl, C₂-C₆-Alkenyl, C₂-C₆-Alkinyl, C₁-C₆-Alkoxy, C₃-C₆-Alkenyloxy, C₁-C₆-Alkylthio, C₁-C₆-Alkylsulfinyl, C₁-C₆-Alkylsulfonyl, C₁-C₆-Halogenalkyl, C₂-C₆-Halogenalkenyl, C₁-C₆- Halogenalkoxy, C₃-C₆-Halogenalkenyloxy, Nitro, Cyano oder jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Benzyloxy oder Benzylthio.
- Y: steht bevorzugt für Wasserstoff, Halogen, C₁-C₆-Alkyl, C₂-C₆-Alkenyl, C₂-C₆-Alkinyl, C₁-C₆-Alkoxy, C₃-C₆-Alkenyloxy, C₁-C₆-Alkylthio, C₁-C₆-Alkylsulfinyl, C₁-C₆-Alkylsulfonyl, C₁-C₆-Halogenalkyl, C₂-C₆-Halogenalkenyl, C₁-C₆-Halogenalkoxy, C₃-C₆-Halogenalkenyloxy, Nitro oder Cyano.
- Z: steht bevorzugt für Halogen, C₁-C₆-Alkyl, C₂-C₆-Alkenyl, C₂-C₆-Alkinyl, C₁-C₆-Halogenalkyl, C₂-C₆-Halogenalkenyl, C₁-C₆-Alkoxy, C₃-C₆-Alkenyloxy, C₁-C₆-Halogenalkoxy, C₃-C₆-Halogenalkenyloxy, Nitro oder Cyano,
- n: steht bevorzugt für 0, 1, 2 oder 3,
- A: steht bevorzugt für Wasserstoff oder jeweils gegebenenfalls durch Halogen substituiertes C₁-C₁₂-Alkyl, C₂-C₈-Alkenyl, C₂-C₈-Alkinyl, C₁-C₁₀-Alkoxy-C₁-C₈-alkyl, Poly-C₁-C₈-alkoxy-C₁-C₈-alkyl oder C₁-C₁₀-Alkylthio-C₁-C₆-alkyl, gegebenenfalls durch Halogen, C₁-C₆-Alkyl oder C₁-C₆-Alkoxy substituiertes C₃-C₈-Cycloalkyl, in welchem gegebenenfalls eine Methylen-gruppe durch Sauerstoff oder Schwefel ersetzt ist oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl oder Naphthyl, Hetaryl mit 5 bis 6 Ringatomen, bevorzugt Furanyl, Pyridyl, Imidazolyl, Triazolyl, Pyrazolyl, Pyrimidyl, Thiazolyl oder Thienyl oder Phenyl- oder Naphthyl-C₁-C₆-alkyl, die außerdem durch gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenoxy substituiert sein können,
- B: steht bevorzugt für Wasserstoff oder jeweils gegebenenfalls durch Halogen substituiertes C₁-C₁₂-Alkyl oder C₁-C₈-Alkoxy-C₁-C₆-alkyl oder
- A, B: und das Kohlenstoffatom an das sie gebunden sind, stehen bevorzugt für gesättigtes oder ungesättigtes C₃-C₁₀-Cycloalkyl (insbesondere C₅-C₈-Cycloalkyl), in welchem gegebenenfalls eine Methylengruppe durch Sauerstoff oder Schwefel ersetzt ist und welches gegebenenfalls einfach oder mehrfach durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert ist oder
- A, B: und das Kohlenstoffatom, an das sie gebunden sind, stehen bevorzugt für C₅-C₈-Cycloalkyl oder C₅-C₈-Cycloalkenyl, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₅-C₆-Alkandiyl, C₅-C₆-Alkendiyl oder C₆-Alkandiendiyl stehen, worin gegebenenfalls eine Methylengruppe durch Sauerstoff oder Schwefel ersetzt ist.
- G: steht bevorzugt für Wasserstoff (a) oder für eine der Gruppen 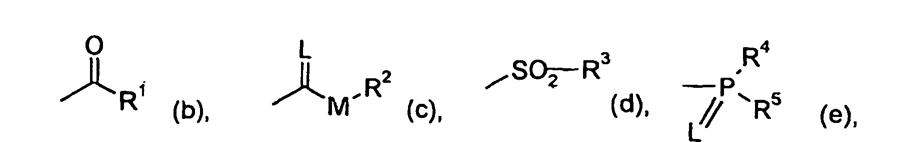 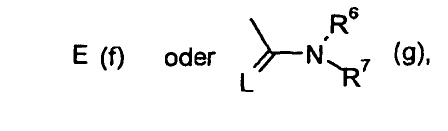
in welchen
- E: für ein Metallionäquivalent oder ein Ammoniumion steht,
- L: für Sauerstoff oder Schwefel steht und
- M: für Sauerstoff oder Schwefel steht.

- R¹: M für Sauerstoff oder Schwefel steht. steht bevorzugt für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls mindestens eine Methylengruppe durch Sauerstoff oder Schwefel ersetzt ist,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl (insbesondere Pyrazolyl, Thiazolyl, Pyridyl, Pyrimidyl, Furanyl oder Thienyl),
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl (insbesondere Pyridyloxy-C₁-C₆-alkyl, Pyrimidyloxy-C₁-C₆-alkyl oder Thiazolyloxy-C₁-C₆-alkyl).
- R²: steht bevorzugt für jeweils gegebenenfalls durch Halogen substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl.
- R³: steht bevorzugt für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl.
- R⁴ und R⁵: stehen bevorzugt unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio, C₂-C₈-Alkenylthio oder C₃-C₇-Cycloalkylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio.
- R⁶ und R⁷: stehen unabhängig voneinander bevorzugt für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl oder C₁-C₈-Alkoxy-C₁-C₈-alkyl, für gegebenenfalls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen C₃-C₆-Alkylenrest, in welchem gegebenenfalls eine Methylengruppe durch Sauerstoff oder Schwefel ersetzt ist.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Weiterhin wurde gefunden, daß man die neuen Verbindungen der Formel (I) nach einem der im folgenden beschriebenen Verfahren erhält:
(A) Man erhält substituierte 3-Phenylpyrrolidin-2,4-dione bzw. deren Enole der Formel (I-a) 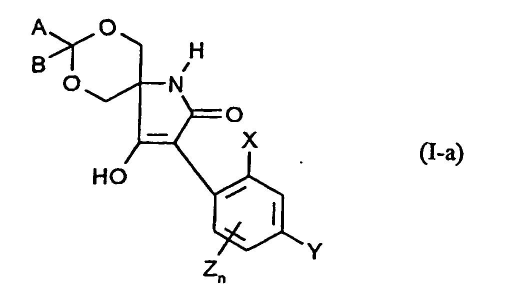 in welcher
   A, B, X, Y, Z und n die oben angegebenen Bedeutungen haben,
   wenn man
   N-Acylaminosäureester der Formel (II) 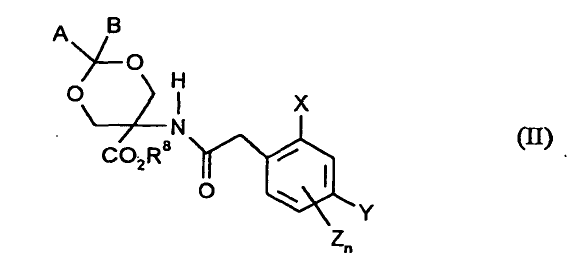 in welcher
   A, B, X, Y, Z und n die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert.
   Außerdem wurde gefunden,
(B)
   α) daß man Verbindungen der oben gezeigten Formel (I) erhält, in welcher A, B, X, Y, Z, R¹ und n die oben angegebenen Bedeutungen haben, und G für die Grüppe (b) steht man Verbindungen der Formel (I-a), in welcher A, B, X, Y, Z und n die oben angegebenen Bedeutungen haben, mit Säurehalogeniden der Formel (III) 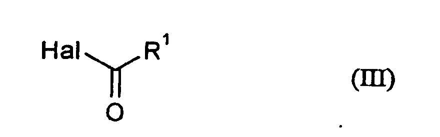 in welcher
      - R¹: die oben angegebene Bedeutung hat und
      - Hal: für Halogen (insbesondere Chlor oder Brom) steht
      oder
   β) mit Carbonsäureanhydriden der Formel (IV)

      R¹-CO-O-CO-R¹ (IV)

      in welcher
      - R¹: die oben angegebene Bedeutung hat,
      gegebenenfalls in Gegenwart eines Verdünnungsmittels und gegebenenfalls in Gegenwart eines Säurebindemittels umsetzt;
(C) daß man die Verbindungen der oben gezeigten Formel (I), in welcher A, B, R², M, X, Y, Z und n die oben angegebenen Bedeutungen haben und L für Sauerstoff steht, erhält, und G für die Gruppe (c) steht wenn man Verbindungen der oben gezeigten Formel (I-a), in welcher A, B, X, Y, Z und n die oben angegebenen Bedeutungen haben, jeweils mit Chlorameisensäureestern oder Chlorameisensäurethiolestern der Formel (V)

   R²-M-CO-Cl (V)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(D) daß man Verbindungen der oben gezeigten Formel (I), in welcher A, B, R², M, X, Y, Z und n die oben angesehenen Bedeutungen haben und L für Schwefel steht, und G für die Gruppe (c) steht, erhält, wenn man Verbindungen der oben gezeigten Formel (I-a), in welcher A, B, X, Y, Z und n die oben angegebenen Bedeutungen haben, jeweils
   mit Chlormonothioameisensäureestem oder Chlordithioameisensäureestern der Formel (VI) 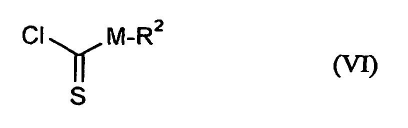 in welcher
   - M und R²: die oben angegebenen Bedeutungen haben,
   gegebenenfalls in Gegenwart eines Verdünnungsmittels und gegebenenfalls in Gegenwart eines Säurebindemittels umsetzt,
(E) daß man Verbindungen der oben gezeigten Formel (I) in welcher A, B, R³, X, Y, Z und n die oben angegebenen Bedeutungen haben, und G für die Gruppe (d) steht erhält, wenn man Verbindungen der oben gezeigten Formeln (I-a), in welcher A, B, X, Y, Z und n die oben angegebenen Bedeutungen haben, jeweils
   mit Sulfonsäurechloriden der Formel (VII)

   R³-SO₂-Cl (VII)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(F) daß man Verbindungen der oben gezeigten Formel (I), in welcher A, B, L, R⁴, R⁵, X, Y, Z und n die oben angegebenen Bedeutungen haben, und G für die Gruppe (e) steht wenn man Verbindungen der oben gezeigten Formeln (I-a), in welcher A, B, X, Y, Z und n die oben angegebenen Bedeutungen haben, jeweils
   mit Phosphorverbindungen der Formel (VIII) 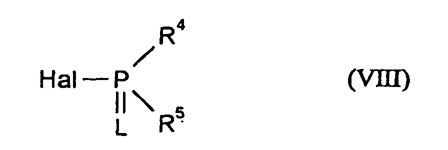 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(G) daß man Verbindungen der oben gezeigten Formel (I), in welcher A, B, E, X, Y, Z und n die oben angegebenen Bedeutungen haben, erhält, und G für die Grüppe (f) steht wenn man Verbindungen der Formeln (I-a), in welcher A, B, X, Y, Z und n die oben angegebenen Bedeutungen haben, jeweils
   mit Metallverbindungen oder Aminen der Formeln (IX) oder (X)

   Me(OR¹⁰)ₜ (IX)

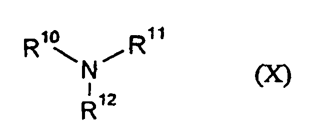

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(H) daß man Verbindungen der oben gezeigten Formel (I), in welcher A, B, L, R⁶, R⁷, X, Y, Z und n die oben angegebenen Bedeutungen haben, und G für die Grüppe (g) steht erhält, wenn man Verbindungen der oben gezeigten Formel (I-a), in welcher A, B, X, Y, Z und n die oben angegebenen Bedeutungen haben,
   mit Carbamidsäurechloriden oder Thiocarbamidsäurechloriden der Formel (XI) 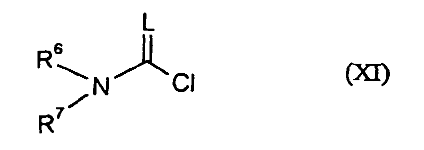
in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt.

Die erfindungsgemäßen Verbindungen der Formeln (I-a) sind somit wichtige Zwischenprodukte für die Herstellung der erfindungsgemäßen Verbindungen der Formeln (I), in welchen G für eine der Gruppen b), c), d), e), f) oder g) steht.

Weiterhin wurde gefunden, daß die neuen Verbindungen der Formel (I) eine sehr gute Wirksamkeit als Schädlingsbekämpfungsmittel, vorzugsweise als Insektizide, Akarizide und Herbizide aufweisen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- X: steht besonders bevorzugt für Fluor, Chlor, Brom, C₁-C₄-Alkyl, C₁-C₄-Alkoxy, C₁-C₄-Halogenalkyl, C₁-C₄-Halogenalkoxy, Nitro, Cyano oder jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl oder Benzyloxy.
- Y: steht besonders bevorzugt für Wasserstoff, Fluor, Chlor, Brom, C₁-C₄-Alkyl, C₁-C₄-Alkoxy, C₃-C₄-Alkenyloxy, C₁-C₄-Halogenalkyl, C₁-C₄-Halogenalkoxy, Nitro oder Cyano.
- Z: steht besonders bevorzugt für Fluor, Chlor, Brom, C₁-C₄-Alkyl, C₁-C₄-Halogenalkyl, C₁-C₄-Alkoxy, C₁-C₄-Halogenalkoxy, Nitro oder Cyano.
- n: steht besonders bevorzugt für 0, 1, 2 oder 3 (insbesondere für 0, 1 oder 2).
- A: steht besonders bevorzugt für Wasserstoff oder jeweils gegebenenfalls durch Fluor oder Chlor substituiertes C₁-C₁₀-Alkyl oder C₁-C₈-Alkoxy-C₁-C₆-alkyl, gegebenenfalls durch Fluor, Chlor, C₁-C₄-Alkyl oder C₁-C₄-Alkoxy substituiertes C₃-C₇-Cycloalky, oder jeweils gegebenenfalls durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl, Furanyl, Pyridyl, Imidazolyl, Triazolyl, Pyrazolyl, Pyrimidyl, Thiazolyl, Thienyl oder Phenyl-C₁-C₄-alkyl.
- B: steht besonders bevorzugt für Wasserstoff oder jeweils gegebenenfalls durch Fluor oder Chlor substituiertes C₁-C₁₀-Alkyl oder C₁-C₆-Alkoxy-C₁-C₄-alkyl oder
- A, B: und das Kohlenstoffatom an das sie gebunden sind, stehen besonders bevorzugt für gesättigtes oder ungesättigtes C₅-C₈-Cycloalkyl, in welchem gegebenenfalls eine Methylengruppe durch Sauerstoff oder Schwefel ersetzt ist und welches gegebenenfalls durch C₁-C₆-Alkyl, C₃-C₈-Cycloalkyl, C₁-C₃-Halogenalkyl, C₁-C₆-Alkoxy, C₁-C₆-Alkylthio, Fluor, Chlor oder Phenyl substituiert ist oder
- A, B: und das Kohlenstoffatom, an das sie gebunden sind, stehen besonders bevorzugt für C₅-C₆-Cycloalkyl oder C₅-C₆-Cycloalkenyl, in welchen zwei Substituenten gemeinsam mit den Kohlenstoffatomen, an die sie gebunden sind, für jeweils gegebenenfalls durch C₁-C₅-Alkyl, C₁-C₅-Alkoxy, Fluor, Chlor oder Brom substituiertes C₅-C₆-Alkandiyl, C₅-C₆-Alkendiyl oder C₆-Alkandiendiyl stehen, worin gegebenenfalls eine Methylengruppe durch Sauerstoff oder Schwefel ersetzt ist.
- G: steht besonders bevorzugt für Wasserstoff (a) oder eine der Gruppen 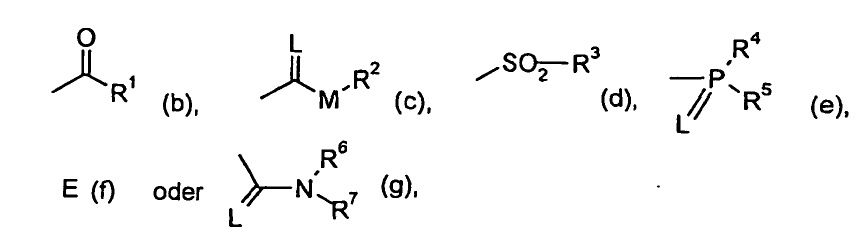
in welchen
- E: für ein Metallionäquivalent oder ein Ammoniumion steht,
- L: für Sauerstoff oder Schwefel steht und
- M: für Sauerstoff oder Schwefel steht.

- R¹: steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl oder Poly-C₁-C₆-alkoxy-C₁-C₆-alkyl oder gegebenenfalls durch Fluor, Chlor, C₁-C₅-Alkyl oder C₁-C₅-Alkoxy substituiertes C₃-C₇-Cycloalkyl, in welchem gegebenenfalls ein oder zwei nicht direkt benachbarte Methylengruppen durch Sauerstoff oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls durch Fluor, Chlor, Brom oder C₁-C₄-Alkyl substituiertes Phenoxy-C₁-C₅-alkyl oder
für jeweils gegebenenfalls durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl.
- R²: steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl.
- R³: steht besonders bevorzugt für gegebenenfalls durch Fluor oder Chlor substituiertes C₁-C₆-Alkyl oder für jeweils gegebenenfalls durch Fluor, Chlor, Brom, C₁-C₅-Alkyl, C₁-C₅-Alkoxy, C₁-C₃-Halogenalkyl, C₁-C₃-Halogenalkoxy, Cyano oder Nitro substituiertes Phenyl oder Benzyl,
- R⁴ und R⁵: stehen besonders bevorzugt unabhängig voneinander für jeweils gegebenenfalls durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio oder C₃-C₆-Cycloalkylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio.
- R⁶ und R⁷: stehen unabhängig voneinander besonders bevorzugt für Wasserstoff, für jeweils gegebenenfalls durch Halogen substituiertes C₁-C₆-Alkyl, C₃-C₆-Cycloalkyl, C₁-C₆-Alkoxy, C₃-C₆-Alkenyl oder C₁-C₆-Alkoxy-C₁-C₆-alkyl, für gegebenenfalls durch Halogen, C₁-C₅-Halogenalkyl, C₁-C₅-Alkyl oder C₁-C₅-Alkoxy substituiertes Phenyl, für gegebenenfalls durch Halogen, C₁-C₅-Alkyl, C₁-C₅-Halogenalkyl oder C₁-C₅-Alkoxy substituiertes Benzyl, oder zusammen für einen C₃-C₆-Alkylenrest, in welchem gegebenenfalls eine Methylengruppe durch Sauerstoff oder Schwefel ersetzt ist.
- X: steht ganz besonders bevorzugt für Fluor, Chlor, Brom, Methyl, Ethyl, Propyl, iso-Propyl, Methoxy, Ethoxy, Propoxy, iso-Propoxy, Trifluormethyl, Difluormethoxy, Trifluormethoxy, Nitro oder Cyano.
- Y: steht ganz besonders bevorzugt für Wasserstoff, Fluor, Chlor, Brom, Methyl, Ethyl, n-Propyl, i-Propyl, n-Butyl, i-Butyl, tert.-Butyl, Methoxy, Ethoxy, Propoxy, iso-Propoxy, Allyloxy, Methallyloxy, Trifluormethyl, Difluormethoxy, Trifluormethoxy, Nitro oder Cyano.
- Z: steht ganz besonders bevorzugt für Fluor, Chlor, Brom, Methyl, Ethyl, n-Propyl, i-Propyl, n-Butyl, i-Butyl, tert.-Butyl, Methoxy, Ethoxy, Propoxy, iso-Propoxy, Difluormethoxy, Trifluormethyl, Trifluormethoxy, Nitro oder Cyano.
- n: steht ganz besonders bevorzugt für 0, 1, 2 oder 3 (insbesondere für 0, 1 oder 2, hervorgehoben für 0 oder 1).
- A: steht ganz besonders bevorzugt für Wasserstoff oder jeweils gegebenenfalls durch Fluor oder Chlor substituiertes C₁-C₈-Alkyl oder C₁-C₆-Alkoxy-C₁-C₄-alkyl, gegebenenfalls durch Fluor, Chlor, Methyl, Ethyl, iso-Propyl, tert.-Butyl, Ethoxy oder Methoxy substituiertes C₅-C₆-Cycloalkyl, oder für jeweils gegebenenfalls durch Fluor, Chlor, Brom, Methyl, Ethyl, iso-Propyl, tert.-Butyl, Methoxy, Ethoxy, Trifluormethyl, Difluormethoxy, Trifluormethoxy, Cyano oder Nitro substituiertes Phenyl oder Benzyl.
- B: steht ganz besonders bevorzugt für Wasserstoff oder jeweils gegebenenfalls durch Fluor substituiertes C₁-C₈-Alkyl oder C₁-C₄-Alkoxy-C₁-C₂-alkyl oder
- A, B: und das Kohlenstoffatom an das sie gebunden sind, stehen ganz besonders bevorzugt für gesättigtes oder ungesättigtes C₅-C₆-Cycloalkyl, in welchem gegebenenfalls eine Methylengruppe durch Sauerstoff oder Schwefel ersetzt ist und welches gegebenenfalls durch Methyl, Ethyl, Isopropyl, tert.-Butyl, Trifluormethyl, Methoxy, Ethoxy, Fluor, Chlor oder Phenyl substituiert ist oder
- A, B: und das Kohlenstoffatom, an das sie gebunden sind, stehen ganz besonders bevorzugt für C₅-C₆-Cycloalkyl oder C₅-C₆-Cycloalkenyl, in dem zwei Substituenten gemeinsam mit den Kohlenstoffatomen, an die sie gebunden sind, für C₅-C₆-Alkandiyl, C₅-C₆-Alkendiyl oder C₆-Alkandiendiyl stehen.
- G: steht ganz besonders bevorzugt für Wasserstoff (a) oder für eine der Gruppen 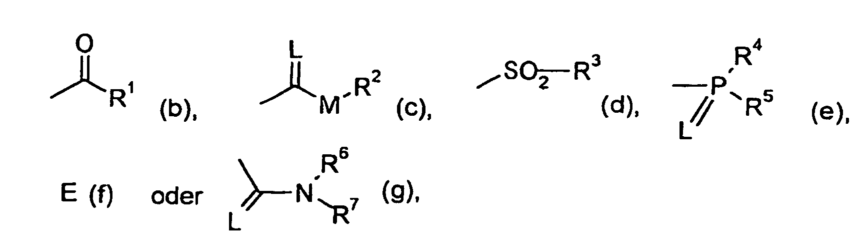
(insbesondere für eine der Gruppen (a), (b) oder (c)),
in welchen
- E: für ein Metallionäquivalent oder ein Ammoniumion steht,
- L: für Sauerstoff oder Schwefel steht und
- M: für Sauerstoff oder Schwefel steht.

- R¹: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl oder Poly-C₁-C₄-alkoxy-C₁-C₄-alkyl oder gegebenenfalls durch Fluor, Chlor, Methyl, Ethyl, Propyl, i-Propyl, Butyl, i-Butyl, tert.-Butyl, Methoxy, Ethoxy, Propoxy oder iso-Propoxy substituiertes C₃-C₆-Cycloalkyl, in welchem gegebenenfalls eine oder zwei nicht direkt benachbarte Methylengruppen durch Sauerstoff oder Schwefel ersetzt sind,
für gegebenenfalls durch Fluor, Chlor, Brom, Cyano, Nitro, Methyl, Ethyl, Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl, Pyridyl, Pyrimidyl, Thiazolyl oder Pyrazolyl,
für gegebenenfalls durch Fluor, Chlor, Methyl oder Ethyl substituiertes Phenoxy-C₁-C₄-alkyl oder
für jeweils gegebenenfalls durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl.
- R²: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, Propyl, i-Propyl, Methoxy, Ethoxy, Trifluormethyl oder Trifluor-methoxy substituiertes Phenyl oder Benzyl.
- R³: steht ganz besonders bevorzugt für jeweils gegebenenfalls durch Fluor oder Chlor substituiertes Methyl, Ethyl, Propyl, Isopropyl oder jeweils gegebenenfalls durch Fluor, Chlor, Brom, Methyl, Ethyl, Propyl, iso-Propyl, tert.-Butyl, Methoxy, Ethoxy, Isopropoxy, tert.-Butoxy, Trifluormethyl, Trifluormethoxy, Cyano oder Nitro substituiertes Phenyl oder Benzyl,
- R⁴ und R⁵: stehen ganz besonders bevorzugt unabhängig voneinander für jeweils gegebenenfalls durch Fluor oder Chlor substituiertes C₁-C₄-Alkyl, C₁-C₄-Alkoxy, C₁-C₄-Alkylamino, Di-(C₁-C₄-alkyl)amino, C₁-C₄-Alkylthio oder für jeweils gegebenenfalls durch Fluor, Chlor, Brom, Nitro, Cyano, C₁-C₂-Alkoxy, C₁-C₂-Fluoralkoxy, C₁-C₂-Alkylthio, C₁-C₂-Fluoralkylthio oder C₁-C₃-Alkyl substituiertes Phenyl, Phenoxy oder Phenylthio.
- R⁶ und R⁷: stehen unabhängig voneinander ganz besonders bevorzugt für Wasserstoff, für jeweils gegebenenfalls durch Fluor oder Chlor substituiertes C₁-C₄-Alkyl, C₃-C₆-Cycloalkyl, C₁-C₄-Alkoxy, C₃-C₄-Alkenyl oder C₁-C₄-Alkoxy-C₁-C₄-alkyl, für gegebenenfalls durch Fluor, Chlor, Brom, C₁-C₄-Halogenalkyl, C₁-C₄-Alkyl oder C₁-C₄-Alkoxy substituiertes Phenyl, für gegebenenfalls durch Fluor, Chlor, Brom, C₁-C₄-Alkyl, C₁-C₄-Halogenalkyl oder C₁-C₄-Alkoxy substituiertes Benzyl, oder zusammen für einen C₅-C₆-Alkylenrest, in welchem gegebenenfalls eine Methylengruppe durch Sauerstoff oder Schwefel ersetzt ist.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

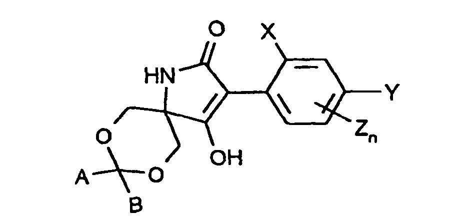

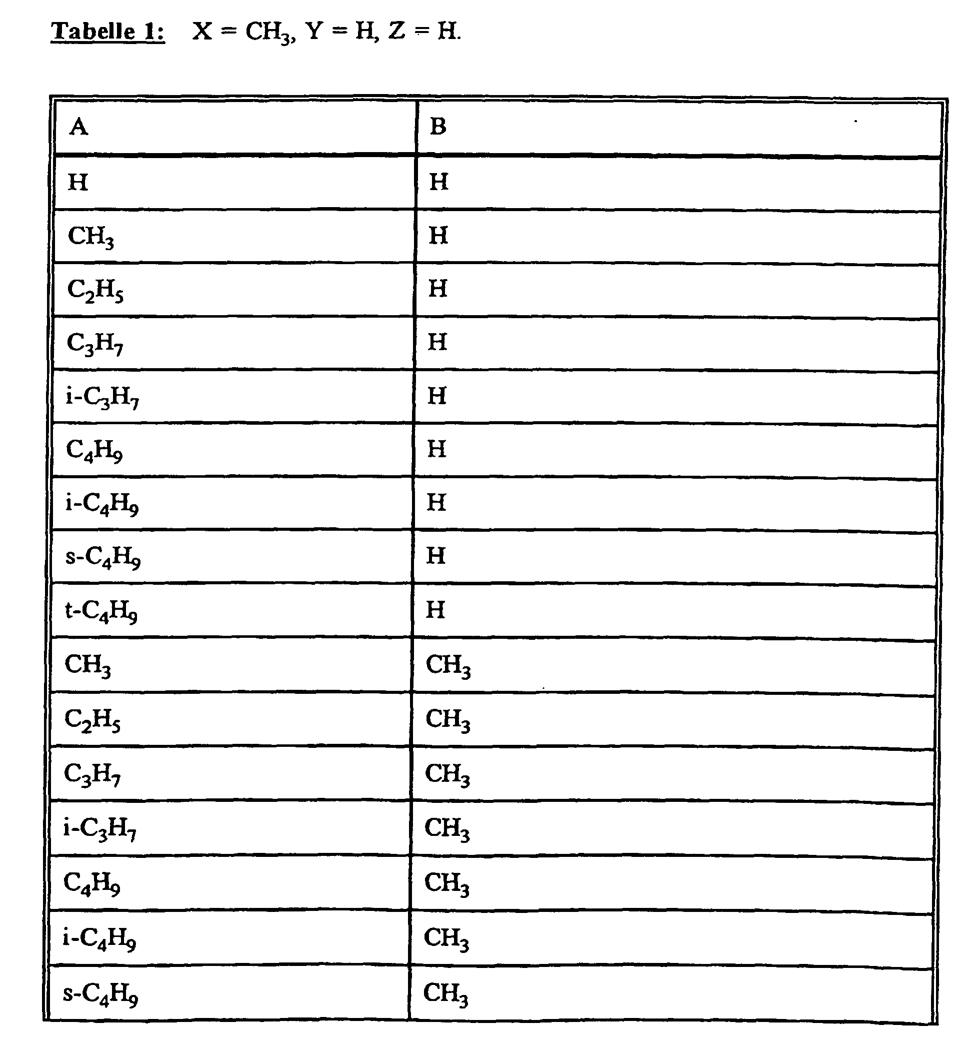

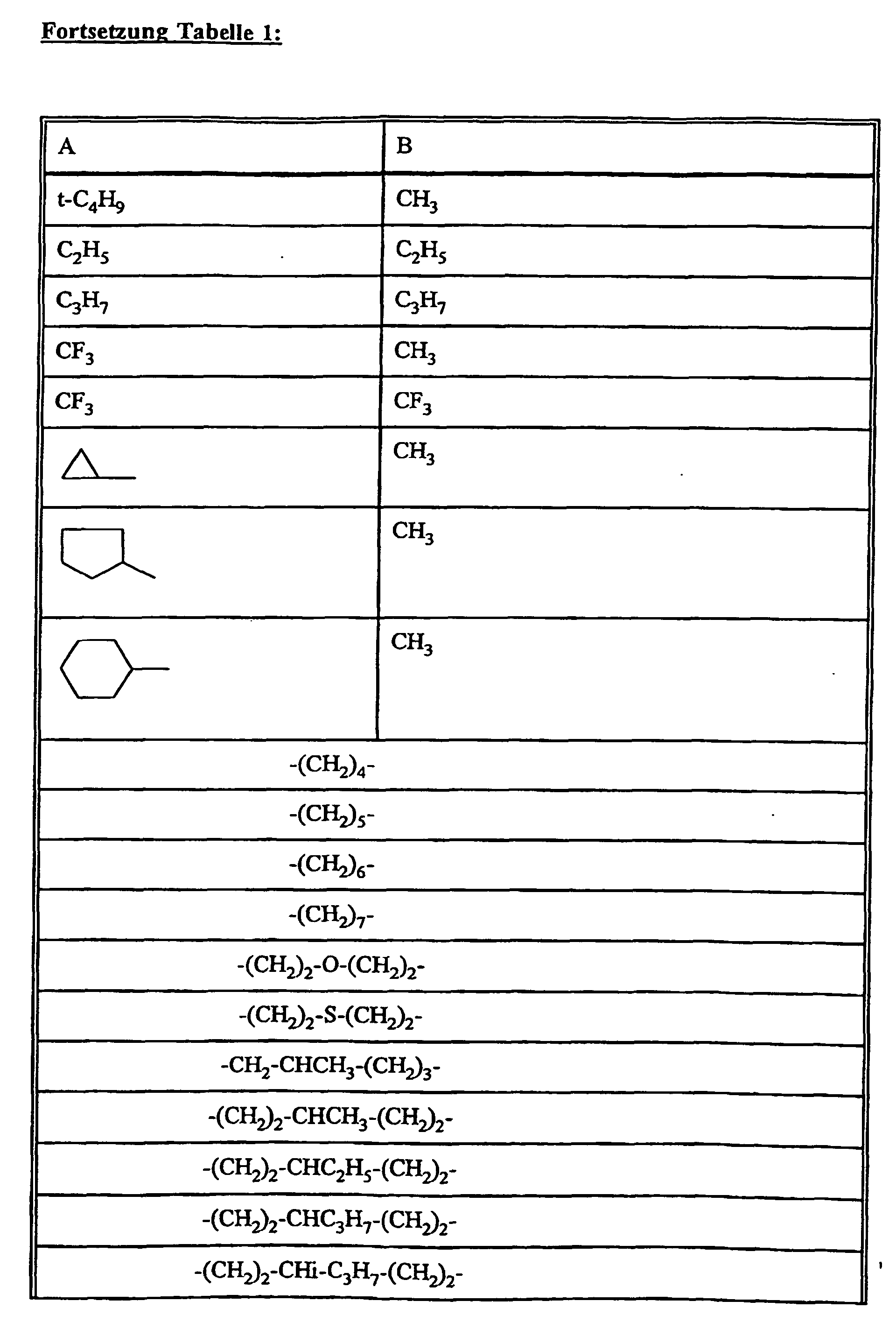

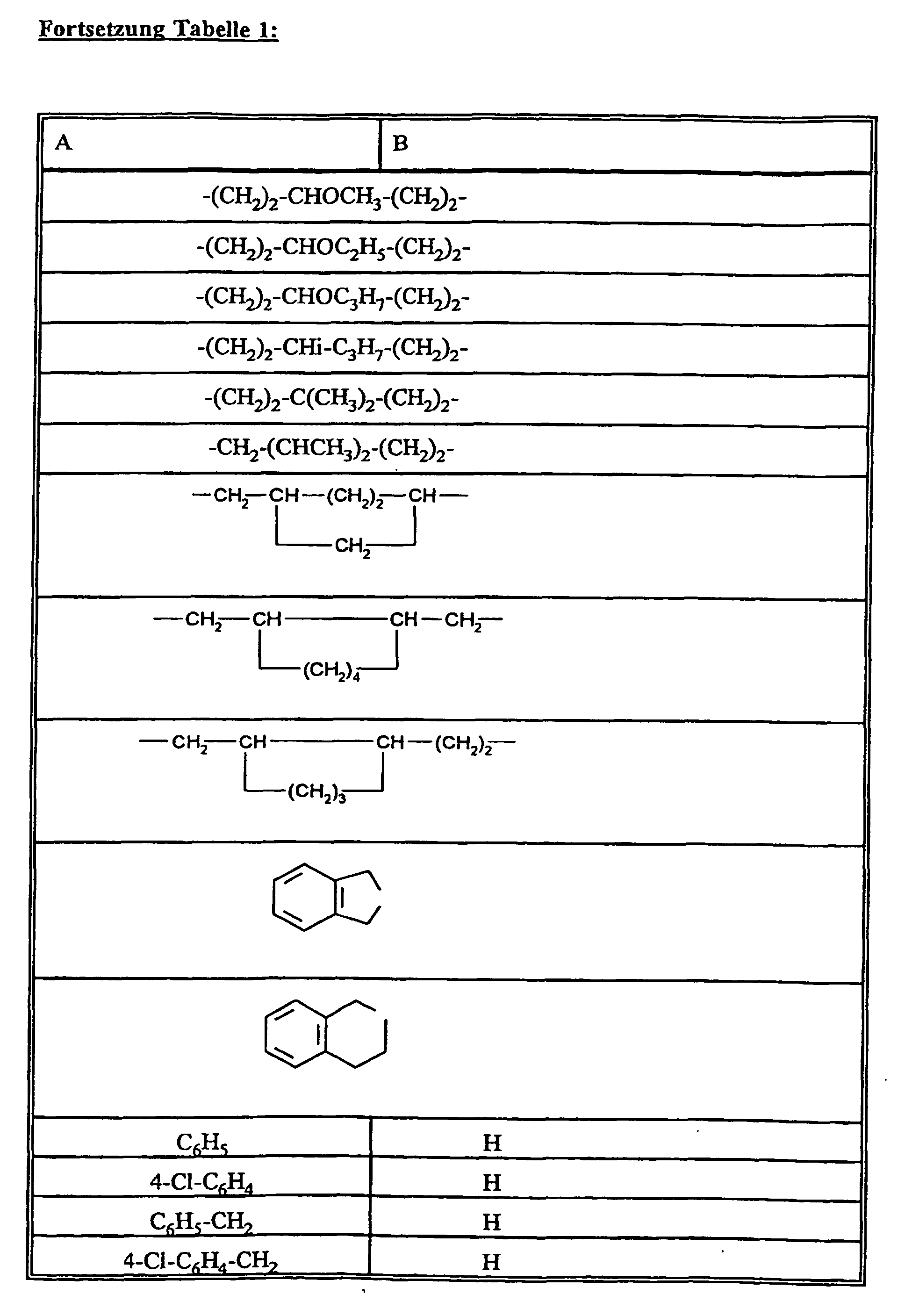

- **Tabelle 2:**: A und B wie in Tabelle 1 angegeben X=Cl; Y=H; Z = H
- **Tabelle 3:**: A und B wie in Tabelle 1 angegeben X = CH₃; Y = CH₃; Z = H
- **Tabelle 4:**: A und B wie in Tabelle 1 angegeben X = CH₃; Y = H; Z = 6-CH₃
- **Tabelle 5:**: A und B wie in Tabelle 1 angegeben X = CH₃; Y = CH₃; Z = 5-CH₃
- **Tabelle 6:**: A und B wie in Tabelle 1 angegeben X = CH₃; Y = CH₃; Z = 6-CH₃
- **Tabelle 7:**: A und B wie in Tabelle 1 angegeben X = Cl; Y = Cl; Z = H
- **Tabelle 8:**: A und B wie in Tabelle 1 angegeben X = Cl; Y = CH₃; Z = H
- **Tabelle 9:**: A und B wie in Tabelle 1 angegeben X = CH₃; Y = Cl; Z = H
- **Tabelle 10:**: A und B wie in Tabelle 1 angegeben X = Cl; Y = Cl; Z = 6-CH₃
- **Tabelle 11:**: A und B wie in Tabelle 1 angegeben X = Cl; Y = CH₃; Z = Cl
- **Tabelle 12:**: A und B wie in Tabelle 1 angegeben X = CH₃; Y = CH₃; Z = 6-Cl
- **Tabelle 13:**: A und B wie in Tabelle 1 angegeben X = CH₃, Y = Cl; Z = 6-CH₃
- **Tabelle 14:**: A und B wie in Tabelle 1 angegeben X = CH₃; Y = CH₃; Z = 6-Br
- **Tabelle 15:**: A und B wie in Tabelle 1 angegeben X = CH₃; Y = Br; Z = 6-CH₃
- **Tabelle 16:**: A und B wie in Tabelle 1 angegeben X = CH₃; Y = Cl; Z = 5-CH₃
- Tabelle 17:: A und B wie in Tabelle 1 angegeben X = CH₃; Y = Br; Z = 5-CH₃
- **Tabelle 18:**: A und B wie in Tabelle 1 angegeben X = Br; Y = CH₃; Z = 5-CH₃
- **Tabelle 19:**: A und B wie in Tabelle 1 angegeben X = Br; Y = Cl, Z = 6-CH₃
- **Tabelle 20:**: A und B wie in Tabelle 1 angegeben X = Br; Y = CH₃; Z = 6-Cl
- **Tabelle 21:**: A und B wie in Tabelle 1 angegeben X = Cl, Y= Br; Z = 6-CH₃

Verwendet man gemäß Verfahren (A) N-[(2,4,6-Trimethylphenyl]-acetyl-2,2-dimethyl-5-amino-1,3-dioxan-5-carbonsäuremethylester, so kann der Verlauf des erfindungsgemäßen Verfahrens durch folgendes Reaktionsschema wiedergegeben werden:

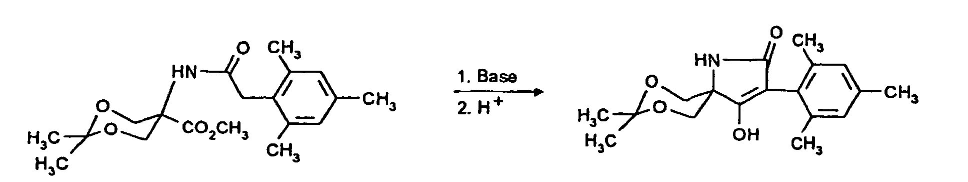

Verwendet man gemäß Verfahren (Bα) 3-[(2,4-Dichlor)-phenyl]-5,5-(2,4-dioxapentamethylen)-2-pyrrolidin-2,4-dion und Pivaloylchlorid als Ausgangsstoffe, so kann der Verlauf des erfindungsgemäßen Verfahrens durch folgendes Reaktionsschema wiedergegeben werden:

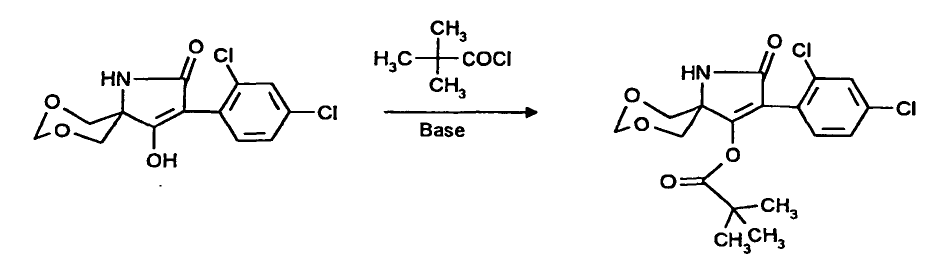

Verwendet man gemäß Verfahren (Bβ) cis-3-[(2,4-Dimethyl)-phenyl]-5,5-[(3-methyl)-2,4-dioxapentamethylen]-Δ²-pyrrolidin-2,4-dion und Acetanhydrid als Ausgangsverbindung, so kann der Verlauf des erfindungsgemäßen Verfahrens durch folgendes Reaktionsschema wiedergegeben werden:

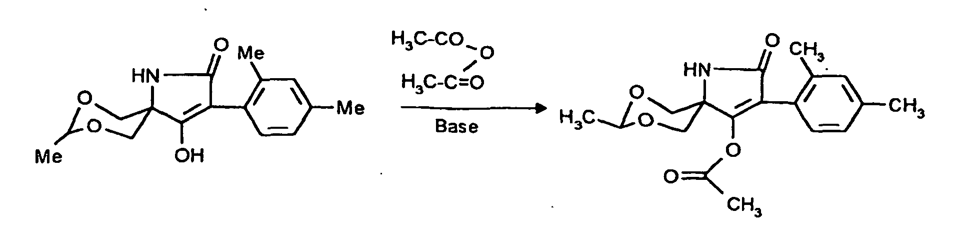

Verwendet man gemäß Verfahren (C) 3-[(2,4,5-Trimethyl)-phenyl)]-5,5-[(3,3-tetramethylen)-2,4-dioxapentamethylen]-Δ²-pyrrolidin-2,4-dion und Chlorameisensäureethylester als Ausgangsverbindungen, so kann der Verlauf des erfindungsgemäßen Verfahrens durch folgendes Reaktionsschema dargestellt werden:

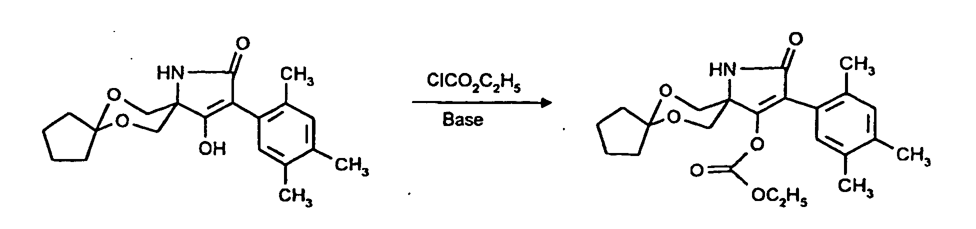

Verwendet man gemäß Verfahren (D) trans-3-[(2,6-Dimethyl-4-brom)-phenyl]-5,5-[(3-methyl)-2,4-dioxapentamethylen]-Δ³-pyrrolidin-2,4-dion und Chlormonothioameisensäuremethylester als Ausgangsprodukt, so kann der Reaktionsverlauf folgendermaßen wiedergegeben werden:

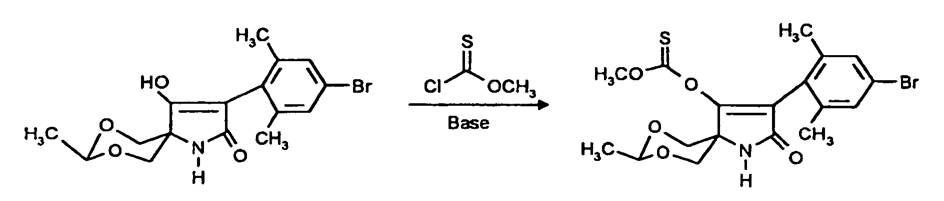

Verwendet man gemäß Verfahren (E) 3-[(2-Chlor-4-methyl)-phenyl]-5,5-[(3,3-pentamethylen)-2,4-dioxapentamethylen]-Δ³-pyrrolidin-2,4-dion und Methansulfonsäurechlorid als Ausgangsprodukte, so kann der Reaktionsverlauf folgendermaßen wiedergegeben werden:

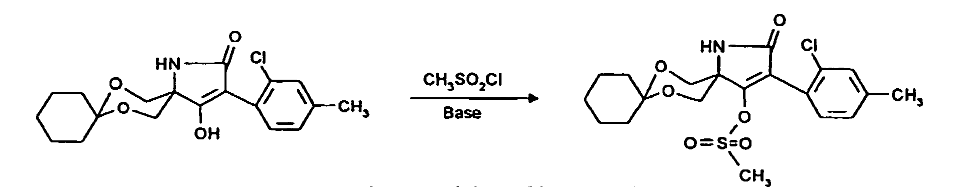

Verwendet man gemäß Verfahren (F) 3-[(2,4,6-Trimethyl)-phenyl]-5,5-(2,4-dioxapentamethylen)-Δ³-pyrrolidin-2,4-dion und Methanthio-phosphorsäurechlorid-(2,2,2-trifluormethylester) als Ausgangsprodukte, so kann der Verlauf durch folgendes Reaktionsschema wiedergegeben werden:

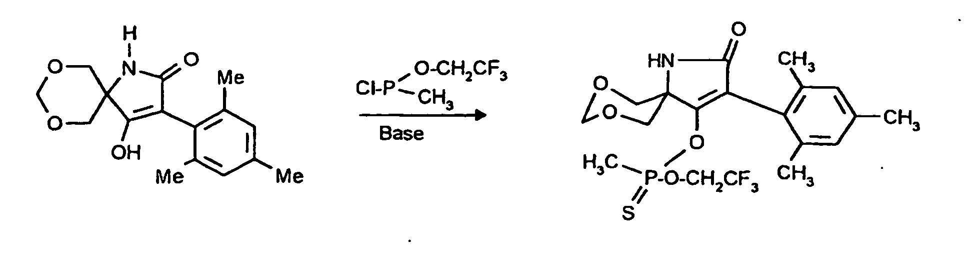

Verwendet man gemäß Verfahren (G) 3-[(2,4,6-Trimethyl)-phenyl]-5,5-[(3,3-di-ethylenyloxy)-2,4-dioxapentamethylen]-Δ³-pyrrolidin-2,4-dion und NaOH als Komponenten, so kann der Reaktionsverlauf des erfindungsgemäßen Verfahrens durch folgendes Reaktionsschema wiedergegeben werden:

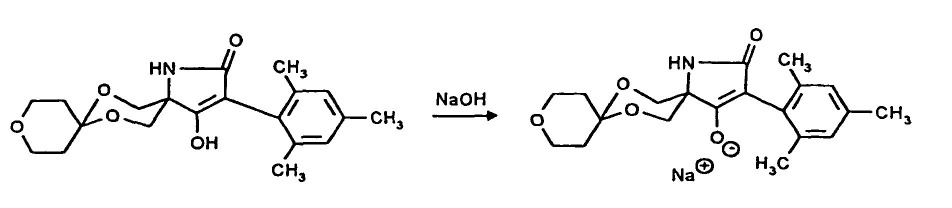

Verwendet man gemäß Verfahren (H) 3-[(2-Methyl-4-chlor)-phenyl]-5,5-[(3,3-dimethyl)-2,4-dioxapentamethylen]-Δ³-pyrrolidin-2,4-dion und Dimethylcarbamidsäurechlorid als Ausgangsprodukte, so kann der Reaktionsverlauf durch folgendes Schema wiedergegeben werden:

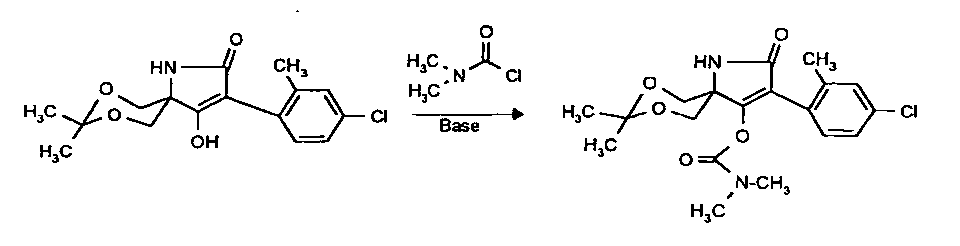

Die beim erfindungsgemäßen Verfahren (A) als Ausgangsstoffe benötigten Verbindungen der Formel (II) 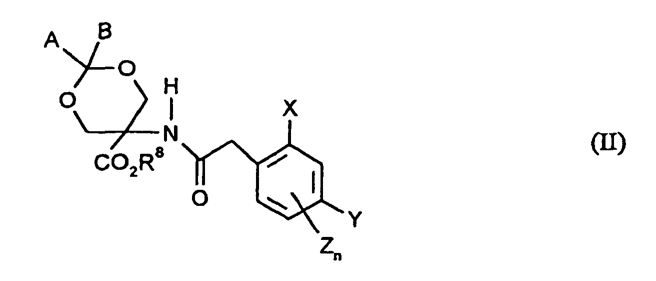 in welcher
A, B, X, Y, Z, R⁸ und n die oben angegebene Bedeutungen haben, sind neu.

Man erhält die Acylaminosäureester der Formel (II) beispielsweise, wenn man Aminosäurederivate der Formel (XII) 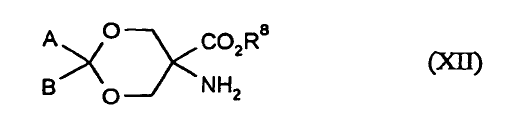 in welcher
A, B und R⁸ die oben angegebenen Bedeutungen haben,
mit substituierten Phenylessigsäurehalogeniden der Formel (XIII) 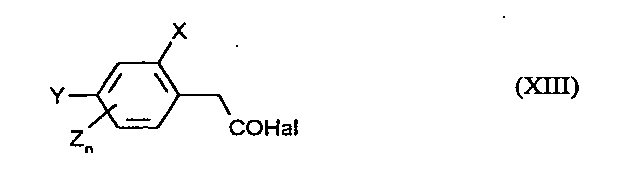 in welcher
X, Y, Z und n die oben angegebenen Bedeutungen haben und
- Hal: für Chlor oder Brom steht,
acyliert (Chem. Reviews 52, 237-416 (1953); Bhattacharya, Indian J. Chem. 6, 341-5, 1968).

Die Verbindungen der Formel (XII) sind neu.

Man erhält die Aminosäureester der Formel (XII) beispielsweise, wenn man Nitrocarbonsäureester der Formel (XIV) 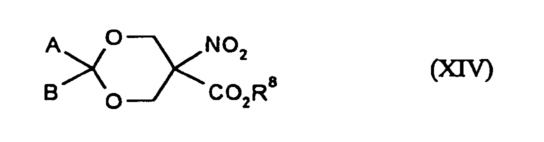 in welcher
A, B und R⁸ die oben angegebene Bedeutung haben,
mit Wasserstoff in Gegenwart eines Hydrierkatalysators wie z.B. Raney-Cobalt oder Raney-Nickel, Palladium oder Platin (z.B. auf Kohle als Trägermaterial), gegebenenfalls in Gegenwart eines Verdünnungsmittels wie z.B. Methanol oder Ethanol bei Temperaturen zwischen -20°C und +200°C, vorzugsweise zwischen 0°C und 150°C und bei Drücken zwischen 1 bar und 300 bar, vorzugsweise zwischen 10 bar und 200 bar umsetzt (vgl. Herstellungsbeispiele).

Die Verbindungen der Formel (XIV) sind teilweise bekannt oder lassen sich nach bekannten Verfahren herstellen (Piotrowska, Urbanski, Wolochowicz; Bulletin de L'Academie Polonaise des Science XIX, 591-94, 1971).

Außerdem erhält man Acylaminoester der Formel (II) beispielsweise, wenn man Aminosäurederivate der Formel (XV) 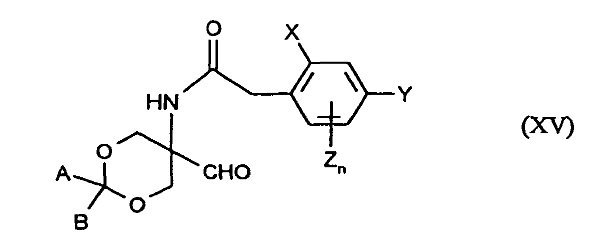 in welcher
A, B, X, Y, Z und n die oben angegebene Bedeutung haben,
in Gegenwart einer Cyanidquelle, einem Oxidationsreagenz und einem Alkohol gegebenenfalls in Gegenwart eines Lösungsmittels oxidiert.

Die Verbindungen der Formel (XV) sind neu, lassen sich aber in Analogie zu einem bekannten Verfahren darstellen (Polniaszek, Stevens, J. Org. Chem. 51, 3023-3027, 1986).

Man erhält die Aminosäurederivate der Formel (XV) beispielsweise, wenn man Acylaminoalkohole der Formel (XVI) 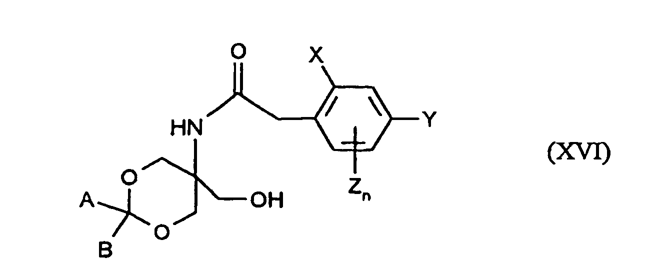 in welcher
A, B, X, Y, Z und n die oben angegebene Bedeutung haben,
beispielsweise nach Swern oxidiert (Polniaszek, Stevens, J. Org. Chem. 51, 3023-3027, 1986; Omura, Swern, Tetrahedron 34, 1951; 1978).

Die Verbindungen der Formel (XVI) sind neu.

Man erhält die Acylaminoalkohole der Formel (XVI) beispielsweise, wenn man 5-Amino-5-hydroxymethyl-1,3-dioxane der Formel (XVII) 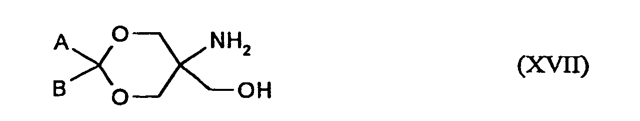 in welcher
A und B die oben angegebene Bedeutung haben,
mit Phenylessigsäurehalogeniden der Formel (XIII) 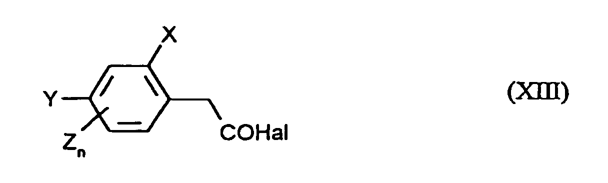 in welcher
X, Y, Z und n die oben angegebene Bedeutung haben und
- Hal: für Chlor oder Brom steht,
gegebenenfalls in Gegenwart eines Lösungsmittels und gegebenenfalls in Gegenwart einer Base acyliert.

Die Verbindungen der Formel (XIII) sind teilweise neu, lassen sich aber nach im Prinzip bekannten Verfahren in Analogie herstellen (siehe Herstellungsbeispiele von Verbindungen der Formel XXXIII-b der Deutschen Patentanmeldung DE-19523850).

Die Verbindungen der Formel (XVII) sind teilweise neu.

Man erhält die Aminoalkohole der Formel (XVII) beispielsweise, wenn man Nitroalkohole der Formel (XVIII) 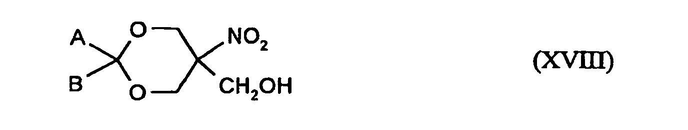 in welcher
A und B die oben angegebene Bedeutung haben,
mit Wasserstoff in Gegenwart eines Hydrierkatalysators wie z.B. Raney-Cobalt oder Raney-Nickel, Palladium oder Platin (z.B. auf Kohle als Trägermaterial), gegebenenfalls in Gegenwart eines Verdünnungsmittels wie z.B. Methanol oder Ethanol bei Temperaturen zwischen -20°C und +200°C, vorzugsweise zwischen 0°C und 150°C und bei Drücken zwischen 1 bar und 300 bar, vorzugsweise zwischen 10 bar und 200 bar umsetzt (vgl. Herstellungsbeispiele).

Die als Vorprodukte benötigten Nitroalkohole der Formel (XVIII) 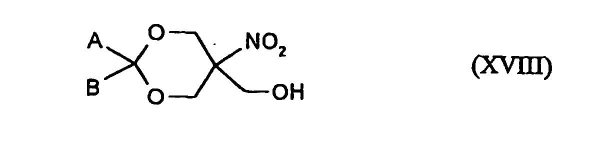 in welcher
A und B die oben angegebene Bedeutung haben,
sind teilweise bekannt und/oder können nach an sich bekannten Verfahren hergestellt werden (vgl. J. Am. Chem. Soc. 63, 2635-2636, (1941); Synthesis 1993, 815-818, WO 95/00020).

Die Verbindungen der Formel (XIII) sind teilweise bekannt und lassen sich darüber hinaus nach den eingangs zitierten Patentanmeldungen bzw. den dort angegebenen Methoden herstellen.

Die zur Durchführung der erfindungsgemäßen Verfahren (B), (C), (D), (E), (F), (G) und (H) außerdem als Ausgangsstoffe benötigten Säurehalogenide der Formel (III), Carbonsäureanhydride der Formel (IV), Chlorameisensäureester oder Chlorameisensäurethioester der Formel (V), Chlormonothioameisensäureester oder Chlordithioameisensäureester der Formel (VI), Sulfonsäurechloride der Formel (VII), Phosphorverbindungen der Formel (VIII) und Metallhydroxide, Metallalkoxide oder Amine der Formel (IX) und (X) und Carbamidsäwechloride der Formel (XI) sind allgemein bekannte Verbindungen der organischen bzw. anorganischen Chemie.

Das Verfahren (A) ist dadurch gekennzeichnet, daß man Verbindungen der Formel (II), in welcher A, B, X, Y, Z, n und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, IsoPropanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die deprotonierenden Basen im allgemeinen in etwa äquimolaren bis doppeltäquimolaren Mengen ein. Es ist jedoch auch möglich, die eine oder andere Komponente in einem größeren Überschuß (bis zu 3 Mol) zu verwenden.

Das Verfahren (Bα) ist dadurch gekennzeichnet, daß man Verbindungen der Formel (I-a) mit Carbonsäurehalogeniden der Formel (III) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Bα) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, Nitrile wie Acetonitril, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (Bα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (I-a) und das Carbonsäurehalogenid der Formel (III) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (I-a) mit Carbonsäureanhydriden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Bβ) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (Bβ) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (Bβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bβ) werden die Ausgangsstoffe der Formel (I-a) und das Carbonsäureanhydrid der Formel (IV) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (I-a) mit Chlorameisensäureestern oder Chlorameisensäurethiolestern der Formel (V) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, Nitrile wie Acetonitril, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (I-a) und der entsprechende Chlorameisensäureester bzw. Chlorameisensäurethiolester der Formel (V) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das erfindungsgemäße Verfahren (D) ist dadurch gekennzeichnet, daß man Verbindungen der Formel (I-a) mit Verbindungen der Formel (VI) in Gegenwart eines Verdünnungsmittels und gegebenenfalls in Gegenwart eines Säurebindemittels umsetzt.

Beim Herstellungsverfahren (D) setzt man pro Mol Ausgangsverbindung der Formel (I-a) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Nitrile, Ketone, Carbonsäureester, Amide, Sulfone, Sulfoxide, aber auch Halogenalkane.

Vorzugsweise werden Dimethylsulfoxid, Tetrahydrofuran, Ethylacetat, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen der Formel (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (E) ist dadurch gekennzeichnet, daß man Verbindungen der Formel (I-a) mit Sulfonsäurechloriden der Formel (VII) gegebenenfalls in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formel (I-a) ca. 1 Mol Sulfonsäurechlorid der Formel (VII) bei -20 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Ketone, Carbonsäureester, Nitrile, Sulfone, Sulfoxide oder halogenierte Kohlenwasserstoffe wie Methylenchlorid.

Vorzugsweise werden Dimethylsulfoxid, Ethylacetat, Acetonitril, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist dadurch gekennzeichnet, daß man Verbindungen der Formel (I-a) jeweils mit Phosphorverbindungen der Formel (VIII) gegebenenfalls in Gegenwart eines Verdünnungsmittels und gegebenenfalls in Gegenwart eines Säurebindemittels umsetzt.

Beim Herstellungsverfahren (F) setzt man zum Erhalt von Verbindungen der Formel (I-a) auf 1 Mol der Verbindungen (I-a), 1 bis 2, vorzugsweise 1 bis 1,3 Mol der Phosphorverbindung der Formel (VIII) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Ether, Amide, Ketone, Carbonsäureester, Nitrile, Alkohole, Sulfide, Sulfone, Sulfoxide etc.

Vorzugsweise werden Acetonitril, Dimethylsulfoxid, Ethylacetat, Tetrahydrofuran, Dimethylformamid oder Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (G) ist dadurch gekennzeichnet, daß man Verbindungen der Formel (I-a) mit Metallhydroxiden bzw. Metallalkoxiden der Formel (IX) oder Aminen der Formel (X), gegebenenfalls in Gegenwart eines Verdünnungsmittels, umsetzt.

Als Verdünnungsmittel können bei dem erfindungsgemäßen Verfahren (G) vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (G) wird im allgemeinen unter Normaldruck durchgerührt. Die Reaktionstemperaturen liegen im allgemeinen zwischen -20°C und 100°C, vorzugsweise zwischen 0°C und 50°C.

Beim Herstellungsverfahren (H) setzt man pro Mol Ausgangsverbindung der Formel (I-a) ca. 1 Mol Carbamidsäurechlorid der Formel (XI) bei 0 bis 150°C, vorzugsweise bei 20 bis 100°C um.

Als gegebenenfalls zugesetzte Verdünnungsmittel kommen alle inerten polaren organischen Lösungsmittel in Frage wie Ether, Amide, Ketone, Carbonsäureester, Sulfone, Sulfoxide oder halogenierte Kohlenwasserstoffe.

Vorzugsweise werden Dimethylsulfoxid, Tetrahydrofuran, Ethylacetat, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin und Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.
Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) und gegen die Raupen der Kohlschabe (Plutella maculipennis) einsetzen.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Sachharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindunngen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfrucht-und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

### Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Didoran, Diethofencarb, Difenoconazol, Dimethirimol, Dimethomorph, Diniconazol, Dinocap., Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Inninoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Ntrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin, Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Primiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

### Herbizide:

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cydoxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.
Die erfindungsgemäßen Wirkstoffe wirken nicht nur gegen Pflanzen-, Hygiene-und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Omithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Boophilus microplus.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

### Hautflügler wie

Sirex juvencus, Urocerus gigas, Urocerus gigas taignus, Urocerus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus. Borstenschwänze
wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe gehen aus den nachfolgenden Beispielen hervor.

### Herstellungsbeispiele

### Beispiel (I-a-1)

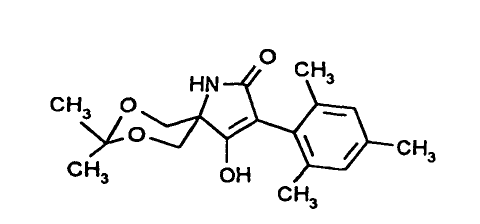

Zu 10 g (0,2 Mol) Kalium-tert.-butylat in 50 ml wasserfreiem Tetrahydrofuran (THF) tropft man bei Rückflußtemperatur 12,5 g (0,0764 Mol) der Verbindung gemäß Beispiel (II-1) in 100 ml wasserfreiem Toluol und rührt 1,5 Stunden unter Rückfluß. Zur Aufarbeitung gibt man 100 ml Wasser zu, trennt die wäßrige Phase ab, extrahiert die Toluolphase mit 50 ml Wasser, vereinigt die wäßrigen Phasen, wäscht sie mit Toluol und säuert bei 10 bis 20°C mit konz. HCl auf pH 6 an. Man extrahiert drei mal mit Methylenchlorid, trocknet, engt ein und chromatographiert den Rückstand an Kieselgel mit Hexan/Aceton 7:3.
Ausbeute: 5,6 g (46 % der Theorie), Fp.: 222-224°C.

Analog bzw. gemäß den allgemeinen Angaben zur Herstellung erhält man die folgenden Verbindungen der Formel (I-a):

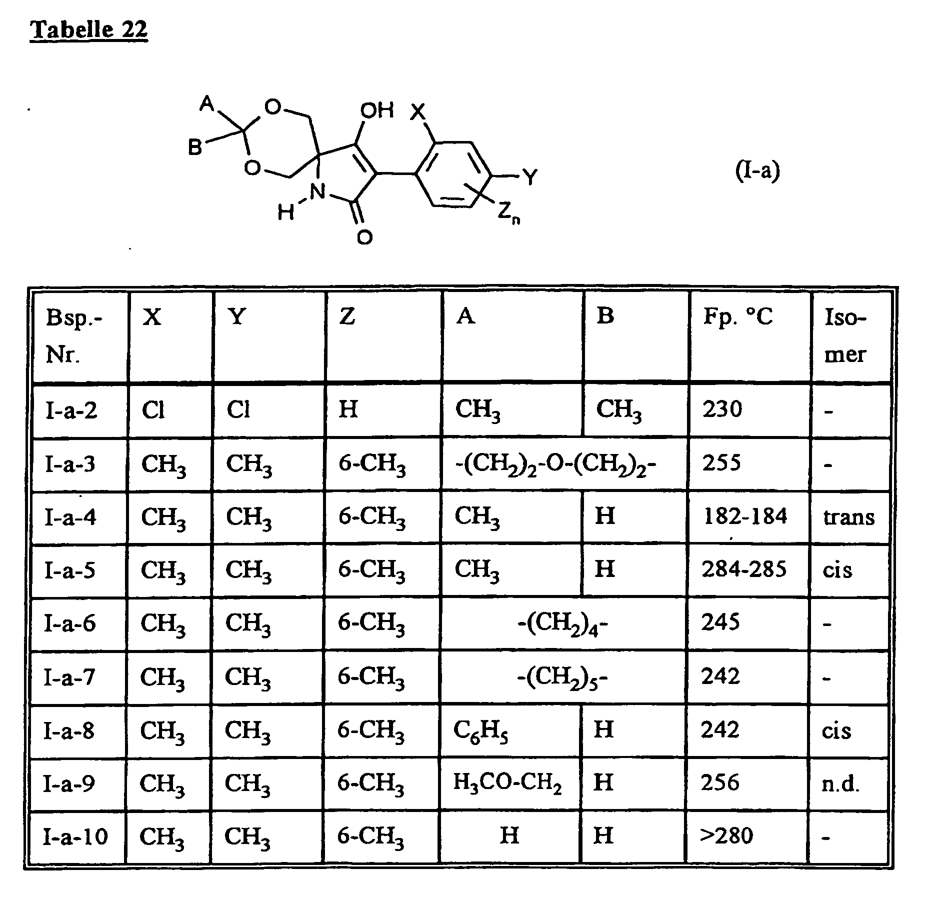

### Beispiel (I-b-1)

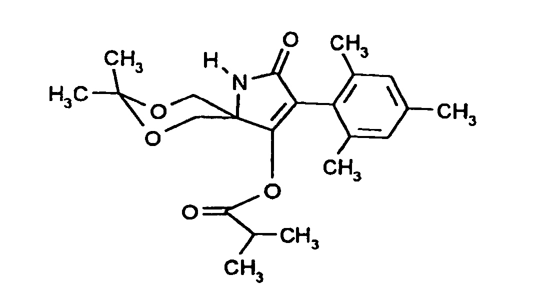

1,2 g (3,75 mMol) der Verbindung gemäß Beispiel (I-a-1) und 1,1 ml (7,5 mmol) Triethylamin in 50 ml wasserfreiem Methylenchlorid werden bei 0 bis 10°C mit 0,8 ml (7,5 mMol) Isobuttersäurechlorid in 5 ml wasserfreiem Methylenchlorid versetzt. Bei Raumtemperatur wird gerührt, bis die Reaktion nach dünnschichtchromatographischer Kontrolle beendet ist. Zur Aufarbeitung wird zwei mal mit 50 ml 0,5 N Natronlauge gewaschen, über Magnesiumsulfat getrocknet und eingedampft. Das Rohprodukt wird mit n-Hexan/Aceton 7:3 an Kieselgel chromatographiert.
Ausbeute: 0,45 g (31 % der Theorie), Fp.: 153-155°C.

Analog bzw. gemäß den allgemeinen Angaben zur Herstellung erhält man die folgenden Verbindungen der Formel (I-b-1):

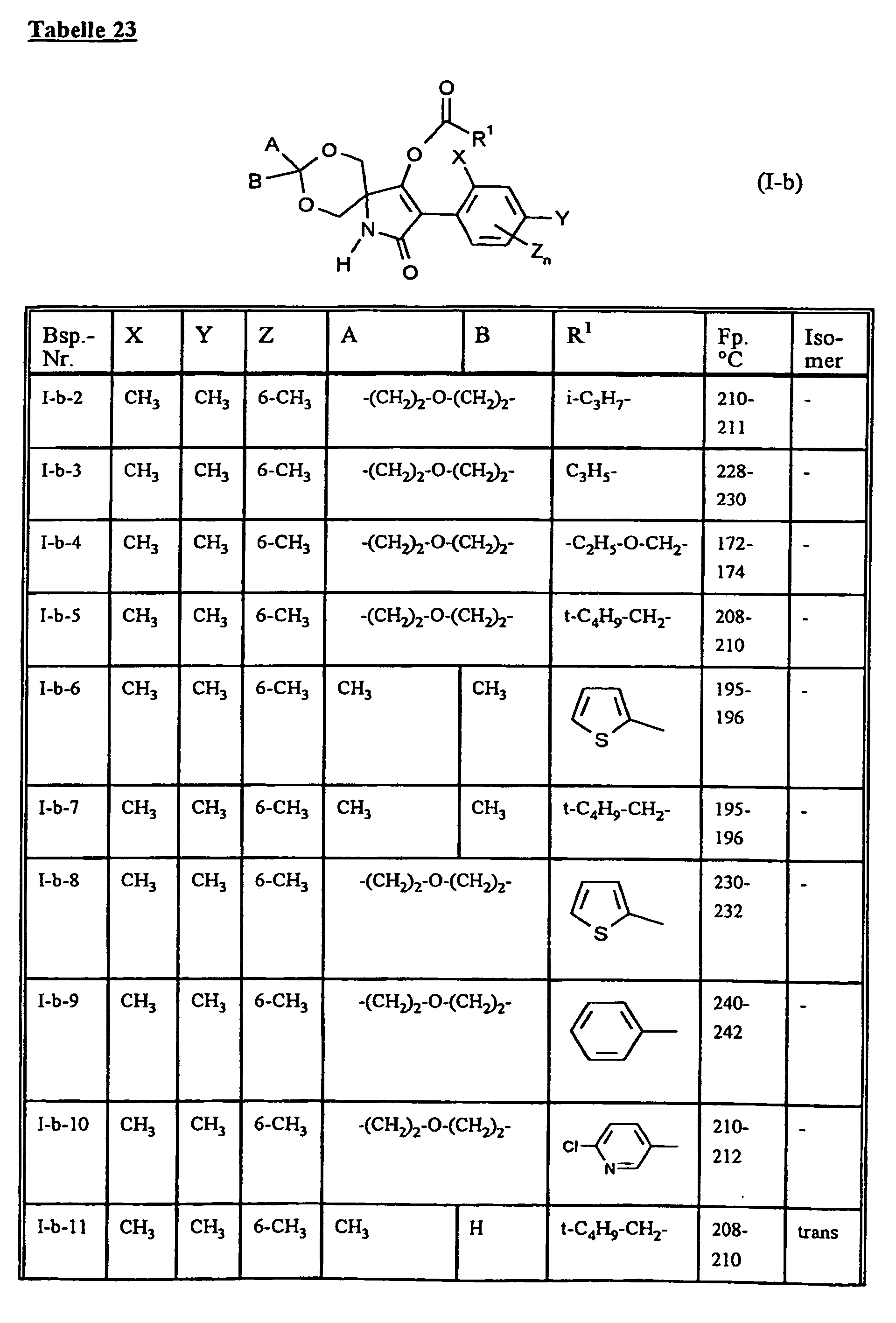

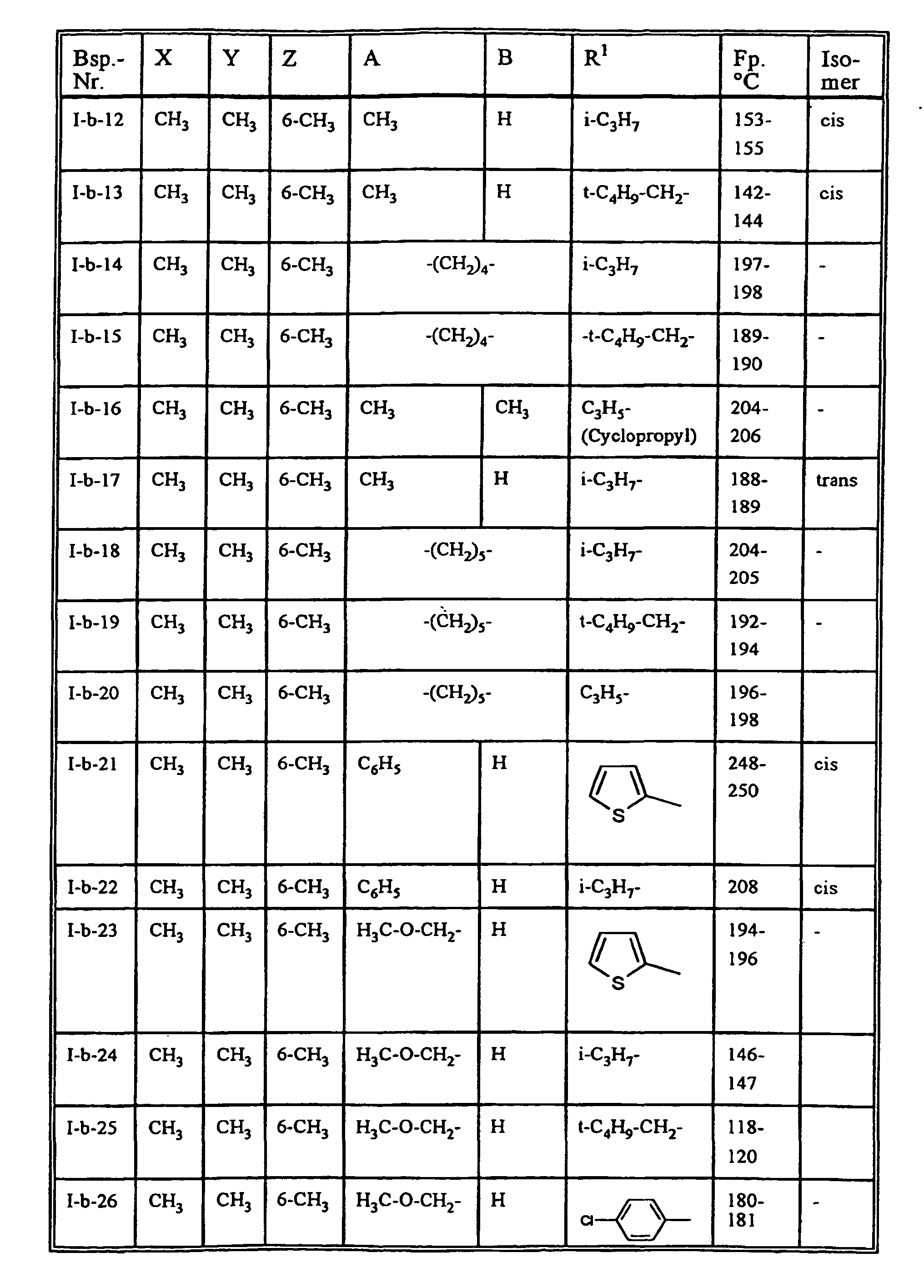

### Beispiel (I-c-1)

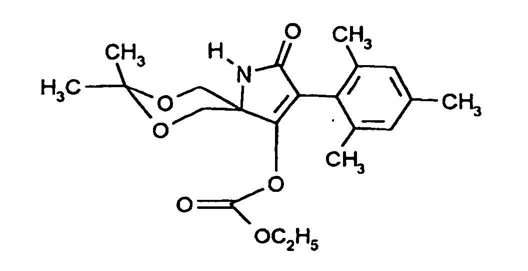

Zu 1,2 g (3,75 mMol) der Verbindung gemäß Beispiel (I-a-1) und 0,8 ml (5,5 mmol) Triethylamin in 50 ml wasserfreiem CH₂Cl₂ tropft man bei 0 bis 10°C 0,55 ml (5 mMol) Chlorameisensäureethylester in 5 ml wasserfreiem Methylenchlorid und rührt bei Raumtemperatur, bis die Reaktion nach dünnschichtchromatographischer Kontrolle beendet ist. Zur Aufarbeitung wird zwei mal mit 50 ml 0,5 N Natronlauge gewaschen, über Magnesiumsulfat getrocknet und eingedampft. Der Rückstand wird mit Hexan/Aceton 7:3 an Kieselgel chromatographiert.
Ausbeute: 0,55 g (38 % der Theorie), Fp.: 138-140°C.

Analog bzw. gemäß den allgemeinen Angaben zur Herstellung erhält man die folgenden Verbindungen der Formel (I-1-c):

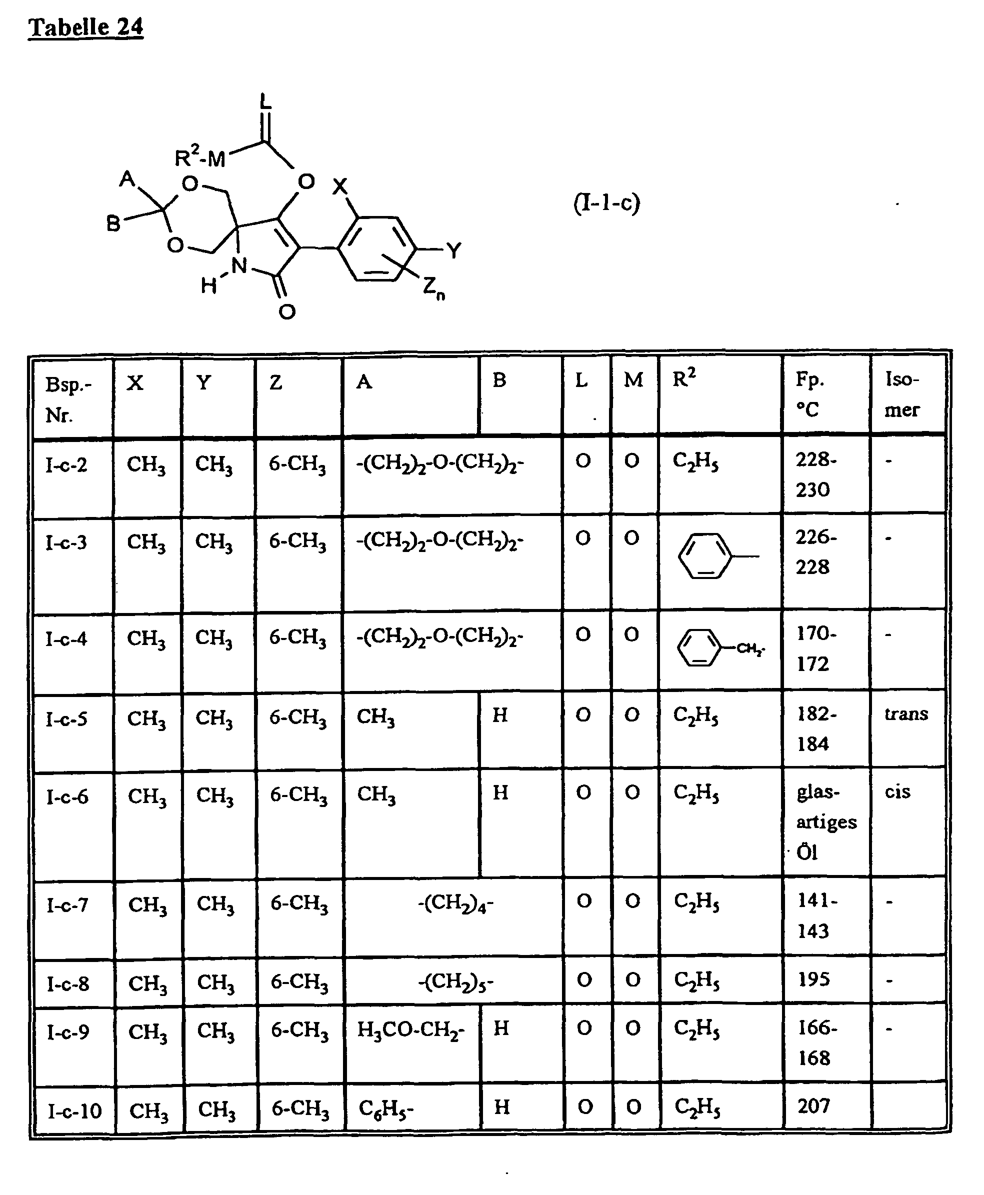

### Beispiel (I-d-1)

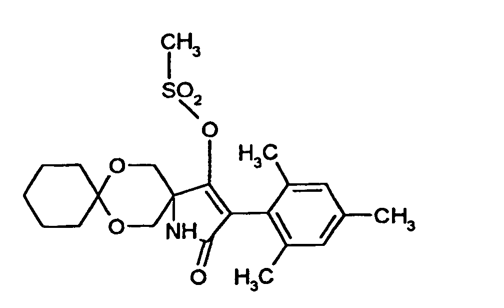

3,6 g der Verbindung gemäß Beispiel (I-a-7) in 50 ml Methylenchlorid und 1,5 ml Triethylamin werden bei 0°C mit 0,8 ml Mesylchlorid in 5 ml Methylenchlorid versetzt und einen Tag ohne Kühlung gerührt. Nach Chromatographie an Kieselgel erhält man 2,50 g (58 % der Theorie), Fp.: 212-214°C.

### Beispiel (I-f-1)

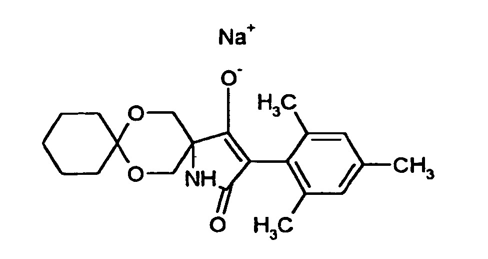

3,6 g der Verbindung gemäß Beispiel (I-a-7) werden in 50ml Methanol bei 20°C mit 2 g Natriummethylatlösung versetzt und einen Tag bei dieser Temperatur gerührt. Dann wird mit Toluol versetzt und im Vakuum eingedampft. Ausbeute: 4,50 g (99 % der Theorie), Fp.: >250°C.

### Beispiel (I-g-1)

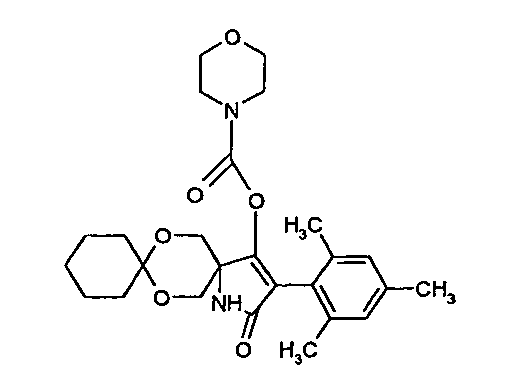

3,6 g der Verbindung gemäß Beispiel (I-a-7) in 50 ml Essigester und 1,5 ml Triethylamin werden bei Rückflußtemperatur mit 1,4 g Morpholincarbamidsäurechlorid in 5 ml Essigester versetzt und noch einen Tag unter Rückfluß erhitzt. Nach Chromatographie an Kieselgel erhält man 0,85 g (18 % der Theorie), Fp.: 190-192°C.

### Beispiel (II-1)

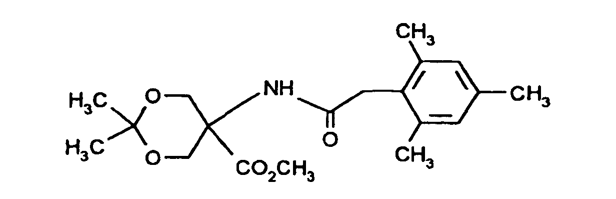

102 g (0,63 Mol) der Verbindung gemäß Beispiel (XV-1) wurden in 1200 ml Methylenchlorid unter Argon mit 54 g Acetoncyanhydrin und 10,5 ml Triethylamin versetzt und einen Tag bei 20°C gerührt und eingedampft (Reagenz A). 46,5 g Oxalylchlorid wurden in 600 ml Methylenchlorid vorgelegt. Dazu tropft man 81 g Dimethylsulfoxid in 210 ml Methylenchlorid bei -78°C. Nach 15 Minuten wird das Reagenz A (Aldehydcyanhydrin) gelöst in 450 ml Methylenchlorid bei -78°C zugetropft, 30 Minuten bei -78°C und 30 Minuten bei -25°C nachgerührt. Es wird erneut auf -78°C abgekühlt und 230 ml Triethylamin zugetropft. Anschließend wird 10 Minuten bei -78°C gerührt. Nach Aufwärmen auf -25°C wird mit 1050 ml Methanol versetzt und einen Tag bei Raumtemperatur gerührt. Der Ansatz wird auf Wasser gegeben, mit Methylenchlorid extrahiert, getrocknet und eingedampft.

Nach Chromatographie an Kieselgel mit n-Hexan/Aceton 7:3 und Kristallisation mit Methylenchlorid/n-Hexan erhält man 43 g des obengenannten Esters (19 % der Theorie) vom Schmelzpunkt 106°C.

Analog zu Beispiel (II-1) und gemäß den allgemeinen Angaben zur Herstellung werden die folgenden Verbindungen der Formel (II) hergestellt.

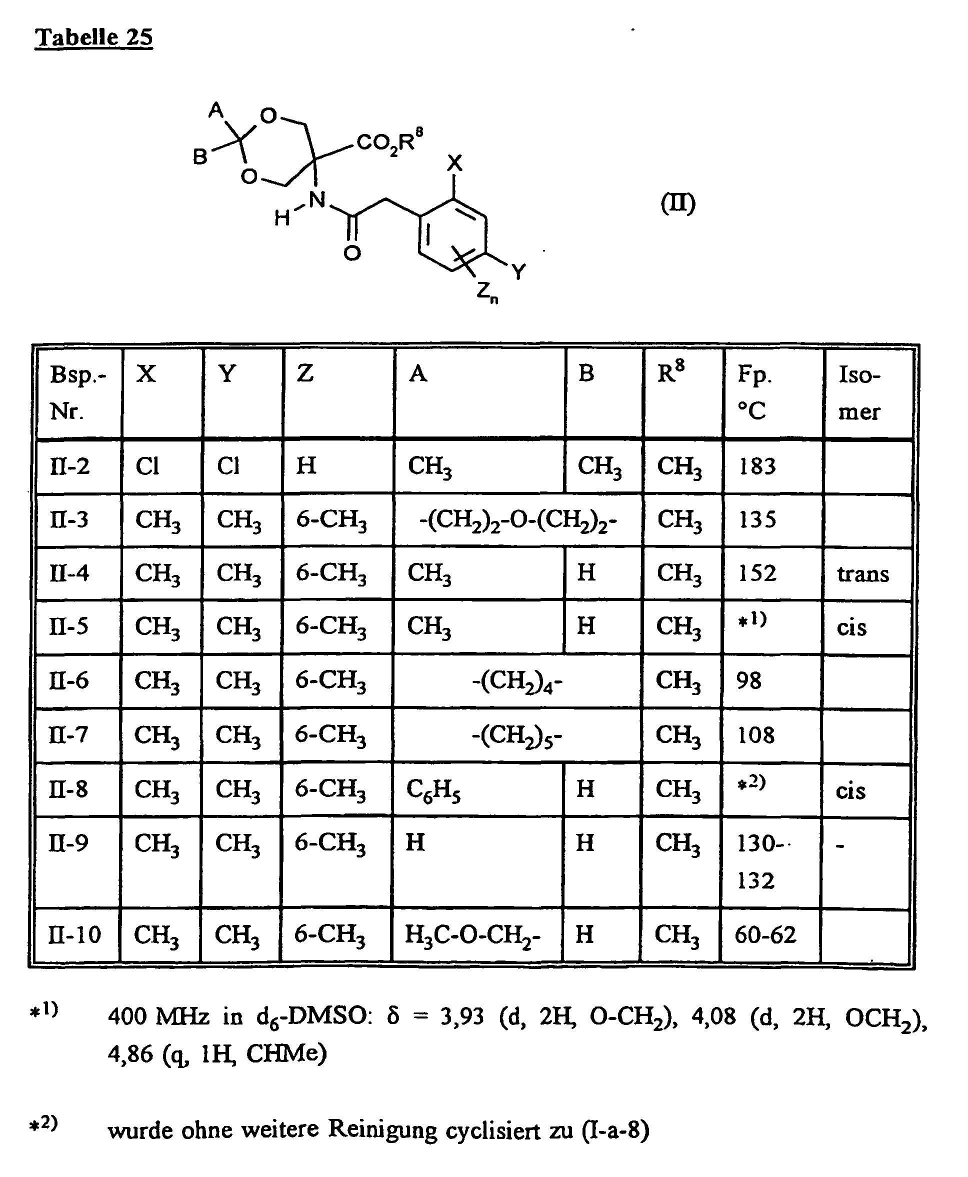

### Beispiel (XV-1)

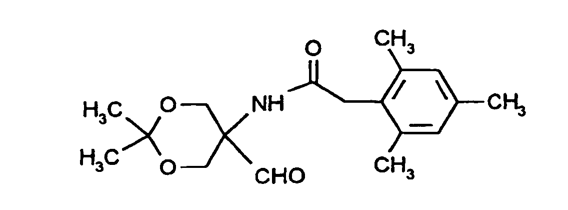

Zu 20 ml Oxalylchlorid in 1000 ml wasserfreiem Tetrahydrofuran tropft man bei -70°C 19 ml DMSO, rührt 3 Minuten bei -35°C nach, kühlt erneut auf -70°C und tropft 65 g (0,25 Mol) der Verbindung gemäß Beispiel XVI-1 in 300 ml wasserfreiem Tetrahydrofuran innerhalb einer Stunde zu. Man rührt 15 Minuten bei -35°C nach, tropft 250 ml Triethylamin zu und rührt einen Tag bei Raumtemperatur. Es wird mit n-Hexan versetzt, der Niederschlag abfiltriert, das Filtrat einrotiert und der Rückstand an Kieselgel mit Methylenchlorid/Aceton 2:1 chromatographiert.
Ausbeute: 44,5 g ( 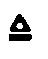 67 % der Theorie) vom Schmp. 110°C

Analog zu Beispiel (XV-1) und gemäß der allgemeinen Angaben wurden die folgenden Verbindungen der Formel (XV) hergestellt.

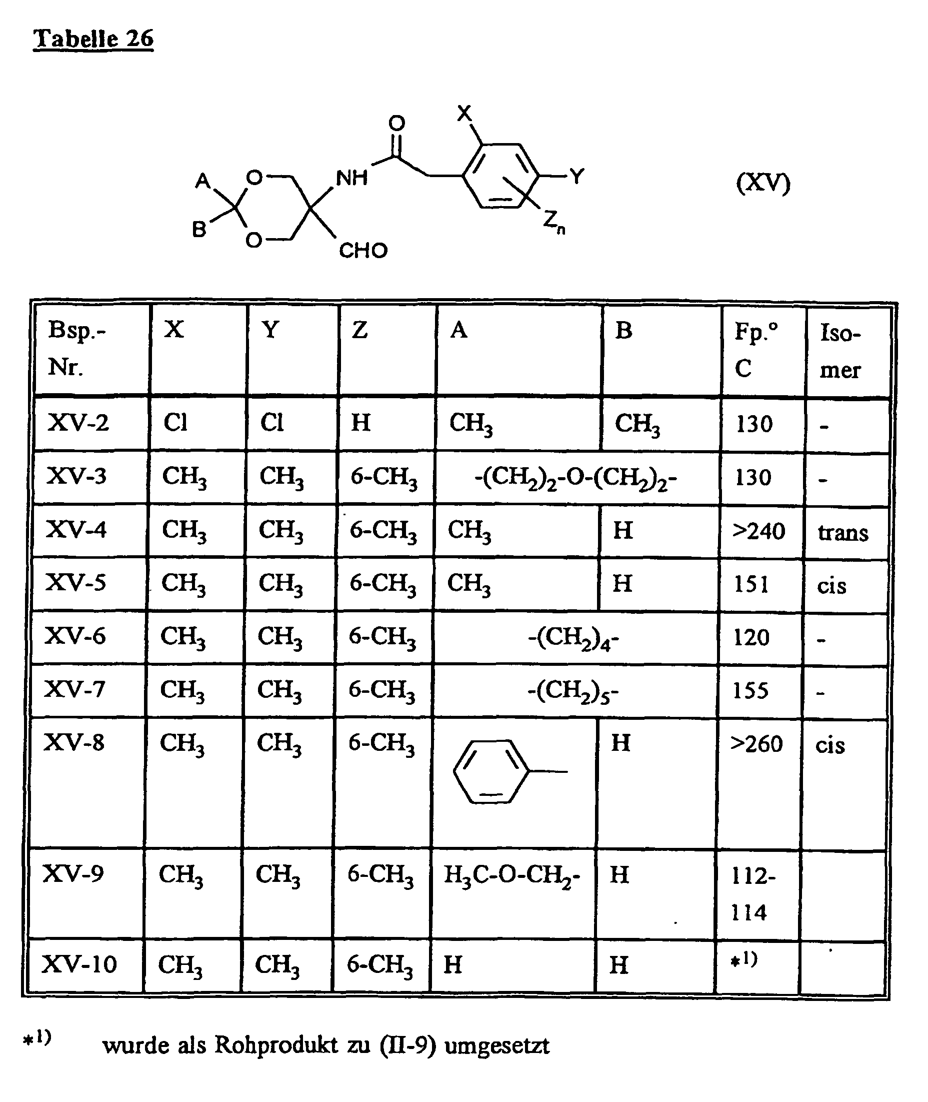

### Beispiel (XVI-1)

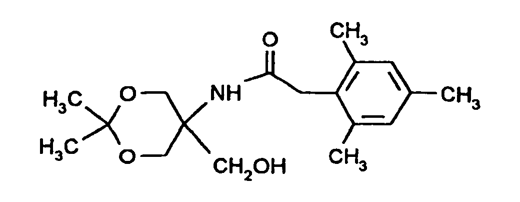

45 g (0,25 Mol) der Verbindung gemäß Beispiel XVII-1 werden in 500 ml wasserfreiem Tetrahydrofuran gelöst, mit 40 ml Triethylamin versetzt und 50 g Misitylenessigsäurechlorid bei 0°C zugetropft und rührt 1 h bei Raumtemperatur. Der Niederschlag wird abfiltriert, das Filtrat eingedampft und der Rückstand an Kieselgel mit Hexan/Essigester 2:1 chromatographiert.
Ausbeute: 66 g ( 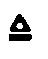 82 % der Theorie) vom Schmelzpunkt 94°C

In Analogie zu Beispiel XVI-1 und gemäß der allgemeinen Angaben wurden die folgenden Verbindungen der Formel XVI hergestellt.

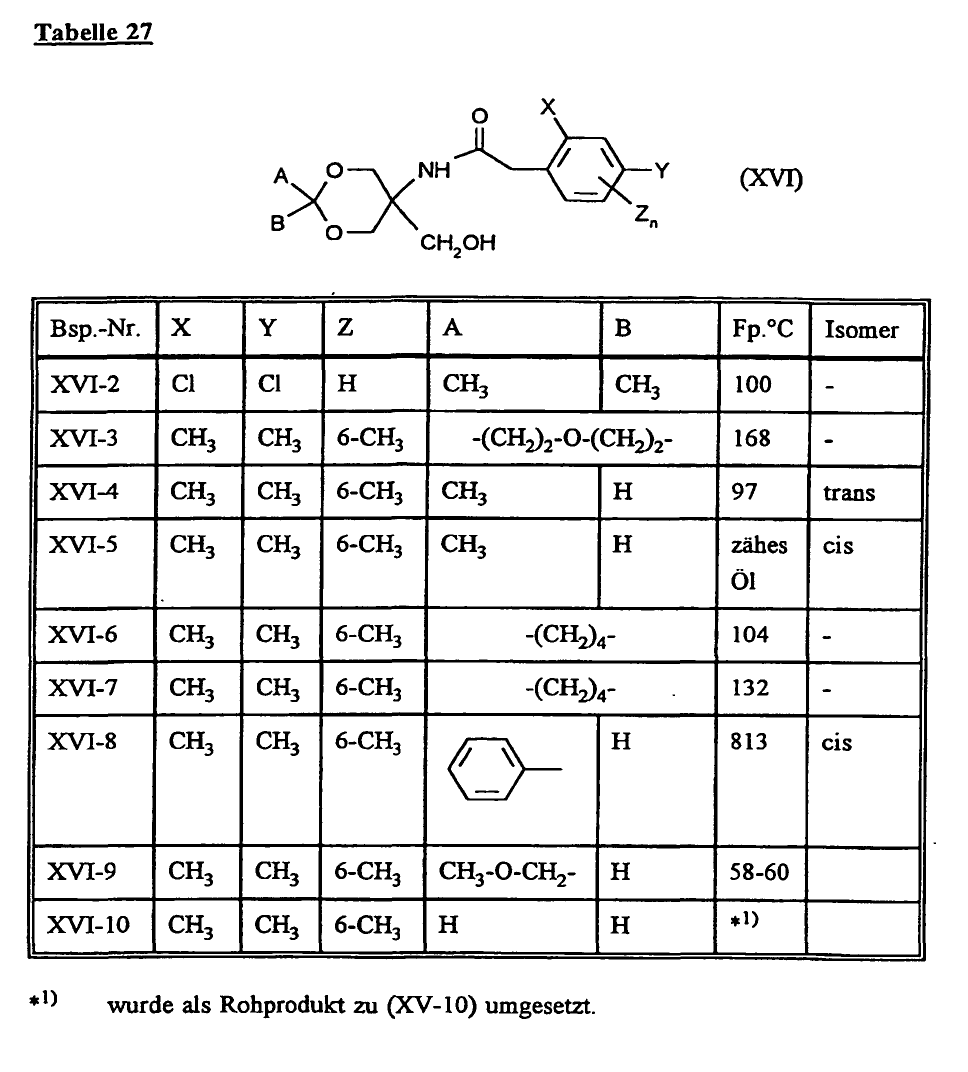

### Beispiel (XVII-1)

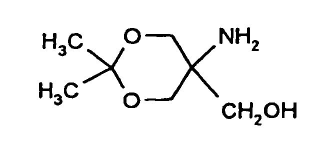

13,5 g (0,07 Mol) 5-Amino-2,2-dimethyl-5-hydroxymethyl-1,3-dioxan werden in 100 ml Ethanol in Gegenwart von 2 g Raney-Nickel mit Wasserstoff bei 20 bar und 40°C im Autoklaven hydriert. Der Katalysator wird abfiltriert, das Filtrat eingeengt, der Rückstand in Methylenchlorid aufgenommen und das Produkt mit n-Hexan gefällt.
Ausbeute: 10,3 g (= 91 % der Theorie)
¹H-NMR (200 MHz in d₆-DMSO): δ = 1,28 (s, 3H, CH₃), 1,32 (s, 3H, CH₃), 3,35 (s, 2H, CH₂OH), 3,4 (d, 2H, OCH₂), 3,57 (d, 2R, OCH₂)

### Beispiel (XVIII-1)

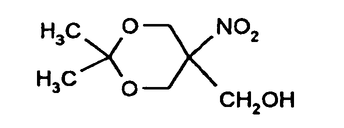

100 g 2,2-Dimethoxypropan werden in 1000 ml Aceton gelöst, 5 g p-Toluolsulfonsäure zugesetzt, 150 g Trishydroxymethylnitromethan eingetragen und 1 h bei Raumtemperatur gerührt. Nach Zugabe von weiteren 100 g 2,2-Dimethoxypropan wird 4 h auf 40°C erwärmt, der Ansatz eingeengt und der Rückstand an Kieselgel mit Hexan/Aceton 7:3 chromatographiert.
Ausbeute: 75 g (= 39 % der Theorie)
¹H-NMR (200 MHz in d₆-DMSO): δ = 1,23 (s, 3H, CH₃), 1,28 (s, 3H, CH₃), 3,7 (d, 2H, CH₂OH), 4,02 (d, 2H, OCH₂), 4,32 (d, 2H, OCH₂)

Die bei Einsatz von Aldehyden anfallenden cis/trans-Isomerengemische von Verbindungen der Formel XVIII werden durch Kristallisation, chromatographische Methoden oder Destillation in die Isomeren getrennt.

### Anwendungsbeispiele

### Beispiel A

| **Tetranychus-Test (OP-resistent / Tauchbehandlung)** | |
|---|---|
| Lösungsmittel | 7,5 Gewichtsteile Dimethylformamid |
| | 100 Gewichtsteile Methanol |
| Emulgator | 2,5 Gewichtsteile Alkylarylpolyglykolether |

Zur Herstellung einer zweckmäßigen Wirkstoffzubereitung vermischt man 1 Gewichtsteil Wirkstoff mit der angegebenen Menge Emulgator und verdünnt das Konzentrat mit Wasser mit emulgatorhaltigem Wasser auf die gewünschten Konzentrationen.

Bohnenpflanzen (Phaseolus vulgaris), die stark von allen Stadien der gemeinen Spinnmilbe Tetranychus urticae befallen sind, werden in eine Wirkstoffzubereitung der gewünschten Konzentration getaucht.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die Verbindungen gemäß den Herstellungsbeispielen I-a-1, I-b-1 und I-c-1 bei einer beispielhaften Wirkstoffkonzentration von 0,1 % eine Abtötung von ≥ 98 % nach 13 d.

### Beispiel B

| **Phaedon-Larven-Test** | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden, 0 % bedeutet, daß keine Käferlarven abgetötet wurden.

Bei diesem Test zeigen z.B. die Verbindungen gemäß den Herstellungsbeispielen Wirksamkeit I-a-1, I-b-1, I-b-2-, I-b-3, I-a-4 und I-b-10 bei einer beispielhaften Wirkstoffkonzentration von 0,1 % eine Abtötung von 100 % nach 7 Tagen.

### Beispiel C

| **Plutella-Test** | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die Verbindungen gemäß den Herstellungsbeispielen Wirksamkeit I-a-1, 1-b-lund I-c-1 bei einer beispielhaften Wirkstoffkonzentration von 0,1 % eine Abtötung von 100 % nach 7 Tagen.

### Beispiel D

| **Nephotettix-Test** | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryzae sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigen z.B. die Verbindungen gemäß den Herstellungsbeispielen I-a-1, I-b-1, I-c-1, I-b-2, I-c-2, I-a-4, I-b-8 und I-c-4 bei einer beispielhaften Wirkstoffkonzentration von 0,1 % eine Abtötung von 100 % nach 6 Tagen.

### Beispiel E

| **Spodoptera-Test** | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die Verbindungen gemäß den Herstellungsbeispielen I-b-2, I-a-4, I-b-8 und I-b-10 bei einer beispielhaften Wirkstoffkonzentration von 0,1 % eine Abtötung von ≥ 90 % nach 7 Tagen.

### Beispiel F

| **Myzus-Test** | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlpflanzen (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die Verbindungen gemäß den Herstellungsbeispielen I-a-1, I-a-3, I-b-2, I-c-2, I-a-4 und I-c-4 bei einer beispielhaften Wirkstoffkonzentration von 0,1 % eine Abtötung von ≥ 90 % nach 6 Tagen.

## Patentansprüche

1. Verbindungen der Formel (I) 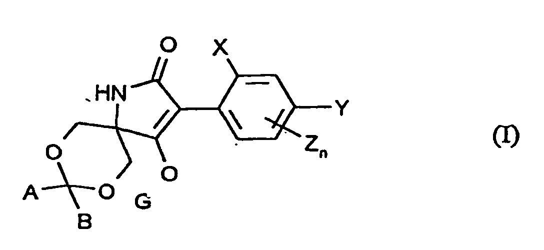 in welcher
X für Halogen, C₁-C₆-Alkyl, C₂-C₆-Alkenyl, C₂-C₆-Alkinyl, C₁-C₆-Alkoxy, C₃-C₆-Alkenyloxy, C₁-C₆-Alkylthio, C₁-C₆-Alkylsulfinyl, C₁-C₆-Alkylsulfonyl, C₁-C₆-Halogenalkyl, C₂-C₆-Halogenalkenyl, C₁-C₆-Halogenalkoxy, C₃-C₆-Halogenalkenyloxy, Nitro, Cyano oder jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Benzyloxy oder Benzylthio steht,
Y für Wasserstoff, Halogen, C₁-C₆-Alkyl, C₂-C₆-Alkenyl, C₂-C₆-Alkinyl, C₁-C₆-Alkoxy, C₃-C₆-Alkenyloxy, C₁-C₆-Alkylthio, C₁-C₆-Alkylsulfinyl, C₁-C₆-Alkylsulfonyl, C₁-C₆-Halogenalkyl, C₂-C₆-Halogenalkenyl, C₁-C₆-Halogenalkoxy, C₃-C₆-Halogenalkenyl- oxy, Nitro oder Cyano steht,
Z für Halogen, C₁-C₆-Alkyl, C₂-C₆-Alkenyl, C₂-C₆-Alkinyl, C₁-C₆-Halogenalkyl, C₂-C₆-Halogenalkenyl, C₁-C₆-Alkoxy, C₃-C₆-Alkenyloxy, C₁-C₆-Halogenalkoxy, C₃-C₆-Halogenalkenyloxy, Nitro oder Cyano steht,
n bevorzugt für 0, 1, 2 oder 3 steht,
A für Wasserstoff oder jeweils gegebenenfalls durch Halogen substituiertes C₁-C₁₂-Alkyl, C₂-C₈-Alkenyl, C₂-C₈-Alkinyl, C₁-C₁₀-Alkoxy-C₁-C₈-alkyl, Poly-C₁-C₈-alkoxy-C₁-C₈-alkyl oder C₁-C₁₀-Alkylthio-C₁-C₆-alkyl, gegebenenfalls durch Halogen, C₁-C₆-Alkyl oder C₁-C₆-Alkoxy substituiertes C₃-C₈-Cycloalkyl, in welchem gegebenenfalls eine Methylengruppe durch Sauerstoff oder Schwefel ersetzt ist oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl oder Naphthyl, Hetaryl mit 5 bis 6 Ringatomen, bevorzugt Furanyl, Pyridyl, Imidazolyl, Triazolyl, Pyrazolyl, Pyrimidyl, Thiazolyl oder Thienyl oder Phenyl- oder Naphthyl-C₁-C₆-alkyl steht, die außerdem durch gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenoxy substituiert sein können,
B für Wasserstoff oder jeweils gegebenenfalls durch Halogen substituiertes C₁-C₁₂-Alkyl oder C₁-C₈-Alkoxy-C₁-C₆-alkyl steht oder
A, B und das Kohlenstoffatom an das sie gebunden sind, für gesättigtes oder ungesättigtes C₃-C₁₀-Cycloalkyl stehen (insbesondere C₅-C₈-Cycloalkyl), in welchem gegebenenfalls eine Methylengruppe durch Sauerstoff oder Schwefel ersetzt ist und welches gegebenenfalls einfach oder mehrfach durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert ist oder
A, B und das Kohlenstoffatom, an das sie gebunden sind, für C₅-C₈-Cycloalkyl oder C₅-C₈-Cycloalkenyl stehen, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₅-C₆-Alkandiyl, C₅-C₆-Alkendiyl oder C₆-Alkandiendiyl stehen, worin gegebenenfalls eine Methylengruppe durch Sauerstoff oder Schwefel ersetzt ist,
G für Wasserstoff (a) oder für eine der Gruppen 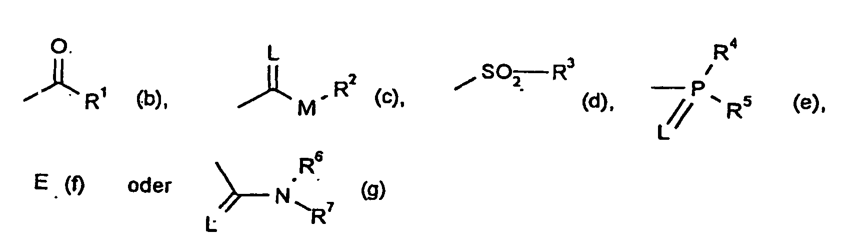
steht,
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls mindestens eine Methylengruppe durch Sauerstoff oder Schwefel ersetzt ist,
für gegebenenfalls durch Halogen, Cyano, Nitro, C₁-C₆-Alkyl, C₁- C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁- C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5-oder 6-gliedriges Hetaryl
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl steht,
R² für jeweils gegebenenfalls durch Halogen substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano; Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio, C₂-C₈-Alkenylthio oder C₃-C₇-Cycloalkylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen und
R⁶ und R⁷ unabhängig voneinander für Wasserstoff, für jeweils gegebenenfalls durch Halogen substituiertes C₁-C₈-Alkyl, C₃-C₈-Cyclo-alkyl, C₁-C₈-Alkoxy, C₃-C₈-Alkenyl oder C₁-C₈-Alkoxy-C₁-C₈-alkyl, für gegebenenfalls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen C₃-C₆-Alkylenrest stehen, in welchem gegebenenfalls eine Methylengruppe durch Sauerstoff oder Schwefel ersetzt ist.

2. Verbindungen der Formel (I) gemäß Anspruch 1, in welcher
X für Fluor, Chlor, Brom, C₁-C₄-Alkyl, C₁-C₄-Alkoxy, C₁-C₄-Halogenalkyl, C₁-C₄-Halogenalkoxy, Nitro, Cyano oder jeweils gegebenenfalls durch Fluor, Chlor, Brom, C₁-C₄-Alkyl, C₁-C₄-Alkoxy; C₁-C₂-Halogenalkyl, C₁-C₂-Halogenalkoxy, Nitro oder Cyano substituiertes Phenyl oder Benzyloxy steht,
Y für Wasserstoff, Fluor, Chlor, Brom, C₁-C₄-Alkyl, C₁-C₄-Alkoxy, C₃-C₄-Alkenyloxy, C₁-C₄-Halogenalkyl, C₁-C₄-Halogenalkoxy, Nitro oder Cyano steht,
Z für Fluor, Chlor, Brom, C₁-C₄-Alkyl, C₁-C₄-Halogenalkyl, C₁-C₄-Alkoxy, C₁-C₄-Halogenalkoxy, Nitro oder Cyano steht,
n für 0, 1, 2 oder 3 steht,
A für Wasserstoff oder jeweils gegebenenfalls durch Fluor oder Chlor substituiertes C₁-C₁₀-Alkyl oder C₁-C₈-Alkoxy-C₁-C₆-alkyl, gegebenenfalls durch Fluor, Chlor, C₁-C₄-Alkyl oder C₁-C₄-Alkoxy substituiertes C₃-C₇-Cycloalky, oder jeweils gegebenenfalls durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl, Furanyl, Pyridyl, Imidazolyl, Triazolyl, Pyrazolyl, Pyrimidyl, Thiazolyl, Thienyl oder Phenyl-C₁-C₄-alkyl steht,
B für Wasserstoff oder jeweils gegebenenfalls durch Fluor oder Chlor substituiertes C₁-C₁₀-Alkyl oder C₁-C₆-Alkoxy-C₁-C₄-alkyl steht oder
A, B und das Kohlenstoffatom an das sie gebunden sind, für gesättigtes oder ungesättigtes C₅-C₈-Cycloalkyl stehen, in welchem gegebenenfalls eine Methylengruppe durch Sauerstoff oder Schwefel ersetzt ist und welches gegebenenfalls durch C₁-C₆-Alkyl, C₃-C₈-Cycloalkyl, C₁-C₃-Halogenalkyl, C₁-C₆-Alkoxy, C₁-C₆-Alkylthio, Fluor, Chlor oder Phenyl substituiert ist oder
A, B und das Kohlenstoffatom, an das sie gebunden sind, für C₅-C₆-Cycloalkyl oder C₅-C₆-Cycloalkenyl stehen, in welchen zwei Substituenten gemeinsam mit den Kohlenstoffatomen, an die sie gebunden sind, für jeweils gegebenenfalls durch C₁-C₅-Alkyl, C₁-C₅-Alkoxy, Fluor, Chlor oder Brom substituiertes C₅-C₆-Alkandiyl, C₅-C₆-Alkendiyl oder C₆-Alkandiendiyl stehen, worin gegebenenfalls eine Methylengruppe durch Sauerstoff oder Schwefel ersetzt ist,
G für Wasserstoff (a) oder eine der Gruppen 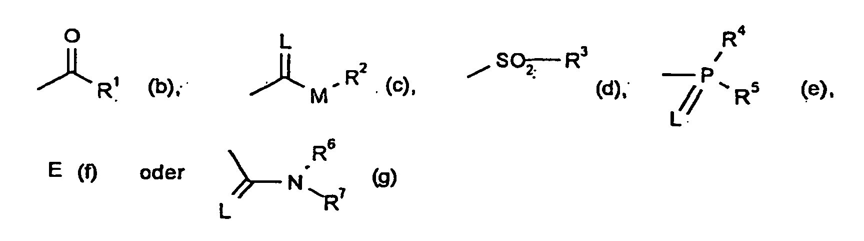
steht,
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₆-Alkyl, C₂-C₁₆-Alkenyl, C₁-C₆-Alkoxy-C₁-C₆-alkyl, C₁-C6-Alkylthio-C₁-C₆-alkyl oder Poly-C₁-C₆-alkoxy-C₁-C₆-alkyl oder gegebenenfalls durch Fluor, Chlor, C₁-C₅-Alkyl oder C₁-C₅-Alkoxy substituiertes C₃-C₇-Cycloalkyl, in welchem gegebenenfalls ein oder zwei nicht direkt benachbarte Methylengruppen durch Sauerstoff oder Schwefel ersetzt sind, für gegebenenfalls durch Fluor, Chlor, Brom, Cyano, Nitro, C₁-C₄Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls durch Fluor, Chlor, Brom oder C₁-C₄-Alkyl substituiertes Phenoxy-C₁-C₅-alkyl oder
für jeweils gegebenenfalls durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl steht,
R² für jeweils gegebenenfalls durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl steht,
R³ für gegebenenfalls durch Fluor oder Chlor substituiertes C₁-C₆-Alkyl oder für jeweils gegebenenfalls durch Fluor, Chlor, Brom, C₁-C₅-Alkyl, C₁-C₅-Alkoxy, C₁-C₃-Halogenalkyl, C₁-C₃-Halogenalkoxy, Cyano oder Nitro substituiertes Phenyl oder Benzyl steht,
R⁴ und R⁵ unabhängig voneinander für jeweils gegebenenfalls durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio oder C₃-C₆-Cycloalkylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio stehen und
R⁶ und R⁷ unabhängig voneinander für Wasserstoff, für jeweils gegebenenfalls durch Halogen substituiertes C₁-C₆-Alkyl, C₃-C₆-Cyclo-alkyl, C₁-C₆-Alkoxy, C₃-C₆-Alkenyl oder C₁-C₆-Alkoxy-C₁-C₆-alkyl, für gegebenenfalls durch Halogen, C₁-C₅-Halogenalkyl, C₁-C₅-Alkyl oder C₁-C₅-Alkoxy substituiertes Phenyl, für gegebenenfalls durch Halogen, C₁-C₅-Alkyl, C₁-C₅-Halogenalkyl oder C₁-C₅-Alkoxy substituiertes Benzyl, oder zusammen für einen C₃-C₆-Alkylenrest stehen, in welchem gegebenenfalls eine Methylengruppe durch Sauerstoff oder Schwefel ersetzt ist.

3. Verbindungen der Formel (I) gemäß Anspruch 1, in welcher
X für Fluor, Chlor, Brom, Methyl, Ethyl, Propyl, iso-Propyl, Methoxy, Ethoxy, Propoxy, iso-Propoxy, Trifluormethyl, Difluormethoxy, Trifluormethoxy, Nitro oder Cyano steht,
Y für Wasserstoff, Fluor, Chlor, Brom, Methyl, Ethyl, n-Propyl, i-Propyl, n-Butyl, i-Butyl, tert.-Butyl, Methoxy, Ethoxy, Propoxy, iso-Propoxy, Allyloxy, Methallyloxy, Trifluormethyl, Difluormethoxy, Trifluormethoxy, Nitro oder Cyano steht,
Z für Fluor, Chlor, Brom, Methyl, Ethyl, n-Propyl, i-Propyl, n-Butyl, i-Butyl, tert.-Butyl, Methoxy, Ethoxy, Propoxy, iso-Propoxy, Difluormethoxy, Trifluormethyl, Trifluormethoxy, Nitro oder Cyano steht,
n für 0, 1, 2 oder 3 steht,
A für Wasserstoff oder jeweils gegebenenfalls durch Fluor oder Chlor substituiertes C₁-C₈-Alkyl oder C₁-C₆-Alkoxy-C₁-C₄-alkyl, gegebenenfalls durch Fluor, Chlor, Methyl, Ethyl, iso-Propyl, tert.-Butyl, Ethoxy oder Methoxy substituiertes C₅-C₆-Cycloalkyl, oder für jeweils gegebenenfalls durch Fluor, Chlor, Brom, Methyl, Ethyl, iso-Propyl, tert.-Butyl, Methoxy, Ethoxy, Trifluormethyl, Difluor- methoxy, Trifluormethoxy, Cyano oder Nitro substituiertes Phenyl oder Benzyl steht,
B für Wasserstoff oder jeweils gegebenenfalls durch Fluor substituiertes C₁-C₈-Alkyl oder C₁-C₄-Alkoxy-C₁-C₂-alkyl steht oder
A, B und das Kohlenstoffatom an das sie gebunden sind, für gesättigtes oder ungesättigtes C₅-C₆-Cycloalkyl stehen, in welchem gegebenenfalls eine Methylengruppe durch Sauerstoff oder Schwefel ersetzt ist und welches gegebenenfalls durch Methyl, Ethyl, Isopropyl, tert.-Butyl, Trifluormethyl, Methoxy, Ethoxy, Fluor, Chlor oder Phenyl substituiert ist oder
A, B und das Kohlenstoffatom, an das sie gebunden sind, für C₅-C₆-Cycloalkyl oder C₅-C₆-Cycloalkenyl stehen, in dem zwei Substituenten gemeinsam mit den Kohlenstoffatomen, an die sie gebunden sind, für C₅-C₆-Alkandiyl, C₅-C₆-Alkendiyl oder C₆-Alkandiendiyl stehen,
G für Wasserstoff (a) oder für eine der Gruppen 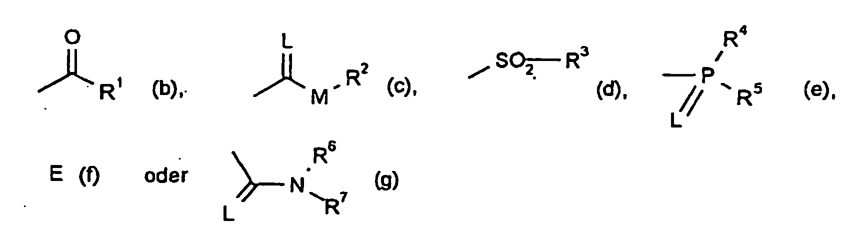
steht,
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl oder Poly-C₁-C₄-alkoxy-C₁-C₄-alkyl oder gegebenenfalls durch Fluor, Chlor, Methyl, Ethyl, Propyl, i-Propyl, Butyl, i-Butyl, tert.-Butyl, Methoxy, Ethoxy, Propoxy oder iso-Propoxy substituiertes C₃-C₆-Cycloalkyl, in welchem gegebenenfalls eine oder zwei nicht direkt benachbarte Methylengruppen durch Sauerstoff oder Schwefel ersetzt sind,
für gegebenenfalls durch Fluor, Chlor, Brom, Cyano, Nitro, Methyl, Ethyl, Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl, Pyridyl, Pyrimidyl, Thiazolyl oder Pyrazolyl,
für gegebenenfalls durch Fluor, Chlor, Methyl oder Ethyl substituiertes Phenoxy-C₁-C₄-alkyl oder
für jeweils gegebenenfalls durch Fluor, Chlor, Amino, Methyl oder Ethyl substituiertes Pyridyloxy-C₁-C₄-alkyl, Pyrimidyloxy-C₁-C₄-alkyl oder Thiaiolyloxy-C₁-C₄-alkyl steht,
R² für jeweils gegebenenfalls durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, Propyl, iso-Propyl oder Methoxy substituiertes C₃-C₆-Cycloalkyl, oder für jeweils gegebenenfalls durch Fluor, Chlor, Cyano, Nitro, Methyl, Ethyl, Propyl, i-Propyl, Methoxy, Ethoxy, Trifluormethyl oder Trifluormethoxy substituiertes Phenyl oder Benzyl steht,
R³ für jeweils gegebenenfalls durch Fluor oder Chlor substituiertes Methyl, Ethyl, Propyl, Isopropyl oder jeweils gegebenenfalls durch Fluor, Chlor, Brom, Methyl, Ethyl, Propyl, iso-Propyl, tert.-Butyl, Methoxy, Ethoxy, Isopropoxy, tert.-Butoxy, Trifluormethyl, Trifluormethoxy, Cyano oder Nitro substituiertes Phenyl oder Benzyl steht,
R⁴ und R⁵ unabhängig voneinander für jeweils gegebenenfalls durch Fluor oder Chlor substituiertes C₁-C₄-Alkyl, C₁-C₄-Alkoxy, C₁-C₄-Alkylamino, Di-(C₁-C₄-alkyl)amino, C₁-C₄-Alkylthio oder für jeweils gegebenenfalls durch Fluor, Chlor, Brom, Nitro, Cyano, C₁-C₂-Alkoxy, C₁-C₂-Fluoralkoxy, C₁-C₂-Alkylthio, C₁-C₂-Fluoralkylthio oder C₁-C₃-Alkyl substituiertes Phenyl, Phenoxy oder Phenylthio stehen und
R⁶ und R⁷ unabhängig voneinander für Wasserstoff, für jeweils gegebenenfalls durch Fluor oder Chlor substituiertes C₁-C₄-Alkyl, C₃-C₆-Cycloalkyl, C₁-C₄-Alkoxy, C₃-C₄-Alkenyl oder C₁-C₄-Alkoxy-C₁-C₄-alkyl, für gegebenenfalls durch Fluor, Chlor, Brom, C₁-C₄-Halogenalkyl, C₁-C₄-Alkyl oder C₁-C₄-Alkoxy substituiertes Phenyl, für gegebenenfalls durch Fluor, Chlor, Brom, C₁-C₄-Alkyl, C₁-C₄-Halogenalkyl oder C₁-C₄-Alkoxy substituiertes Benzyl, oder zusammen für einen C₅-C₆-Alkylenrest stehen, in welchem gegebenenfalls eine Methylengruppe durch Sauerstoff oder Schwefel ersetzt ist.

4. Verfahren zur Herstellung von Verbindungen der Formel (I) gemäß Anspruch I, **dadurch gekennzeichnet, daß** man zum Erhalt von bitte einfügen
(A) Verbindungen der Formel (I-a) 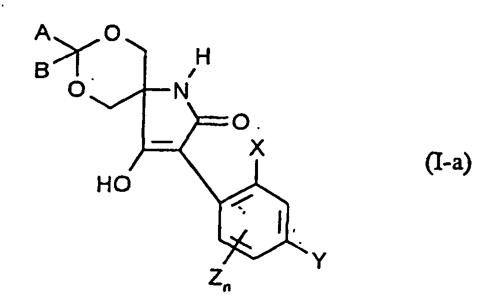 in welcher
A, B, X, Y, Z und n die in Anspruch 1 angegebenen Bedeutungen haben,
N-Acylaminosäureester der Formel (II) 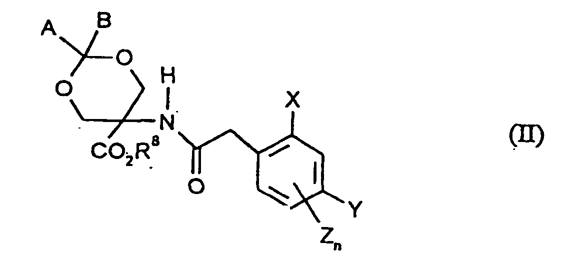 in welcher
A, B, X, Y, Z und n die in Ansprüch 1 angegebenen Bedeutungen haben,
und
R⁸ für Alkyl steht,
in Gegenwart eines Verdünnungsmittels und in Gegenwart einer Base intramolekular kondensiert,
(B)
α) Verbindungen der Formel (I) in welcher A, B, X, Y, Z, R¹ und n die in Ansprüch 1 angegebenen Bedeutungen haben, und G für die Grüppe (b) steht Verbindungen der Formel (I-a), in welcher A, B, X, Y, Z und n die in Auspruüch 1 angegebenen Bedeutungen haben, mit Säurehalogeniden der Formel (III) 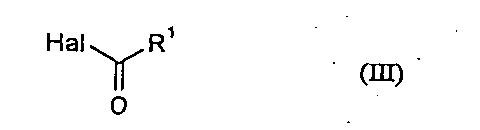 in welcher
R¹ die in Ansprüch 1 angegebene Bedeutung hat und
Hal für Halogen steht
oder
β) mit Carbonsäureanhydriden der Formel (IV)
R¹-CO-O-CO-R¹ (IV)
in welcher
R¹ die in Ansprüch 1 angegebene Bedeutung hat,
gegebenenfalls in Gegenwart eines Verdünnungsmittels und gegebenenfalls in Gegenwart eines Säurebindemittels umsetzt;
(C) Verbindungen der Formel (I), in welcher A, B, R², M, X, Y, Z und n die in Ansprüch 1 angegebenen Bedeutungen haben und L für Sauerstoff steht, und G für die Grüppe (c) steht, Verbindungen der Formel (I-a), in welcher A, B, X, Y, Z und n die in Ansprüch 1 angegebenen Bedeutungen haben, jeweils. mit Chlorameisensäureestern oder Chlorameisensäurethiolestern der Formel (V)
R²-M-CO-Cl (V)
in welcher
R² und M die in Ansprüch 1 angegebenen Bedeutungen haben,
gegebenenfalls in Gegenwart eines Verdünnungsmittels und gegebenenfalls in Gegenwart eines Säurebindemittels umsetzt;
(D) Verbindungen der Formel (I), in welcher A, B, R², M, X, Y, Z und n die in Ansprüch 1 angegebenen Bedeutungen haben und L für Schwefel steht, sind G für die Gruppe (c) steht Verbindungen der Formel (I-a), in welcher A, B, X, Y, Z und n die in Ansprüch 1 angegebenen Bedeutungen haben, jeweils
mit Chlormonothioameisensäureestern oder Chlordithioameisensäureestem der Formel (VI) 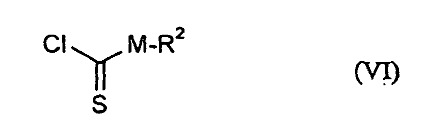 in welcher
M und R² die in Ansprüch 1 angegebenen Bedeutungen haben,
gegebenenfalls in Gegenwart eines Verdünnungsmittels und gegebenenfalls in Gegenwart eines Säurebindemittels umsetzt,
(E) Verbindungen der Formel (I), in welcher A, B, R³, X, Y, Z und n die in Ansprüch 1 angegebenen Bedeutungen haben, und G für die Grüppe (d) steht Verbindungen der Formeln (I-a), in welcher A, B, X, Y, Z und n die in Ansprüch 1 angegebenen Bedeutungen haben, jeweils
mit Sulfonsäurechloriden der Formel (VII)
R³-SO₂-Cl (VII)
in welcher
R³ die in Ansprüch 1 angegebene Bedeutung hat,
gegebenenfalls in Gegenwart eines Verdünnungsmittels und gegebenenfalls in Gegenwart eines Säurebindemittels umsetzt,
(F) Verbindungen der Formel (I), in welcher A, B, L, R⁴, R⁵, X, Y, Z und n die in Ansprüch 1 angegebenen Bedeutungen haben, und G für die Grüppe (e) steht, Verbindungen der Formeln (I-a), in welcher A, B, X, Y, Z und n die in Ansprüch 1 angegebenen Bedeutungen haben, jeweils
mit Phosphorverbindungen der Formel (VIII) 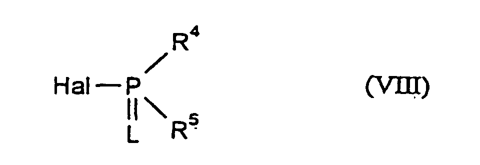 in welcher
L, R⁴ und R⁵ die in Ansprüch 1 angebebenen Bedeutungen haben und
Hal für Halogen steht,
gegebenenfalls in Gegenwart eines Verdünnungsmittels und gegebenenfalls in Gegenwart eines Säurebindemittels umsetzt,
(G) Verbindungen der Formel (I), in welcher A, B, E, X, Y, Z und n die in Ansprüch 1 angegebenen Bedeutungen haben, sind G für die Grüppe (f) steht Verbindungen der Formeln (I-a), in welcher A, B, X, Y, Z und n die in Ansprüch 1 angegebenen Bedeutungen haben, jeweils
mit Metallverbindungen oder Aminen der Formeln (IX) oder (X)
Me(OR¹⁰)ₜ (IX) 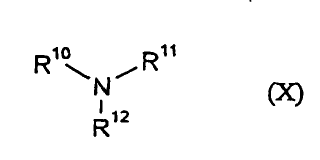
in welchen
Me für ein ein- oder zweiwertiges Metall,
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt,
(H) Verbindungen der Formel (I), in welcher A, B, L, R⁶, R⁷, X, Y, Z und n die in Ansprüch 1 angegebenen Bedeutungen haben, und G für die Grüppe (g) steht Verbindungen der Formel (I-a), in welcher A, B, X, Y, Z und n die in Ansprüch 1 angegebenen Bedeutungen haben,
mit Carbamidsäurechloriden oder Thiocarbamidsäurechloriden der Formel (XI) 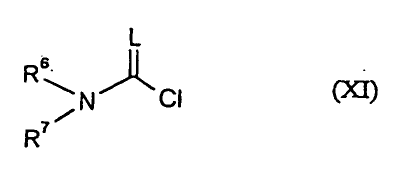
in welcher
L, R⁶ und R⁷ die in Ansprüch 1 angegebenen Bedeutungen haben,
gegebenenfalls in Gegenwart eines Verdünnungsmittels und gegebenenfalls in Gegenwart eines Säurebindemittels, umsetzt.

5. Verbindungen der Formel (XV) 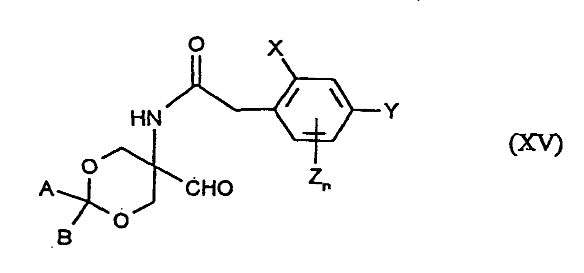 in welcher
A, B, X, Y, Z und n die in Anspruch 1 angegebene Bedeutung haben.

6. Verbindungen der Formel (XVI) 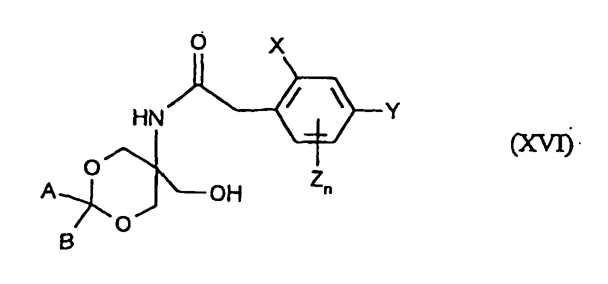 in welcher
A, B, X, Y, Z und n die in Anspruch 1 angegebene Bedeutung haben.

7. Schädlingsbekämpfungsmittel und Herbizide, **gekennzeichnet durch** einen Gehalt an mindestens einer Verbindung der Formel (I) gemäß Anspruch 1.

8. Verwendung von Verbindungen der Formel (I) gemäß Anspruch 1 zur Bekämpfung von Schädlingen und unerwünschtem Pflanzenbewuchs.

9. Verfahren zur Bekämpfung von Schädlingen, **dadurch gekennzeichnet, daß** man Verbindungen der Formel (I) gemäß Anspruch 1 auf Schädlinge, unerwünschten Pflanzenbewuchs und/oder ihren Lebensraum einwirken läßt.

10. Verfahren zur Herstellung von Schädlingsbekämpfungsmitteln und Herbiziden, **dadurch gekennzeichnet, daß** man Verbindungen der Formel (I) gemäß Anspruch 1 mit Streckmitteln und/oder oberflächenaktiven Mitteln vermischt.

11. Verwendung von Verbindungen der Formel (I) gemäß Anspruch 1 zur Herstellung von Schädlingsbekämpfungsmitteln und Herbiziden.

12. Vebindungen der Formel (II) 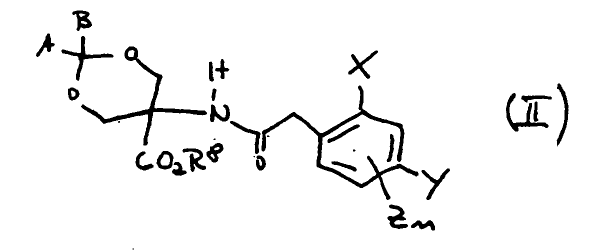 in welcher
A, B, X, Y, Z, R⁸ und n die in Ansprüch 1 angegebene Bedeutungen haben.

## Claims

1. Compounds of the formula (I) 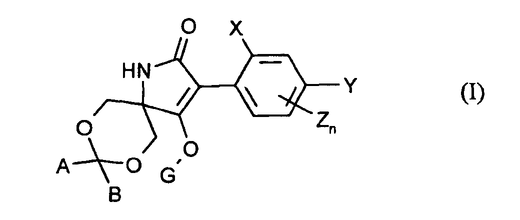 in which
X represents halogen, C₁-C₆-alkyl, C₂-C₆-alkenyl, C₂-C₆-alkinyl, C₁-C₆-alkoxy, C₃-C₆-alkenyloxy, C₁-C₆-alkylthio, C₁-C₆-alkylsulphinyl, C₁-C₆-alkylsulphonyl, C₁-C₆-halogenoalkyl, C₂-C₆-halogenoalkenyl, C₁-C₆-halogenoalkoxy, C₃-C₆-halogenoalkenyloxy, nitro, cyano or represents phenyl, phenoxy, phenylthio, benzyloxy or benzylthio, each of which is optionally substituted by halogen, C₁-C₆-alkyl, C₁-C₆-alkoxy, C₁-C₄-halogenoalkyl, C₁-C₄-halogenoalkoxy, nitro or cyano,
Y represents hydrogen, halogen, C₁-C₆-alkyl, C₂-C₆-alkenyl, C₂-C₆-alkinyl, C₁-C₆-alkoxy, C₃-C₆-alkenyloxy, C₁-C₆-alkylthio, C₁-C₆-alkylsulphinyl, C₁-C₆-alkylsulphonyl, C₁-C₆-halogenoalkyl, C₂-C₆-halogenoalkenyl, C₁-C₆-halogenoalkoxy, C₃-C₆-halogenoalkenyloxy, nitro or cyano,
Z represents halogen, C₁-C₆-alkyl, C₂-C₆-alkenyl, C₂-C₆-alkinyl, C₁-C₆-halogenoalkyl, C₂-C₆-halogenoalkenyl, C₁-C₆-alkoxy, C₃-C₆-alkenyloxy, C₁-C₆-halogenoalkoxy, C₃-C₆-halogenoalkenyloxy, nitro or cyano,
n preferably represents 0, 1, 2 or 3,
A represents hydrogen or represents C₁-C₁₂-alkyl, C₂-C₈-alkenyl, C₂-C₈-alkinyl, C₁-C₁₀-alkoxy-C₁-C₈-alkyl, poly-C₁-C₈-alkoxy-C₁-C₈-alkyl or C₁-C₁₀-alkylthio-C₁-C₆-alkyl, each of which is optionally substituted by halogen, represents optionally halogen-, C₁-C₆-alkyl- or C₁-C₆-alkoxy-substituted C₃-C₈-cycloalkyl in which optionally one methylene group is replaced by oxygen or sulphur or represents phenyl or naphthyl, hetaryl having 5 to 6 ring atoms, preferably furanyl, pyridyl, imidazolyl, triazolyl, pyrazolyl, pyrimidyl, thiazolyl or thienyl or phenyl-or naphthyl-C₁-C₆-alkyl, each of which is optionally substituted by halogen, C₁-C₆-alkyl, C₁-C₆-halogenoalkyl, C₁-C₆-alkoxy, C₁-C₆-halogenoalkoxy, cyano or nitro and which may additionally be substituted by optionally halogen-, C₁-C₄-alkyl-, C₁-C₄-alkoxy-, C₁-C₂-halogenoalkyl-, C₁-C₂-halogenoalkoxy-, cyano- or nitro-substituted phenoxy,
B represents hydrogen or represents C₁-C₁₂-alkyl or C₁-C₈-alkoxy-C₁-C₆-alkyl, each of which is optionally substituted by halogen, or
A, B and the linking carbon atom represent saturated or unsaturated C₃-C₁₀-cycloalkyl (in particular C₅-C₈-cycloalkyl), in which optionally one methylene group is replaced by oxygen or sulphur and which is optionally mono- or polysubstituted by C₁-C₈-alkyl, C₃-C₁₀-cycloalkyl, C₁-C₈-halogenoalkyl, C₁-C₈-alkoxy, C₁-C₈-alkylthio, halogen or phenyl or
A, B and the linking carbon atom represent C₅-C₈-cycloalkyl or C₅-C₈-cycloalkenyl, in which two substituents together with the linking carbon atoms represent C₅-C₆-alkanediyl, C₅-C₆-alkenediyl or C₆-alkanedienediyl, each of which is optionally substituted by C₁-C₆-alkyl, C₁-C₆-alkoxy or halogen and in which optionally one methylene group is replaced by oxygen or sulphur,
G represents hydrogen (a) or represents one of the groups 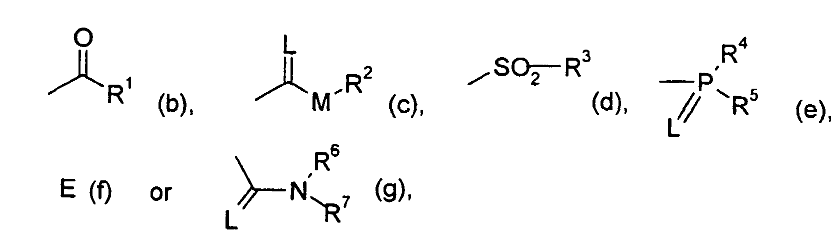 in which
E represents a metal ion equivalent or an ammonium ion,
L represents oxygen or sulphur and
M represents oxygen or sulphur,
R¹ represents C₁-C₂₀-alkyl, C₂-C₂₀-alkenyl, C₁-C₈-alkoxy-C₁-C₈-alkyl, C₁-C₈-alkylthio-C₁-C₈-alkyl or poly-C₁-C₈-alkoxy-C₁-C₈-alkyl, each of which is optionally substituted by halogen, or represents optionally halogen-, C₁-C₆-alkyl- or C₁-C₆-alkoxy-substituted C₃-C₈-cycloalkyl, in which optionally at least one methylene group is replaced by oxygen or sulphur,
represents optionally halogen-, cyano-, nitro-, C₁-C₆-alkyl-, C₁-C₆-alkoxy-, C₁-C₆-halogenoalkyl-, C₁-C₆-halogenoalkoxy-, C₁-C₆-alkylthio-or C₁-C₆-alkylsulphonyl-substituted phenyl,
represents optionally halogen-, nitro-, cyano-, C₁-C₆-alkyl-, C₁-C₆-alkoxy-, C₁-C₆-halogenoalkyl- or C₁-C₆-halogenoalkoxy-substituted phenyl-C₁-C₆-alkyl,
represents optionally halogen- or C₁-C₆-alkyl-substituted 5- or 6-membered hetaryl,
represents optionally halogen- or C₁-C₆-alkyl-substituted phenoxy-C₁-C₆-alkyl or
represents optionally halogen-, amino- or C₁-C₆-alkyl-substituted 5- or 6-membered hetaryloxy-C₁-C₆-alkyl,
R² represents C₁-C₂₀-alkyl, C₂-C₂₀-alkenyl, C₁-C₈-alkoxy-C₂-C₈-alkyl or poly-C₁-C₈-alkoxy-C₂-C₈-alkyl, each of which is optionally substituted by halogen,
represents optionally halogen-, C₁-C₆-alkyl- or C₁-C₆-alkoxy-substituted C₃-C₈-cycloalkyl or
represents phenyl or benzyl, each of which is optionally substituted by halogen, cyano, nitro, C₁-C₆-alkyl, C₁-C₆-alkoxy, C₁-C₆-halogenoalkyl or C₁-C₆-halogenoalkoxy,
R³ represents optionally halogen-substituted C₁-C₈-alkyl or represents phenyl or benzyl, each of which is optionally substituted by halogen, C₁-C₆-alkyl, C₁-C₆-alkoxy, C₁-C₄-halogenoalkyl, C₁-C₄-halogenoalkoxy, cyano or nitro,
R⁴ and R⁵ independently of one another each represent C₁-C₈-alkyl, C₁-C₈-alkoxy, C₁-C₈-alkylamino, di-(C₁-C₈-alkyl)amino, C₁-C₈-alkylthio, C₂-C₈-alkenylthio or C₃-C₇-cycloalkylthio, each of which is optionally substituted by halogen, or represent phenyl, phenoxy or phenylthio, each of which is optionally substituted by halogen, nitro, cyano, C₁-C₄-alkoxy, C₁-C₄-halogenoalkoxy, C₁-C₄-alkylthio, C₁-C₄-halogenoalkylthio, C₁-C₄-alkyl or C₁-C₄-halogenoalkyl and
R⁶ and R⁷ independently of one another each represent hydrogen, represent C₁-C₈-alkyl, C₃-C₈-cycloalkyl, C₁-C₈-alkoxy, C₃-C₈-alkenyl or C₁-C₈-alkoxy-C₁-C₈-alkyl, each of which is optionally substituted by halogen, represent optionally halogen-, C₁-C₈-halogenoalkyl-, C₁-C₈-alkyl- or C₁- C₈-alkoxy-substituted phenyl, optionally halogen-, C₁-C₈-alkyl-, C₁-C₈-halogenoalkyl- or C₁-C₈-alkoxy-substituted benzyl or together represent a C₃-C₆-alkylene radical in which optionally one methylene group is replaced by oxygen or sulphur.

2. Compounds of the formula (I) according to Claim 1 in which
X represents fluorine, chlorine, bromine, C₁-C₄-alkyl, C₁-C₄-alkoxy, C₁-C₄-halogenoalkyl, C₁-C₄-halogenoalkoxy, nitro, cyano or represents phenyl or benzyloxy, each of which is optionally substituted by fluorine, chlorine, bromine, C₁-C₄-alkyl, C₁-C₄-alkoxy, C₁-C₂-halogenoalkyl, C₁-C₂-halogenoalkoxy, nitro or cyano,
Y represents hydrogen, fluorine, chlorine, bromine, C₁-C₄-alkyl, C₁-C₄-alkoxy, C₃-C₄-alkenyloxy, C₁-C₄-halogenoalkyl, C₁-C₄-halogenoalkoxy, nitro or cyano,
Z represents fluorine, chlorine, bromine, C₁-C₄-alkyl, C₁-C₄-halogenoalkyl, C₁-C₄-alkoxy, C₂-C₄-halogenoalkoxy, nitro or cyano,
n represents 0, 1, 2 or 3,
A represents hydrogen or represents C₁-C₁₀-alkyl or C₁-C₈-alkoxy-C₁-C₆-alkyl, each of which is optionally substituted by fluorine or chlorine, optionally fluorine-, chlorine-, C₁-C₄-alkyl- or C₁-C₄-alkoxy-substituted C₃-C₇-cycloalkyl, or represents phenyl, furanyl, pyridyl, imidazolyl, triazolyl, pyrazolyl, pyrimidyl, thiazolyl, thienyl or phenyl-C₁-C₄-alkyl, each of which is optionally substituted by fluorine, chlorine, bromine, C₁-C₄-alkyl, C₁-C₄-halogenoalkyl, C₁-C₄-alkoxy, C₁-C₄-halogenoalkoxy, cyano or nitro,
B represents hydrogen or represents C₁-C₁₀-alkyl or C₁-C₆-alkoxy-C₁-C₄-alkyl, each of which is optionally substituted by fluorine or chlorine, or
A, B and the linking carbon atom represent saturated or unsaturated C₅-C₈-cycloalkyl in which optionally one methylene group is replaced by oxygen or sulphur and which is optionally substituted by C₁-C₆-alkyl, C₃-C₈-cycloalkyl, C₁-C₃-halogenoalkyl, C₁-C₆-alkoxy, C₁-C₆-alkylthio, fluorine, chlorine or phenyl or
A, B and the linking carbon atom represent C₅-C₆-cycloalkyl or C₅-C₆-cycloalkenyl in which two substituents together with the linking carbon atoms represent C₅-C₆-alkanediyl, C₅-C₆-alkenediyl or C₆-alkanedienediyl, each of which is optionally substituted by C₁-C₅-alkyl, C₁-C₅-alkoxy, fluorine, chlorine or bromine, and in which optionally one methylene group is replaced by oxygen or sulphur,
G represents hydrogen (a) or one of the groups 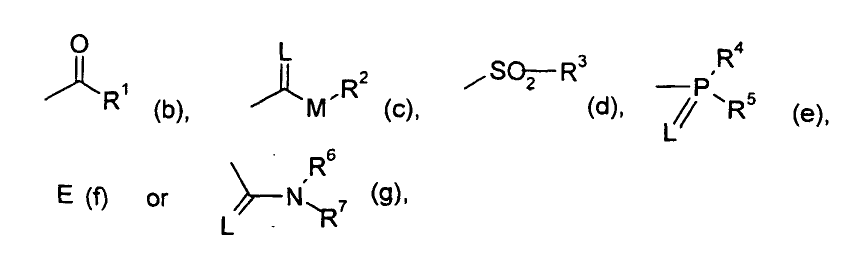 in which
E represents a metal ion equivalent or an ammonium ion,
L represents oxygen or sulphur and
M represents oxygen or sulphur,
R¹ represents C₁-C₁₆-alkyl, C₂-C₁₆-alkenyl, C₁-C₆-alkoxy-C₁-C₆-alkyl, C₁-C₆-alkylthio-C₁-C₆-alkyl or poly-C₁-C₆-alkoxy-C₁-C₆-alkyl, each of which is optionally substituted by fluorine or chlorine, or optionally fluorine-, chlorine-, C₁-C₅-alkyl- or C₁-C₅-alkoxy-substituted C₃-C₇-cycloalkyl in which optionally one or two not directly adjacent methylene groups are replaced by oxygen or sulphur,
represents optionally fluorine-, chlorine-, bromine-, cyano-, nitro-, C₁- C₄-alkyl-, C₁-C₄-alkoxy-, C₁-C₃-halogenoalkyl-, C₁-C₃-halogenoalkoxy-, C₁-C₄-alkylthio- or C₁-C₄-alkylsulphonyl-substituted phenyl,
represents optionally fluorine-, chlorine-, bromine-, C₁-C₄-alkyl-, C₁-C₄-alkoxy-, C₁-C₃-halogenoalkyl- or C₁-C₃-halogenoalkoxy-substituted phenyl-C₁-C₄-alkyl,
represents pyrazolyl, thiazolyl, pyridyl, pyrimidyl, furanyl or thienyl, each of which is optionally substituted by fluorine, chlorine, bromine or C₁-C₄-alkyl,
represents optionally fluorine-, chlorine-, bromine- or C₁-C₄-alkyl-substituted phenoxy-C₁-C₅-alkyl or
represents pyridyloxy-C₁-C₅-alkyl, pyrimidyloxy-C₁-C₅-alkyl or thiazolyloxy-C₁-C₅-alkyl, each of which is optionally substituted by fluorine, chlorine, bromine, amino or C₁-C₄-alkyl,
R² represents C₁-C₁₆-alkyl, C₂-C₁₆-alkenyl, C₁-C₆-alkoxy-C₂-C₆-alkyl or poly-C₁-C₆-alkoxy-C₂-C₆-alkyl, each of which is optionally substituted by fluorine or chlorine,
represents optionally fluorine-, chlorine-, C₁-C₄-alkyl- or C₁-C₄-alkoxy-substituted C₃-C₇-cycloalkyl or
represents phenyl or benzyl, each of which is optionally substituted by fluorine, chlorine, bromine, cyano, nitro, C₁-C₄-alkyl, C₁-C₃-alkoxy, C₁-C₃-halogenoalkyl or C₁-C₃-halogenoalkoxy,
R³ represents optionally fluorine- or chlorine-substituted C₁-C₆-alkyl or represents phenyl or benzyl, each of which is optionally substituted by fluorine, chlorine, bromine, C₁-C₅-alkyl, C₁-C₅-alkoxy, C₁-C₃-halogenoalkyl, C₁-C₃-halogenoalkoxy, cyano or nitro,
R⁴ and R⁵ independently of one another each represent C₁-C₆-alkyl, C₁-C₆-alkoxy, C₁-C₆-alkylamino, di-(C₁-C₆-alkyl)amino, C₁-C₆-alkylthio, C₃-C₄-alkenylthio or C₃-C₆-cycloalkylthio, each of which is optionally substituted by fluorine or chlorine, or represent phenyl, phenoxy or phenylthio, each of which is optionally substituted by fluorine, chlorine, bromine, nitro, cyano, C₁-C₃-alkoxy, C₁-C₃-halogenoalkoxy, C₁-C₃-alkylthio, C₁-C₃-halogenoalkylthio, C₁-C₃-alkyl or C₁-C₃-halogenoalkyl and
R⁶ and R⁷ independently of one another each represent hydrogen, represent C₁-C₆-alkyl, C₃-C₆-cycloalkyl, C₁-C₆-alkoxy, C₃-C₆-alkenyl or C₁-C₆-alkoxy-C₁-C₆-alkyl, each of which is optionally substituted by halogen, represent optionally halogen-, C₁-C₅-halogenoalkyl-, C₁-C₅-alkyl- or C₁-C₅-alkoxy-substituted phenyl, represent optionally halogen-, C₁-C₅-alkyl-, C₁-C₅-halogenoalkyl- or C₁-C₅-alkoxy-substituted benzyl, or together represent a C₃-C₆-alkylene radical in which optionally one methylene group is replaced by oxygen or sulphur.

3. Compounds of the formula (I) according to Claim 1 in which
X represents fluorine, chlorine, bromine, methyl, ethyl, propyl, iso-propyl, methoxy, ethoxy, propoxy, iso-propoxy, trifluoromethyl, difluoromethoxy, trifluoromethoxy, nitro or cyano,
Y represents hydrogen, fluorine, chlorine, bromine, methyl, ethyl, n-propyl, i-propyl, n-butyl, i-butyl, tert-butyl, methoxy, ethoxy, propoxy, iso-propoxy, allyloxy, methallyloxy, trifluoromethyl, difluoromethoxy, trifluoromethoxy, nitro or cyano,
Z represents fluorine, chlorine, bromine, methyl, ethyl, n-propyl, i-propyl, n-butyl, i-butyl, tert-butyl, methoxy, ethoxy, propoxy, iso-propoxy, difluoromethoxy, trifluoromethyl, trifluoromethoxy, nitro or cyano,
n represents 0, 1, 2 or 3,
A represents arydrogen or represents C₁-C₈-alkyl or C₁-C₆-alkoxy-C₁-C₄-alkyl, each of which is optionally substituted by fluorine or chlorine, optionally fluorine-, chlorine-, methyl-, ethyl-, iso-propyl-, tert-butyl-, ethoxy- or methoxy-substituted C₅-C₆-cycloalkyl, or represents phenyl or benzyl, each of which is optionally substituted by fluorine, chlorine, bromine, methyl, ethyl, iso-propyl, tert-butyl, methoxy, ethoxy, trifluoromethyl, difluoromethoxy, trifluoromethoxy, cyano or nitro,
B represents hydrogen or represents C₁-C₈-alkyl or C₁-C₄-alkoxy-C₁-C₂-alkyl, each of which is optionally substituted by fluorine, or
A, B and the linking carbon atom represent saturated or unsaturated C₅-C₆-cycloalkyl in which optionally one methylene group is replaced by oxygen or sulphur and which is optionally substituted by methyl, ethyl, isopropyl, tert-butyl, trifluoromethyl, methoxy, ethoxy, fluorine, chlorine or phenyl or
A, B and the linking carbon atom represent C₅-C₆-cycloalkyl or C₅-C₆-cycloalkenyl in which two substituents together with the linking carbon atoms represent C₅-C₆-alkanediyl, C₅-C₆-alkenediyl or C₆-alkanedienediyl,
G represents hydrogen (a) or represents one of the groups 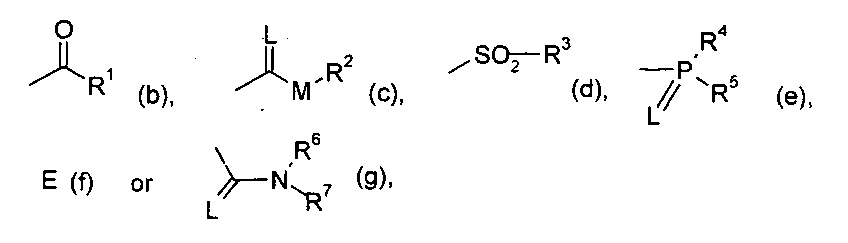 in which
E represents a metal ion equivalent or an ammonium ion,
L represents oxygen or sulphur and
M represents oxygen or sulphur,
R¹ represents C₁-C₁₄-alkyl, C₂-C₁₄-alkenyl, C₁-C₄-alkoxy-C₁-C₆-alkyl, C₁-C₄-alkylthio-C₁-C₆-alkyl or poly-C₁-C₄-alkoxy-C₁-C₄-alkyl, each of which is optionally substituted by fluorine or chlorine, or optionally fluorine-, chlorine-, methyl-, ethyl-, propyl-, i-propyl-, butyl-, i-butyl-, tert-butyl-, methoxy-, ethoxy-, propoxy- or iso-propoxy-substituted C₃-C₆-cycloalkyl in which optionally one or two not directly adjacent methylene groups are replaced by oxygen or sulphur,
represents optionally fluorine-, chlorine-, bromine-, cyano-, nitro-, methyl-, ethyl-, propyl-, i-propyl-, methoxy-, ethoxy-, trifluoromethyl-, trifluoromethoxy-, methylthio-, ethylthio-, methylsulphonyl- or ethylsulphonyl-substituted phenyl,
represents optionally fluorine-, chlorine-, bromine-, methyl-, ethyl-, propyl-, i-propyl-, methoxy-, ethoxy-, trifluoromethyl- or trifluoromethoxy-substituted benzyl,
represents furanyl, thienyl, pyridyl, pyrimidyl, thiazolyl or pyrazolyl, each of which is optionally substituted by fluorine, chlorine, bromine, methyl or ethyl,
represents optionally fluorine-, chlorine-, methyl- or ethyl-substituted phenoxy-C₁-C₄-alkyl or
represents pyridyloxy-C₁-C₄-alkyl, pyrimidyloxy-C₁-C₄-alkyl or thiazolyloxy-C₁-C₄-alkyl, each of which is optionally substituted by fluorine, chlorine, amino, methyl or ethyl,
R² represents C₁-C₁₄-alkyl, C₂-C₁₄-alkenyl, C₁-C₄-alkoxy-C₂-C₆-alkyl or poly-C₁-C₄-alkoxy-C₂-C₆-alkyl, each of which is optionally substituted by fluorine or chlorine,
represents optionally fluorine-, chlorine-, methyl-, ethyl-, propyl-, isopropyl- or methoxy-substituted C₃-C₆-cycloalkyl,
or represents phenyl or benzyl, each of which is optionally substituted by fluorine, chlorine, cyano, nitro, methyl, ethyl, propyl, i-propyl, methoxy, ethoxy, trifluoromethyl or trifluormethoxy,
R³ represents methyl, ethyl, propyl, isopropyl, each of which is optionally substituted by fluorine or chlorine, or represents phenyl or benzyl, each of which is optionally substituted by fluorine, chlorine, bromine, methyl, ethyl, propyl, iso-propyl, tert-butyl, methoxy, ethoxy, isopropoxy, tertbutoxy, trifluoromethyl, trifluoromethoxy, cyano or nitro,
R⁴ and R⁵ independently of one another each represent C₁-C₄-alkyl, C₁-C₄-alkoxy, C₁-C₄-alkylamino, di-(C₁-C₄-alkyl)amino, C₁-C₄-alkylthio, each of which is optionally substituted by fluorine or chlorine, or represent phenyl, phenoxy or phenylthio, each of which is optionally substituted by fluorine, chlorine, bromine, nitro, cyano, C₁-C₂-alkoxy, C₁-C₂-fluoroalkoxy, C₁-C₂-alkylthio, C₁-C₂-fluoroalkylthio or C₁-C₃-alkyl and
R⁶ and R⁷ independently of one another each represent hydrogen, represent C₁-C₄-alkyl, C₃-C₆-cycloalkyl, C₁-C₄-alkoxy, C₃-C₄-alkenyl or C₁-C₄-alkoxy-C₁-C₄-alkyl, each of which is optionally substituted by fluorine or chlorine, represent optionally fluorine-, chlorine-, bromine-, C₁-C₄-halogenoalkyl-, C₁-C₄-alkyl- or C₁-C₄-alkoxy-substituted phenyl, represent optionally fluorine-, chlorine-, bromine-, C₁-C₄-alkyl-, C₁-C₄-halogenoalkyl- or C₁-C₄-alkoxy-substituted benzyl, or together represent a C₅-C₆-alkylene radical in which optionally one methylene group is replaced by oxygen or sulphur.

4. Process for preparing compounds of the formula (I) according to Claim 1, **characterized in that**, in order to obtain
(A) compounds of the formula (I-a) 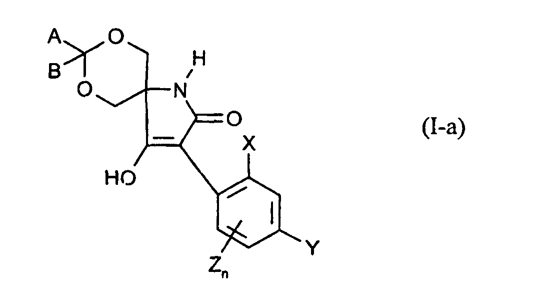 in which
A, B, X, Y, Z and n are each as defined in Claim 1,
N-acylamino acid esters of the formula (II) 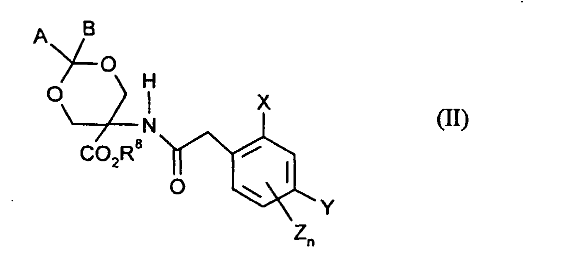 in which
A, B, X, Y, Z and n are each as defined in Claim 1
and
R⁸ represents alkyl,
are condensed intramolecularly in the presence of a diluent and in the presence of a base,
(B)
α) compounds of the formula (I) in which A, B, X, Y, Z, R¹ and n are each as defined in Claim 1 and G represents the group (b), compounds of the formula (I-a) in which A, B, X, Y, Z and n are each as defined in Claim 1 are reacted with acyl halides of the formula (III) 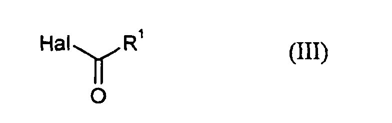 in which
R¹ is as defined in Claim 1 and
Hal represents halogen
or are reacted
β) with carboxylic anhydrides of the formula (IV)
R¹-CO-O-CO-R¹ (IV)
in which
R¹ is as defined in Claim 1,
if appropriate in the presence of a diluent and if appropriate in the presence of an acid binder;
(C) compounds of the formula (I) in which A, B, R², M, X, Y, Z and n are each as defined in Claim 1 and L represents oxygen and G represents the group (c), compounds of the formula (I-a) in which A, B, X, Y, Z and n are each as defined in Claim 1 are in each case
reacted with chloroformic esters or chloroformic thiol esters of the formula (V)
R²-M-CO-Cl (V)
in which
R² and M are each as defined in Claim 1,
if appropriate in the presence of a diluent and if appropriate in the presence of an acid binder;
(D) compounds of the formula (I) in which A, B, R², M, X, Y, Z and n are each as defined in Claim 1 and L represents sulphur and G represents the group (c), compounds of the formula (I-a) in which A, B, X, Y, Z and n are each as defined in Claim 1 are in each case
reacted with chloromonothioformic esters or chlorodithioformic esters of the formula (VI) 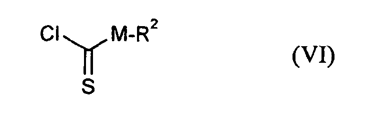 in which
M and R² xare each as defined in Claim 1,
if appropriate in the presence of a diluent and if appropriate in the presence of an acid binder,
(E) compounds of the formula (I) in which A, B, R³, X, Y, Z and n are each as defined in Claim 1 and G represents the group (d), compounds of the formula (I-a) in which A, B, X, Y, Z and n are each as defined in Claim 1 are in each case
reacted with sulphonyl chlorides of the formula (VII)
R³-SO₂-Cl (VII)
in which
R³ is as defined in Claim 1,
if appropriate in the presence of a diluent and if appropriate in the presence of an acid binder,
(F) compounds of the formula (I) in which A, B, L, R⁴, R⁵, X, Y, Z and n are each as defined in Claim 1 and G represents the group (e), compounds of the formula (I-a) in which A, B, X, Y, Z and n are each as defined in Claim 1 are in each case
reacted with phosphorus compounds of the formula (VIII) 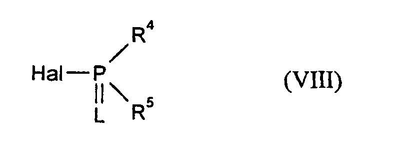 in which
L, R⁴ and R⁵ are each as defined in Claim 1 and
Hal represents halogen,
if appropriate in the presence of a diluent and if appropriate in the presence of an acid binder,
(G) compounds of the formula (I) in which A, B, E, X, Y, Z and n are each as defined in Claim 1 and G represents the group (f), compounds of the formula (I-a) in which A, B, X, Y, Z and n are each as defined in Claim 1 are in each case
reacted with metal compounds or amines of the formulae (IX) or (X)
Me(OR¹⁰)ₜ (IX) 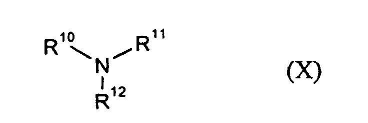
in which
Me represents a mono- or divalent metal,
t represents the number 1 or 2 and
R¹⁰, R¹¹, R¹² independently of one another each represent hydrogen or alkyl,
if appropriate in the presence of a diluent,
(H) compounds of the formula (I) in which A, B, L, R⁶, R⁷, X, Y, Z and n are each as defined in Claim 1 and G represents the group (g), compounds of the formula (I-a) in which A, B, X, Y, Z and n are each as defined in Claim 1
are reacted with carbamoyl chlorides or thiocarbamoyl chlorides of the formula (XI), 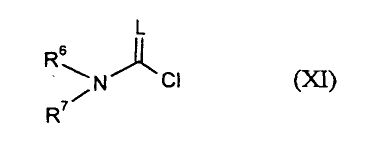
in which
L, R⁶ and R⁷ are each as defined in Claim 1,
if appropriate in the presence of a diluent and if appropriate in the presence of an acid binder.

5. Compounds of the formula (XV) 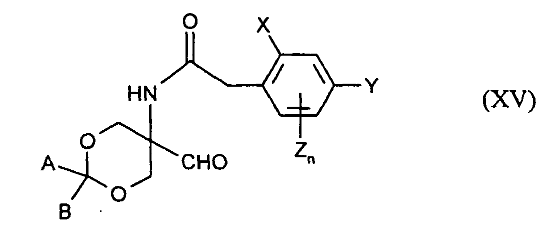 in which
A, B, X, Y, Z and n are each as defined in Claim 1.

6. Compounds of the formula (XVI) 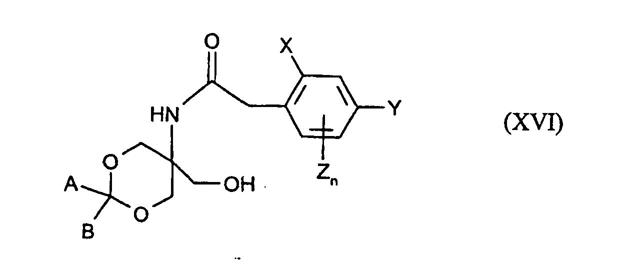 in which
A, B, X, Y, Z and n are each as defined in Claim 1.

7. Pesticides and herbicides, **characterized in that** they contain at least one compound of the formula (I) according to Claim 1.

8. Use of compounds of the formula (I) according to Claim 1 for controlling pests and undesirable vegetation.

9. Method for controlling pests, **characterized in that** compounds of the formula (I) according to Claim 1 are allowed to act on pests, undesirable vegetation and/or their habitat.

10. Process for preparing pesticides and herbicides, **characterized in that** compounds of the formula (I) according to Claim 1 are mixed with extenders and/or surfactants.

11. Use of compounds of the formula (I) according to Claim 1 for preparing pesticides and herbicides.

12. Compounds of the formula (II) 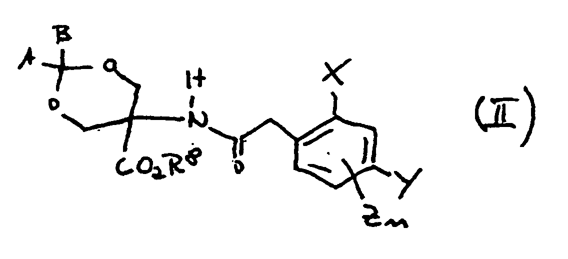 in which
A, B, X, Y, Z, R⁸ and n are each as defined in Claim 1.

## Revendications

1. Composés de la formule (I) : 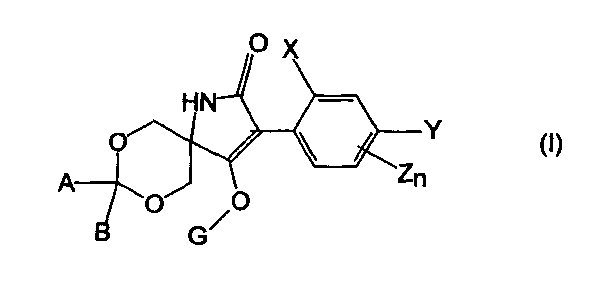 dans laquelle :
X représente un atome d'halogène, un radical alcoyle en C₁-C₆, alcényle en C₂-C₆, alcynyle en C₂-C₆, alcoxy en C₁-C₆, alcényloxy en C₃-C₆, alcoylthio en C₁-C₆, alcoylsulfinyle en C₁-C₆, alcoylsulfonyle en C₁-C₆, halogénoalcoyle en C₁-C₆, halogénoalcényle en C₂-C₆, halogénoalcoxy en C₁-C₆, halogénoalcényloxy en C₃-C₆, le radical nitro, cyano ou représente un radical phényle, phénoxy, phénylthio, benzyloxy ou benzylthio chaque fois le cas échéant substitué par un atome d'halogène, un radical alcoyle en C₁-C₆, alcoxy en C₁-C₆, halogénoalcoyle en C₁-C₄, halogénoalcoxy en C₁-C₄, le radical nitro ou cyano,
Y représente l'atome d'hydrogène, un atome d'halogène, un radical alcoyle en C₁-C₆, alcényle en C₂-C₆, alcynyle en C₂-C₆, alcoxy en C₁-C₆, alcényloxy en C₃-C₆, alcoylthio en C₁-C₆, alcoylsulfinyle en C₁-C₆, alcoylsulfonyle en C₁-C₆, halogénoalcoyle en C₁-C₆, halogénoalcényle en C₂-C₆, halogénoalcoxy en C₁-C₆, halogénoalcényloxy en C₃-C₆, le radical cyano ou nitro,
Z représente un atome d'halogène, un radical alcoyle en C₁-C₆, alcényle en C₂-C₆, alcynyle en C₂-C₆, halogénoalcoyle en C₁-C₆, halogénoalcényle en C₂-C₆, alcoxy en C₁-C₆, alcényloxy en C₃-C₆, halogénoalcoxy en C₁-C₆, halogénoalcényloxy en C₃-C₆, le radical cyano ou nitro,
n représente de préférence, 0, 1, 2 ou 3,
A représente l'atome d'hydrogène ou un radical alcoyle en C₁-C₁₂, alcényle en C₂-C₈, alcynyle en C₂-C₈, (alcoxy en C₁-C₁₀)alcoyle en C₁-C₈, poly(alcoxy en C₁-C₈)alcoyle en C₁-C₈ ou (alcoylthio en C₁-C₁₀)alcoyle en C₁-C₆, chaque fois le cas échéant substitué par un atome d'halogène, ou représente un radical cycloalcoyle en C₃-C₈ le cas échéant substitué par un atome d'halogène, un radical alcoyle en C₁-C₆ ou alcoxy en C₁-C₆, dans lequel un radical méthylène peut être le cas échéant, remplacé par l'atome d'oxygène ou l'atome de soufre, ou représente le radical phényle ou naphtyle, un radical hétaryle avec 5 à 6 atomes cycliques, de préférence furannyle, pyridyle, imidazolyle, triazolyle, pyrazolyle, pyrimidyle, thiazolyle ou thiényle, chaque fois le cas échéant substitué par un atome d'halogène, un radical alcoyle en C₁-C₆, halogénoalcoyle en C₁-C₆, alcoxy en C₁-C₆, halogénoalcoxy en C₁-C₆, cyano ou nitro, ou représente un radical phényl- ou naphtyl(alcoyle en C₁-C₆), qui peuvent être substitués, de plus, par le radical phénoxy, le cas échéant substitué par un atome d' halogène, un radical alcoyle en C₁-C₄, alcoxy en C₁-C₄, halogénoalcoyle en C₁-C₂, halogénoalcoxy en C₁-C₂, cyano ou nitro,
B représente l'atome d'hydrogène ou un radical alcoyle en C₁-C₁₂ ou (alcoxy en C₁-C₈)alcoyle en C₁-C₆, chaque fois le cas échéant substitué par un atome d'halogène, ou
A, B et l'atome de carbone sur lequel ils sont liés, représentent un radical cycloalcoyle en C₃-C₁₀ saturé ou insaturé (en particulier cycloalcoyle en C₅-C₈), dans lequel un radical méthylène peut être le cas échéant, remplacé par l'atome d'oxygène ou l'atome de soufre et qui est substitué le cas échéant, une ou plusieurs fois, par un radical alcoyle en C₁-C₈, cycloalcoyle en C₃-C₁₀, halogénoalcoyle en C₁-C₈, alcoxy en C₁-C₈, alcoylthio en C₁-C₈, un atome d'halogène ou le radical phényle, ou
A, B et l'atome de carbone sur lequel ils sont liés, représentent un radical cycloalcoyle en C₅-C₈ ou cycloalcényle en C₅-C₈, dans lequel deux substituants avec l'atome de carbone sur lequel ils sont liés, représentent un radical alcanediyle en C₅-C₆, alcènediyle en C₅-C₆ ou alcanediènediyle en C₆, le cas échéant substitué par un radical alcoyle en C₁-C₆, alcoxy en C₁-C₆, un atome d'halogène, où le cas échéant, un radical méthylène est remplacé par l'atome d'oxygène ou l'atome de soufre,
G représente l'atome d'hydrogène (a) ou un des restes 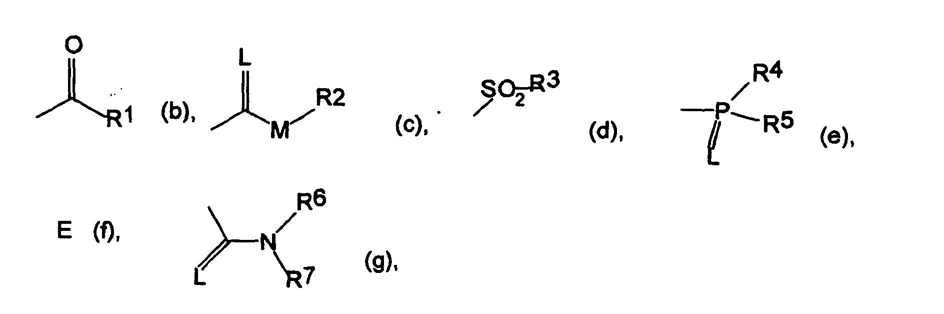
dans lesquels :
E représente un équivalent d'ion métallique ou un ion ammonium,
L représente l'atome d'oxygène ou de soufre, et
M représente l'atome d'oxygène ou de soufre,
R¹ représente un radical alcoyle en C₁-C₂₀, alcényle en C₂-C₂₀, (alcoxy en C₁-C₈)alcoyle en C₁-C₈, (alcoythio en C₁-C₈)alcoyle en C₁-C₈ ou poly (alcoxy en C₁-C₈)alcoyle en C₁-C₈, chaque fois le cas échéant substitué par un atome d'halogène, ou représente un radical cycloalcoyle en C₃-C₈, le cas échéant substitué par un atome d'halogène, un radical alcoyle en C₁-C₆ ou alcoxy en C₁-C₆, dans lequel le cas échéant, au moins un radical méthylène est remplacé par l'atome d'oxygène ou l'atome de soufre,
représente le radical phényle, le cas échéant substitué par un atome d'halogène, le radical cyano, nitro, un radical alcoyle en C₁-C₆, alcoxy en C₁-C₆, halogénoalcoyle en C₁-C₆, halogénoalcoxy en C₁-C₆, alcoylthio en C₁-C₆ ou alcoylsulfonyle en C₁-C₆,
représente le radical phényl(alcoyle en C₁-C₆), le cas échéant substitué par un atome d'halogène, le radical nitro, cyano, un radical alcoyle en C₁-C₆, alcoxy en C₁-C₆, halogénoalcoyle en C₁-C₆ ou halogénoalcoxy en C₁-C₆,
représente un radical hétéroaryle à 5 ou 6 membres, le cas échéant substitué par un atome d'halogène ou un radical alcoyle en C₁-C₆,
représente le radical phénoxy(alcoyle en C₁-C₆), le cas échéant substitué par un atome d'halogène ou un radical alcoyle en C₁-C₆,
un radical hétéroaryloxy (alcoyle en C₁-C₆) à 5 ou 6 membres, le cas échéant substitué par un atome d'halogène, le radical amino ou un radical alcoyle en C₁-C₆,
R² représente un radical alcoyle en C₁-C₂₀, alcényle en C₂-C₂₀, (alcoxy en C₁-C₈)alcoyle en C₂-C₈ ou poly (alcoxy en C₁-C₈)alcoyle en C₂-C₈, chaque fois le cas échéant substitué par un atome d'halogène,
représente un radical cycloalcoyle en C₃-C₈, le cas échéant substitué par un atome d'halogène, un radical alcoyle en C₁-C₆ ou alcoxy en C₁-C₆, ou
représente le radical phényle ou benzyle, chaque fois le cas échéant substitué par un atome d'halogène, le radical cyano, nitro, un radical alcoyle en C₁-C₆, alcoxy en C₁-C₆, halogénoalcoyle en C₁-C₆ ou halogénoalcoxy en C₁-C₆,
R³ représente un radical alcoyle en C₁-C₈ le cas échéant substitué par un atome d'halogène, ou représente le radical phényle ou benzyle, chaque fois le cas échéant substitué par un atome d'halogène, un radical alcoyle en C₁-C₆, alcoxy en C₁-C₆, halogénoalcoyle en C₁-C₄, halogénoalcoxy en C₁-C₄ ou le radical cyano ou nitro,
R⁴ et R⁵ représentent indépendamment l'un de l'autre, un radical alcoyle en C₁-C₈, alcoxy en C₁-C₈, alcoylamino en C₁-C₈, di (alcoyl en C₁-C₈)amino, alcoylthio en C₁-C₈, alcénylthio en C₂-C₈ ou cycloalcoylthio en C₃-C₇, chaque fois le cas échéant substitué par un atome d'halogène, ou représentent un radical phényle, phénoxy ou phénylthio, chaque fois le cas échéant substitué par un atome d'halogène, le radical nitro ou cyano, un radical alcoxy en C₁-C₄, halogénoalcoxy en C₁-C₄, alcoylthio en C₁-C₄, halogénoalcoylthio en C₁-C₄, alcoyle en C₁-C₄ ou halogénoalcoyle en C₁-C₄,
R⁶ et R⁷ représentent indépendamment l'un de l'autre, l'atome d'hydrogène, un radical alcoyle en C₁-C₈, cycloalcoyle en C₃-C₈, alcoxy en C₁-C₈, alcényle en C₃-C₈, (alcoxy en C₁-C₈)alcoyle en C₁-C₈, chaque fois le cas échéant substitué par un atome d'halogène, ou représentent le radical phényle le cas échéant substitué par un atome d'halogène, un radical halogénoalcoyle en C₁-C₈, alcoyle en C₁-C₈ ou alcoxy en C₁-C₈, ou représentent le radical benzyle le cas échéant substitué par un atome d'halogène, un radical alcoyle en C₁-C₈, halogénoalcoyle en C₁-C₈ ou alcoxy en C₁-C₈, ou ensemble, représentent un reste alcoylène en C₃-C₆, dans lequel un radical méthylène est le cas échéant, remplacé par l'atome d'oxygène ou de soufre.

2. Composés de la formule (I) suivant la revendication 1, dans lesquels :
X représente l'atome de fluor, de chlore, de brome, un radical alcoyle en C₁-C₄, alcoxy en C₁-C₄, halogénoalcoyle en C₁-C₄, halogénoalcoxy en C₁-C₄, le radical cyano, nitro ou représente le radical phényle ou benzyloxy, chaque fois le cas échéant substitué par l'atome de fluor, de chlore, de brome, un radical alcoyle en C₁-C₄, alcoxy en C₁-C₄, halogénoalcoyle en C₁-C₂, halogénoalcoxy en C₁-C₂, le radical nitro ou cyano,
Y représente l'atome d'hydrogène, de fluor, de chlore, de brome, un radical alcoyle en C₁-C₄, alcoxy en C₁-C₄, alcényloxy en C₃-C₄, halogénoalcoyle en C₁-C₄, halogénoalcoxy en C₁-C₄, le radical cyano ou nitro,
Z représente l'atome de fluor, de chlore, de brome, un radical alcoyle en C₁-C₄, halogénoalcoyle en C₁-C₄, alcoxy en C₁-C₄, halogénoalcoxy en C₁-C₄, le radical nitro ou cyano,
n représente 0, 1, 2 ou 3,
A représente l'atome d'hydrogène ou un radical alcoyle en C₁-C₁₀ ou (alcoxy en C₁-C₈)alcoyle en C₁-C₆, chaque fois le cas échéant substitué par l'atome de fluor ou de chlore, ou représente un radical cycloalcoyle en C₃-C₇ le cas échéant substitué par l'atome de fluor ou de chlore, un radical alcoyle en C₁-C₄ ou alcoxy en C₁-C₄, ou représente le radical phényle, furannyle, pyridyle, imidazolyle, triazolyle, pyrazolyle, pyrimidyle, thiazolyle, thiényle ou phényl-(alcoyle en C₁-C₄), chaque fois le cas échéant substitué par l'atome de fluor, de chlore, de brome, un radical alcoyle en C₁-C₄, halogénoalcoyle en C₁-C₄, alcoxy en C₁-C₄, halogénoalcoxy en C₁-C₄, cyano ou nitro,
B représente l'atome d'hydrogène ou un radical alcoyle en C₁-C₁₀ ou (alcoxy en C₁-C₆)alcoyle en C₁-C₄, chaque fois le cas échéant substitué par l'atome de fluor ou de chlore, ou
A, B et l'atome de carbone sur lequel ils sont liés, représentent un radical cycloalcoyle en C₅-C₈ saturé ou insaturé, dans lequel un radical méthylène peut être le cas échéant, remplacé par l'atome d'oxygène ou l'atome de soufre et qui est substitué le cas échéant, par un radical alcoyle en C₁-C₆, cycloalcoyle en C₃-C₈, halogénoalcoyle en C₁-C₃, alcoxy en C₁-C₆, alcoylthio en C₁-C₆, l'atome de fluor, de chlore ou le radical phényle, ou
A, B et l'atome de carbone sur lequel ils sont liés, représentent un radical cycloalcoyle en C₅-C₆ ou cycloalcényle en C₅-C₆, dans lequel deux substituants avec l'atome de carbone sur lequel ils sont liés, représentent un radical alcanediyle en C₅-C₆, alcènediyle en C₅-C₆ ou alcanediènediyle en C₆, le cas échéant substitué par un radical alcoyle en C₁-C₅, alcoxy en C₁-C₅, l'atome de fluor, de chlore ou de brome, où le cas échéant, un radical méthylène est remplacé par l'atome d'oxygène ou l'atome de soufre,
G représente l'atome d'hydrogène (a) ou un des restes 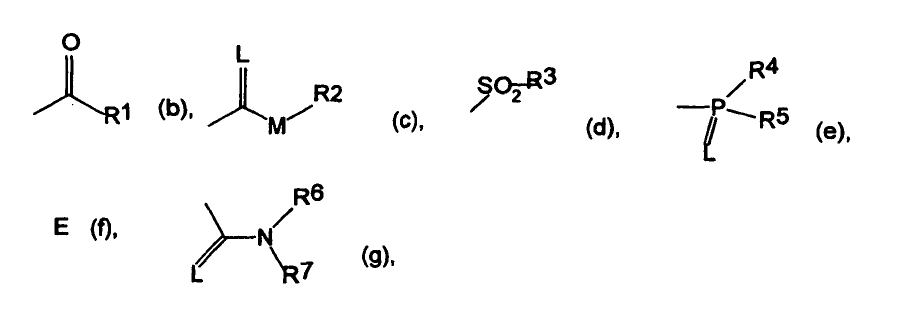 dans lesquels :
E représente un équivalent d'ion métallique ou un ion ammonium,
L représente l'atome d'oxygène ou de soufre, et
M représente l'atome d'oxygène ou de soufre,
R¹ représente un radical alcoyle en C₁-C₁₆, alcényle en C₂-C₁₆, (alcoxy en C₁-C₆)alcoyle en C₁-C₆, (alcoythio en C₁-C₆)alcoyle en C₁-C₆ ou poly(alcoxy en C₁-C₆)alcoyle en C₁-C₆, chaque fois le cas échéant substitué par l'atome de fluor ou de chlore, ou représente un radical cycloalcoyle en C₃-C₇, le cas échéant substitué par l'atome de fluor ou de chlore, un radical alcoyle en C₁-C₅ ou alcoxy en C₁-C₅, dans lequel le cas échéant, un ou deux radicaux méthylène non directement voisins sont remplacés par l'atome d'oxygène ou de soufre,
représente le radical phényle, le cas échéant substitué par l'atome de fluor, de chlore, de brome, le radical cyano, nitro, un radical alcoyle en C₁-C₄, alcoxy en C₁-C₄, halogénoalcoyle en C₁-C₃, halogénoalcoxy en C₁-C₃, alcoylthio en C₁-C₄ ou alcoylsulfonyle en C₁-C₄,
représente un radical phényl(alcoyle en C₁-C₄), le cas échéant substitué par l'atome de fluor, de chlore, de brome, un radical alcoyle en C₁-C₄, alcoxy en C₁-C₄, halogénoalcoyle en C₁-C₃ ou halogénoalcoxy en C₁-C₃,
représente le radical pyrazolyle, thiazolyle, pyridyle, pyrimidyle, furannyle ou thiényle chaque fois le cas échéant substitué par l'atome de fluor, de chlore, de brome ou un radical alcoyle en C₁-C₄,
représente le radical phénoxy(alcoyle en C₁-C₅), le cas échéant substitué par l'atome de fluor, de chlore, de brome ou un radical alcoyle en C₁-C₄,
représente un radical pyridyloxy(alcoyle en C₁-C₅), pyrimidyloxy(alcoyle en C₁-C₅) ou thiazolyloxy(alcoyle en C₁-C₅), chaque fois le cas échéant substitué par l'atome de fluor, de chlore, de brome, le radical amino ou un radical alcoyle en C₁-C₄,
R² représente un radical alcoyle en C₁-C₁₆, alcényle en C₂-C₁₆, (alcoxy en C₁-C₆)alcoyle en C₂-C₆ ou poly(alcoxy en C₁-C₆)alcoyle en C₂-C₆, chaque fois le cas échéant substitué par l'atome de fluor ou de chlore,
représente un radical cycloalcoyle en C₃-C₇, le cas échéant substitué par l'atome de fluor, de chlore, un radical alcoyle en C₁-C₄ ou alcoxy en C₁-C₄, ou
représente le radical phényle ou benzyle, chaque fois le cas échéant substitué par l'atome de fluor, de chlore, de brome, le radical cyano, nitro, un radical alcoyle en C₁-C₄, alcoxy en C₁-C₃, halogénoalcoyle en C₁-C₃ ou halogénoalcoxy en C₁-C₃,
R³ représente un radical alcoyle en C₁-C₆ le cas échéant substitué par l'atome de fluor ou de chlore, ou représente le radical phényle ou benzyle, chaque fois le cas échéant substitué par l'atome de fluor, de chlore, de brome, un radical alcoyle en C₁-C₅, alcoxy en C₁-C₅, halogénoalcoyle en C₁-C₃, halogénoalcoxy en C₁-C₃ ou le radical cyano ou nitro,
R⁴ et R⁵ représentent indépendamment l'un de l'autre, un radical alcoyle en C₁-C₆, alcoxy en C₁-C₆, alcoylamino en C₁-C₆, di (alcoyl en C₁-C₆)amino, alcoylthio en C₁-C₆ ou alcénylthio en C₃-C₄ ou cycloalcoylthio en C₃-C₆, chaque fois le cas échéant substitué par l'atome de fluor ou de chlore, ou représentent un radical phényle, phénoxy ou phénylthio, chaque fois le cas échéant substitué par l'atome de fluor, de chlore, de brome, le radical nitro ou cyano, un radical alcoxy en C₁-C₃, halogénoalcoxy en C₁-C₃, alcoylthio en C₁-C₃, halogénoalcoylthio en C₁-C₃, alcoyle en C₁-C₃ ou halogénoalcoyle en C₁-C₃,
R⁶ et R⁷ représentent indépendamment l'un de l'autre, l'atome d'hydrogène, un radical alcoyle en C₁-C₆, cycloalcoyle en C₃-C₆, alcoxy en C₁-C₆, alcényle en C₃-C₆ ou (alcoxy en C₁-C₆)alcoyle en C₂-C₆, chaque fois le cas échéant substitué par un atome d'halogène, ou représentent le radical phényle le cas échéant substitué par un atome d'halogène, un radical halogénoalcoyle en C₁-C₅, alcoyle en C₁-C₅ ou alcoxy en C₁-C₅, ou représentent le radical benzyle le cas échéant substitué par un atome d'halogène, un radical halogénoalcoyle en C₁-C₅, alcoyle en C₁-C₅ ou alcoxy en C₁-C₅, ou ensemble, représentent un reste alcoylène en C₃-C₆, dans lequel un radical méthylène est le cas échéant, remplacé par l'atome d'oxygène ou de soufre.

3. Composés de la formule (I) suivant la revendication 1, dans lesquels :
X représente l'atome de fluor, de chlore, de brome, le radical méthyle, éthyle, propyle, isopropyle, méthoxy, éthoxy, propoxy, isopropoxy, trifluorométhyle, difluorométhoxy, triflorométhoxy, le radical nitro ou cyano,
Y représente l'atome d'hydrogène, de fluor, de chlore, de brome, le radical méthyle, éthyle, n-propyle, isopropyle, n-butyle, isobutyle, t-butyle, méthoxy, éthoxy, propoxy, isopropoxy, allyloxy, méthallyloxy, trifluorométhyle, difluorométhoxy, triflorométhoxy, le radical cyano ou nitro,
Z représente l'atome de fluor, de chlore, de brome, le radical méthyle, éthyle, n-propyle, isopropyle, n-butyle, isobutyle, t-butyle, méthoxy, éthoxy, propoxy, isopropoxy, difluorométhoxy, trifluorométhyle, triflorométhoxy, le radical nitro ou cyano,
n représente 0, 1, 2 ou 3,
A représente l'atome d'hydrogène ou un radical alcoyle en C₁-C₈ ou (alcoxy en C₁-C₆)alcoyle en C₁-C₄, chaque fois le cas échéant substitué par l'atome de fluor ou de chlore, ou représente un radical cycloalcoyle en C₅-C₆ le cas échéant substitué par l'atome de fluor ou de chlore, le radical méthyle, éthyle, isopropyle, t-butyle, éthoxy ou méthoxy, ou représente le radical phényle ou benzyle, chaque fois le cas échéant substitué par l'atome de fluor, de chlore, de brome, le radical méthyle, éthyle, isopropyle, t-butyle, méthoxy, éthoxy, trifluorométhyle, difluorométhoxy, trifluorométhoxy, cyano ou nitro,
B représente l'atome d'hydrogène ou un radical alcoyle en C₁-C₈ ou (alcoxy en C₁-C₄)alcoyle en C₁-C₂, chaque fois le cas échéant substitué par l'atome de fluor, ou
A, B et l'atome de carbone sur lequel ils sont liés, représentent un radical cycloalcoyle en C₅-C₆ saturé ou insaturé, dans lequel un radical méthylène peut être le cas échéant, remplacé par l'atome d'oxygène ou l'atome de soufre et qui est substitué le cas échéant, par le radical méthyle, éthyle, isopropyle, t-butyle, trifluorométhyle, méthoxy, éthoxy, l'atome de fluor, de chlore ou le radical phényle, ou
A, B et l'atome de carbone sur lequel ils sont liés, représentent un radical cycloalcoyle en C₅-C₆ ou cycloalcényle en C₅-C₆, dans lequel deux substituants avec l'atome de carbone sur lequel ils sont liés, représentent un radical alcanediyle en C₅-C₆, alcènediyle en C₅-C₆ ou alcanediènediyle en C₆,
G représente l'atome d'hydrogène (a) ou un des restes 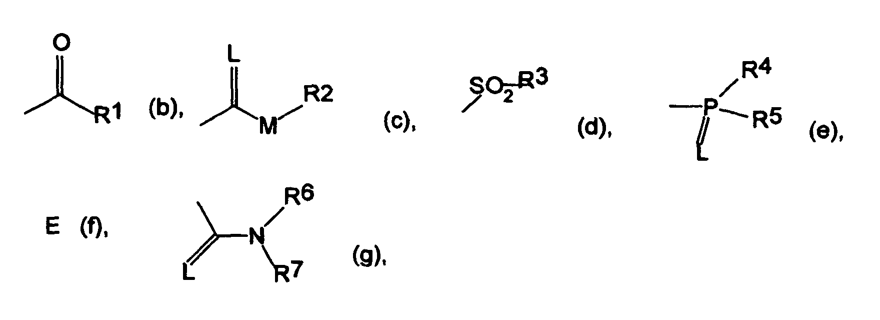
dans lesquels :
E représente un équivalent d'ion métallique ou un ion ammonium,
L représente l'atome d'oxygène ou de soufre, et
M représente l'atome d'oxygène ou de soufre,
R¹ représente un radical alcoyle en C₁-C₁₄, alcényle en C₂-C₁₄, (alcoxy en C₁-C₄)alcoyle en C₁-C₆, (alcoythio en C₁-C₄)alcoyle en C₁-C₆ ou poly(alcoxy en C₁-C₄)alcoyle en C₁-C₄, chaque fois le cas échéant substitué par l'atome de fluor ou de chlore, ou représente un radical cycloalcoyle en C₃-C₆, le cas échéant substitué par l'atome de fluor ou de chlore, le radical méthyle, éthyle, propyle, isopropyle, butyle, isobutyle, t-butyle, méthoxy, éthoxy, propoxy ou isopropoxy, dans lequel le cas échéant, un ou deux radicaux méthylène non directement voisins sont remplacés par l'atome d'oxygène et/ou de soufre,
représente le radical phényle, le cas échéant substitué par l'atome de fluor, de chlore, de brome, le radical cyano, nitro, méthyle, éthyle, propyle, isopropyle, méthoxy, éthoxy, trifluorométhyle, triflorométhoxy, méthylthio, éthylthio, méthylsulfonyle ou éthylsulfonyle,
représente le radical benzyle, le cas échéant substitué par l'atome de fluor, de chlore, de brome, le radical méthyle, éthyle, propyle, isopropyle, méthoxy, éthoxy, trifluorométhyle ou triflorométhoxy,
représente le radical furannyle, thiényle, pyridyle, pyrimidyle, thiazolyle ou pyrazolyle, chaque fois le cas échéant substitué par l'atome de fluor, de chlore, de brome, le radical méthyle ou éthyle,
représente le radical phénoxy(alcoyle en C₁-C₄), le cas échéant substitué par l'atome de fluor ou de chlore, le radical méthyle ou éthyle, ou
un radical pyridyloxy (alcoyle en C₁-C₄), pyrimidyloxy(alcoyle en C₁-C₄) ou thiazolyloxy(alcoyle en C₁-C₄), chaque fois le cas échéant substitué par l'atome de fluor ou de chlore, le radical amino, méthyle ou éthyle,
R² représente un radical alcoyle en C₁-C₁₄, alcényle en C₂-C₁₄, (alcoxy en C₁-C₄)alcoyle en C₂-C₆ ou poly(alcoxy en C₁-C₄)alcoyle en C₂-C₆, chaque fois le cas échéant substitué par l'atome de fluor ou de chlore,
représente un radical cycloalcoyle en C₃-C₆, le cas échéant substitué par l'atome de fluor, de chlore, le radical méthyle, éthyle, propyle, isopropyle
ou méthoxy, ou
représente le radical phényle ou benzyle, chaque fois le cas échéant substitué par l'atome de fluor, de chlore, le radical cyano, nitro, le radical méthyle, éthyle, propyle, isopropyle, méthoxy, éthoxy, trifluorométhyle, ou triflorométhoxy,
R³ représente le radical méthyle, éthyle, propyle, isopropyle, chaque fois le cas échéant substitué par l'atome de fluor ou de chlore, ou représente le radical phényle ou benzyle, chaque fois le cas échéant substitué par l'atome de fluor, de chlore, de brome, le radical méthyle, éthyle, propyle, isopropyle, t-butyle, méthoxy, éthoxy, isopropoxy, t-butoxy, trifluorométhyle, triflorométhoxy, cyano ou nitro,
R⁴ et R⁵ représentent indépendamment l'un de l'autre, un radical alcoyle en C₁-C₄, alcoxy en C₁-C₄, alcoylamino en C₁-C₄, di(alcoyl en C₁-C₄)amino ou alcoylthio en C₁-C₄, chaque fois le cas échéant substitué par l'atome de fluor ou de chlore, ou représentent le radical phényle, phénoxy ou phénylthio, chaque fois le cas échéant substitué par l'atome de fluor, de chlore, de brome, le radical nitro, cyano, alcoxy en C₁-C₂, fluoroalcoxy en C₁-C₂ ou alcoyle en C₁-C₃, et
R⁶ et R⁷ représentent indépendamment l'un de l'autre, l'atome d'hydrogène, un radical alcoyle en Ci-C₄, cycloalcoyle en C₃-C₆, alcoxy en C₁-C₄, alcényle en C₃-C₄, (alcoxy en C₁-C₄)alcoyle en C₁-C₄, chaque fois le cas échéant substitué par l'atome de fluor ou de chlore, ou représentent le radical phényle le cas échéant substitué par l'atome de fluor, de chlore, de brome, un radical halogénoalcoyle en C₁-C₄, alcoyle en C₁-C₄ ou alcoxy en C₁-C₄, ou représentent le radical benzyle le cas échéant substitué par l'atome de fluor, de chlore, de brome, un radical halogénoalcoyle en C₁-C₄, alcoyle en C₁-C₄ ou alcoxy en C₁-C₄, ou ensemble, représentent un reste alcoylène en C₅-C₆, dans lequel un radical méthylène est le cas échéant, remplacé par l'atome d'oxygène ou de soufre.

4. Procédé de préparation des composés de la formule (I) suivant la revendication 1, **caractérisé en ce que** :
(A) on condense de manière intramoléculaire, pour donner des composés de la formule (I-a) : 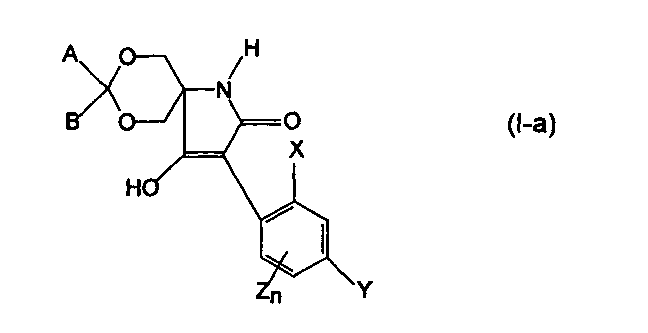 dans laquelle :
A, B, X, Y, Z et n ont les significations indiquées à la revendication 1,
des esters de N-acylaminoacides de la formule (II) : 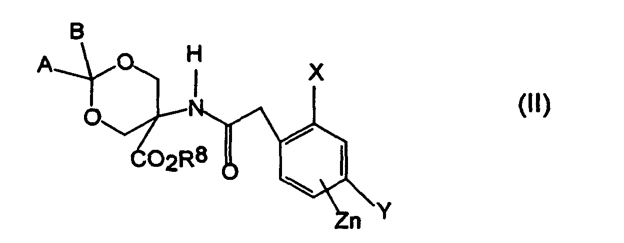
dans laquelle :
A, B, X, Y, Z et n ont les significations indiquées à la revendication 1, et
R⁸ représente un radical alcoyle,
en présence d'un agent de dilution et en présence d'une base,
(Bα) on transforme en des composés de la formule (I), dans laquelle A, B, X, Y, Z, R¹ et n ont les significations données à la revendication 1 et G représente le groupe (b), les composés de la formule (I-a), dans laquelle A, B, X, Y, Z et n ont les significations données à la revendication 1, avec des halogénures d'acide de la formule (III) : 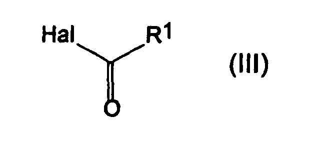
dans laquelle :
R¹ a la signification donnée à la revendication 1, et
Hal représente un atome d'halogène, ou
β) avec des anhydrides d'acide carboxylique de la formule (IV) :
R¹ - CO - O - CO- - R¹ (IV)
dans laquelle :
R¹ a la signification donnée ci-dessus,
le cas échéant en présence d'un agent de dilution et le cas échéant, en présence d'un agent liant les acides ;
ou
(C) on transforme en des composés de la formule (I), dans laquelle A, B, R², M, X, Y, Z et n ont les significations données à la revendication 1, L représente l'atome d'oxygène et G représente le groupe (c), les composés de la formule (I-a), dans laquelle A, B, X, Y, Z et n ont les significations données à la revendication 1, chaque fois avec des esters de l'acide chloroformique ou des thioesters de l'acide chloroformique de la formule (V) :
R² - M - CO - Cl (V)
dans laquelle :
R² et M ont les significations données à la revendication 1, et
le cas échéant en présence d'un agent de dilution et le cas échéant, en présence d'un agent liant les acides ;
ou
(D) on transforme en des composés de la formule (I), dans laquelle A, B, R², M, X, Y, Z et n ont les significations données à la revendication 1, L représente l'atome de soufre et G représente le groupe (c), les composés de la formule (I-a), dans laquelle A, B, X, Y, Z et n ont les significations données à la revendication 1, avec des esters de l'acide chloromonothioformique ou des esters de l'acide chlorodithioformique de la formule (VI) : 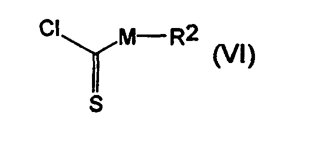 dans laquelle:
R² et M ont les significations données à la revendication 1, et
le cas échéant en présence d'un agent de dilution et le cas échéant, en présence d'un agent liant les acides ;
ou
(E) on transforme en des composés de la formule (I), dans laquelle A, B, R³, X, Y, Z et n ont les significations données à la revendication 1, et G représente le groupe (d), les composés de la formule (I-a), dans laquelle A, B, X, Y, Z et n ont les significations données à la revendication 1, avec des chlorures d'acide sulfonique de la formule (VII) :
R³ - SO₂ - Cl (VII)
dans laquelle:
R³ a la signification donnée à la revendication 1, et
le cas échéant en présence d'un agent de dilution et le cas échéant, en présence d'un agent liant les acides ;
ou
(F) on transforme en des composés de la formule (I), dans laquelle A, B, L, R⁴, R⁵, X, Y, Z, et n ont les significations données à la revendication 1, et G représente le groupe (e), les composés de la formule (I-a), dans laquelle A, B, X, Y, Z et n ont les significations données à la revendication 1, avec des composés du phosphore de la formule (VIII) : 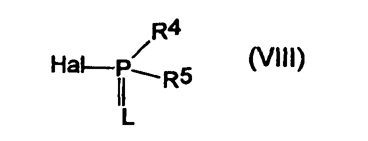 dans laquelle:
L, R⁴ et R⁵ ont les significations données à la revendication 1, et
Hal représente un atome d'halogène,
le cas échéant en présence d'un agent de dilution et le cas échéant, en présence d'un agent liant les acides ;
ou
(G) on transforme en des composés de la formule (I), dans laquelle A, B, E, X, Y, Z et n ont les significations données à la revendication 1, et G représente le groupe (f), les composés de la formule (I-a), dans laquelle A, B, X, Y, Z et n ont les significations données à la revendication 1, avec des composés métalliques ou des amines des formules (IX) ou (X) :
Me(OR¹⁰)ₜ (IX) 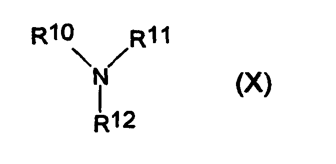
dans laquelle:
Me représente un métal mono- ou bivalent,
t représente le nombre 1 ou 2, et
R¹⁰, R¹¹ et R¹² représentent indépendamment l'un de l'autre, l'atome d'hydrogène ou un radical alcoyle,
le cas échéant en présence d'un agent de dilution ;
ou
(H) on transforme en des composés de la formule (I), dans laquelle A, B, L, R⁶, R⁷, X, Y, Z, et n ont les significations données à la revendication 1, et G représente le groupe (g), les composés de la formule (I-a), dans laquelle A, B, X, Y, Z et n ont les significations données à la revendication 1, avec des chlorures d'acide carbamique ou des chlorures d'acide thiocarbamique de la formule (XI) : 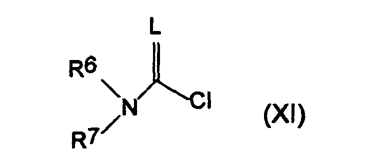 dans laquelle :
L, R⁶ et R⁷ ont les significations données à la revendication 1,
le cas échéant en présence d'un agent de dilution et le cas échéant, en présence d'un agent liant les acides.

5. Composés de la formule (XV) : 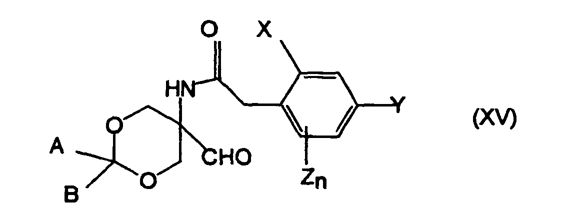 dans laquelle :
A, B, X, Y, Z et n ont les significations indiquées à la revendication 1.

6. Composés de la formule (XVI) : 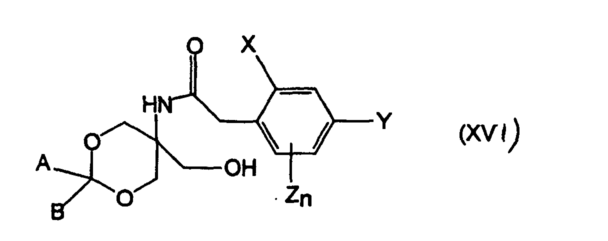 dans laquelle :
A, B, X, Y, Z et n ont les significations indiquées à la revendication 1.

7. Agent de lutte contre les parasites et herbicide, **caractérisé par** une teneur en un composé de la formule (I) suivant la revendication 1.

8. Utilisation des composés de la formule (I) suivant la revendication 1, pour lutter contre des parasites et la croissance non souhaitée de végétaux.

9. Procédé pour lutter contre des parasites, **caractérisé en ce que** l'on fait agir des composés de la formule (I) suivant la revendication 1, sur les parasites ou mauvaises herbes et/ou leur biotope.

10. Procédé pour préparation d'agents de lutte contre des parasites et d'agents herbicides, **caractérisé en ce que** l'on mélange des composés de la formule (I) suivant la revendication 1, avec des diluants et/ou agents tensioactifs.

11. Utilisation des composés de la formule (I) suivant la revendication 1, pour la préparation d'agents de lutte contre des parasites et d'agents herbicides.

12. Composés de la formule (II) : 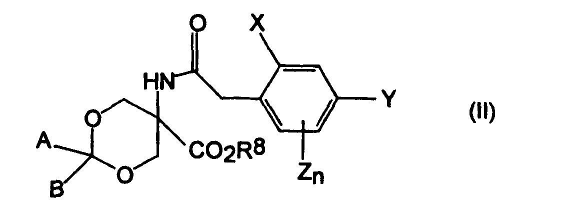 dans laquelle :
A, B, X, Y, Z, R⁸ et n ont les significations indiquées à la revendication 1.
